# EUROPEAN PATENT SPECIFICATION

(11) **EP 4 422 729 B1**
(45) Date of publication and mention of the grant of the patent: **19.08.2026**
(21) Application number: 22887777.5
(22) Date of filing: 25.10.2022
(51) Int. Cl.: A61M 16/06, A61M 16/08, A61M 16/00, A61M 16/10, A61M 16/16

(54) **HEADGEAR AND PATIENT INTERFACE ASSEMBLY**
KOPFHAUBE UND PATIENTENSCHNITTSTELLENANORDNUNG
HARNAIS ET ARRANGEMENT D'INTERFACE PATIENT

(30) Priority: 26.10.2021 US 202163263050 P; 29.04.2022 US 202263363916 P; 12.08.2022 US 202263371296 P
(43) Date of publication of application: 04.09.2024
(60) Divisional application: 26191342.0
(73) Proprietor: Fisher & Paykel Healthcare Limited, Auckland, 2013 (NZ)
(72) Inventor: BECKLEY, Amelia Rhian, Auckland, 2013 (NZ); FAN, Andrew Chun Mon, Auckland, 2013 (NZ); HOWARTH, Brad Michael, Auckland, 2013 (NZ); DIXON, Freya Refilwe, Auckland, 2013 (NZ); PEMMARAJU, Hemanth, Auckland, 2013 (NZ); O'BRIEN, Jed James, Auckland, 2013 (NZ); SOMERVILLE, Jemma Tamsin, Auckland, 2013 (NZ); SMALL, Matthew Ivan, Auckland, 2013 (NZ); MACLEAN, Miriam Rose, Auckland, 2013 (NZ); MCGERTY, Oscar, Auckland, 2013 (NZ); HENSMAN, Sally Margaret, Auckland, 2013 (NZ); DAVIS, Samuel Rollo Ross, Auckland, 2013 (NZ); HENDRICKSEN, Tyler Dean Goff, Auckland, 2013 (NZ)
(74) Representative: Ström & Gulliksson AB
(86) International application number: PCT/NZ2022/050130
(87) International publication number: WO 2023/075610

(56) References cited:
- WO-A1-2011/022779
- WO-A1-2013/026092
- WO-A1-2014/142681
- WO-A1-2014/175753
- WO-A1-2016/077876
- WO-A1-2016/077876
- WO-A1-2017/021836
- WO-A1-2021/176338
- WO-A2-2015/057087
- DE-B4- 102012 005 535
- US-A1- 2004 025 884
- US-A1- 2009 145 435
- US-A1- 2009 260 134
- US-A1- 2016 143 766
- US-A1- 2018 344 960
- US-B2- 10 980 659

## Description

### FIELD OF THE INVENTION

The present disclosure generally relates to a respiratory patient interface, and to headgear for a patient interface.

### BACKGROUND

In assisted breathing, respiratory gases are supplied to a patient through a patient interface via one or more flexible breathing tubes. Such therapies may include but are not limited to continuous positive airway pressure (CPAP) therapy, including for example VPAP and BiPAP systems, non-invasive ventilation (NIV) therapy, and high flow rate therapy.

Various types of respiratory patient interfaces may be used for the provision of different respiratory therapies. For example, the patient interface can be a nasal cannula, nasal mask, oral mask, or oro-nasal mask, endotracheal tube, or other known types of interfaces.

Headgear for a respiratory interface may be used to retain the interface in an operative position on the patients face. The headgear may include straps or other members extending between the patient interface and the headgear. Different style patient interfaces may require different arrangements of straps and other members or may require different headgear entirely.

In the specification where reference has been made to patent specifications, other external documents, or other sources of information, this is generally for the purpose of providing a context for discussing the features of the disclosure. Unless specifically stated otherwise, reference to such external documents is not to be construed as an admission that such documents, or such sources of information, in any jurisdiction, are prior art, or form part of the common general knowledge in the art.

WO 2015/057087 A2 relates to a headgear for a respiratory interface.

### SUMMARY OF THE INVENTION

The invention is defined by the claims.

Patients may use various types of respiratory interfaces for the provision of different respiratory therapies. Some patient interfaces may apply pressure to the skin and facial tissue of a wearer as they are held in place to deliver the respiratory therapy. This can lead to damage to the skin and facial tissue, particularly for infant and neonatal patients who have delicate skin and facial tissue. Further, in medical applications, respiratory patient interfaces are often used for extended periods of time, for example, weeks or months, exacerbating the risk of skin or tissue damage.

For these reasons it can be desirable to periodically change the type of respiratory interface worn by a patient, to vary the areas of skin in contact with the interface. It is also desirable for respiratory patient interfaces and other associated patient-contacting components to be designed and positioned to minimise or avoid pressure points on a wearer's skin.

To enable the respiratory therapy to be provided to the patient, the interface must be retained in some way relative to the patient's mouth and/or nose. This is especially important where the respiratory therapy involves the provision of pressurised gases; the interface must be retained against the patient's face in a manner to provide at least some degree of a seal and prevent undesirable leakage of the respiratory therapy gases from the periphery of the interface.

Headgear may be used to retain the interface on the patient's face, and to maintain the interface in a position such that therapy can be delivered effectively. The headgear typically transfers forces to the patient's head and so may significantly influence the patient's comfort. For infant and neonatal patients in particular, the headgear and associated securement members can apply forces that may lead to skin or tissue damage. It is desirable that headgear and associated securement members minimise or avoid any damage to skin or tissue.

In addition, different patients may have significantly different head and face anatomies. For example, head circumference, skull shape, face shape, neck shape, and face tissue depths are all variable. This is especially pronounced among populations of infant and neonatal patients, due to the high rate of growth of the head in the early months of life. It is desirable that headgear be adjustable or customisable to securely fit each patient in a manner that minimises or avoids pressure points and the risk of skin or tissue damage.

In infant and neonatal patients, head and face anatomy may change in a single patient as the patient grows and develops. It is also desirable that headgear be adjustable or customisable to securely fit the patient to accommodate this growth and development. In addition, infant and neonatal patients may have varying or changing respiratory support requirements. For example, as an infant patient's lungs develop, the infant may require a lesser degree of, or different type of respiratory support. Some patients may experience setbacks and require higher levels respiratory support, requiring change of therapy type. It is desirable that headgear for respiratory interfaces be accommodating of different types of respiratory interfaces to deliver varying degrees of respiratory support.

In a **first aspect,** the present disclosure relates to headgear for securing a patient interface to patient, the headgear comprising a base layer forming a body of the headgear, and a headband region. The headband region comprises an outer engagement layer that at least partly overlaps a lower portion of the base layer, the outer engagement layer being fused to an underlying portion of the base layer. The headband region comprises areas of fused material and areas of unfused material, the non-fused areas at least in part defining connection zones for releasably securing connectors to the headgear.

The outer engagement layer may be fused to the underlying portion of the base layer by way of Radiofrequency (RF) welding or high-frequency (HF) welding, or ultrasonic, vibration or friction welding, hot edge welding, hot air welding, or induction welding.

The outer engagement layer may be fused to the underlying portion of the base layer across a majority of the outer engagement layer.

The outer engagement layer may be fused to the underlying portion of the base layer about substantially all the entire periphery of the outer engagement layer.

The outer engagement layer may be fused to the underlying portion of the base layer exclusively by fusing. That is, said layers may be bonded together without any additive material such as a stitching or an adhesive.

Alternatively, the outer engagement layer may be otherwise attached to the underlying portion of the base layer. For example, the outer engagement layer may be stitched or adhered via adhesive to the base layer. In other embodiments, the outer engagement layer or the base layer may comprise a material that at least partly infuses into the material of the other of the outer engagement layer or the base layer during manufacture, to bond the two layers together. For example, the outer engagement layer or the base layer may comprise a heat and/or pressure sensitive film for bonding to the other layer.

In an embodiment, the outer engagement layer covers substantially all of the headband region. Alternatively, the outer engagement layer may cover the majority of the headband region. As a further alternative, the outer engagement layer may only cover portions of the headband region, for example the front and sides of the headband.

The base layer may comprise a panel, for example a rectangular panel. The panel may be a single-ply material.

The outer engagement layer may comprise a single panel of material. The panel may be a single-ply material.

The patient interface may be a respiratory interface such as a respiratory mask or nasal cannula. In an embodiment, the headgear and the patient interface is for an infant or neonate.

In an embodiment, the unfused material at least in part defines a connection zone for releasably securing to a fastening portion on a patient-facing side of the headgear.

In an embodiment, the fused or unfused regions form a pattern comprising dots and/or stripes. The stripes may be straight, curved, wavy, or angled, for example, and they may be arranged parallel to each other, forming a grid, or be otherwise orientated. Dots in a pattern may be arranged uniformly or non-uniformly, for example, in rows or grids, radiating from a point, or randomly. Alternatively, or additionally, the fused or unfused regions may form text or decorative or identifying shapes such as a logo.

According to the invention, unfused regions form a raised engagement surface, while fused regions form depressions.

In an embodiment, the headband region comprises enlarged over-ear regions shaped to at least partly cover the patient's ears.

The ear regions may be configured to provide a larger engagement surface compared to other regions of the headband, for example by having a larger area of unfused engagement layer, to facilitate the attachment of securing members to the headband to hold the patient interface in place.

The ear regions may be configured to provide protection to a patient's ears against contact with or friction from other connectors or the headgear or interface, or associated components.

In an embodiment, each over-ear region comprises a lobe defined by a rounded, downwardly projecting lower edge of the headband region. Additionally, or alternatively the over-ear regions may each comprise a rounded, upwardly projecting upper edge.

The headband may comprise a bridging portion between the over-ear regions, for positioning at or above the nape of the patient's neck, the bridging section narrower in height than the ear regions.

In an embodiment, the bridging section is shaped to reduce pressure on the nape of a patient's neck. The lower edge of the bridging section may be higher than the lower edge of the ear regions and/or the lower edge of a front portion of the headband. In an embodiment, the bridging region has an arcuate lower edge that is highest at a centre of the bridging region such that the bridging region is narrowest at its centre. In an embodiment, the lower edge of the bridging region is contiguous with the lower edge of the ear regions, with no abrupt transitions.

In an embodiment, the bridging section minimises or prevents creasing of a rear portion of the headband, such as may occur as a result of neck flexion. This may reduce the risk of imprinting of the headgear into a patient's skin.

A portion of the base layer may extend beyond a lower edge of the bridging portion.

The headband region may further comprise at least one extension portion extending from one or both over-ear portions. In an embodiment, the headband region comprises two extension portions, each extending forward from a respective over-ear portion. The extension portion(s) may extend forward from the respective over-ear portion(s).

The headband region and a lower portion of the body are configured to wrap around the head of the wearer such that opposing end portions of the headband overlap and secure to each other, thereby providing an adjustable fit to accommodate a range of head circumferences. The extension portions may be configured to at least partly overlap when the headgear is in use.

In an embodiment, the headgear is free from internal and external seams.

The headgear may include an adjustment device, the body of the headgear passing through the adjustment device. The adjustment device may be selectively slidable along the body of the headgear, towards and away from the headband region to adjust the wearable length of the headgear. The adjustment device may be adjustable between a locked state, and an adjustment state in which the adjustment device is slidable along the body of the headgear. In the locked state the adjustment device may resist movement along the body of the headgear.

This ability to adjust the wearable length of the fabric portion of the headgear ensures the headgear can accommodate a range of head sizes with the headband region correctly positioned over the patient's ears. The adjustment device is generally positioned closer to the headband for a smaller head, and further from the headband for a larger head. This adjustment is typically used in combination with adjusting the circumferential overlap of the fabric and headband, to ensure a snug fit of the headgear.

The adjustment device may comprise one or more of the features described in relation to the third aspect of the disclosure, below.

The headgear may include an end fixture securing a top edge of the headgear body together at a securement point. The end fixture may limit travel of the adjustment device along the fabric thereby prevent inadvertent removal of the adjustment device from the headgear.

In an embodiment, the headgear is a bonnet. In an embodiment, the end fixture forms a bobble at an apex of the bonnet. The adjustment device is generally positioned closer to the bobble for a larger head, and further from the bobble for a smaller head.

A top edge of the headgear body may be gathered, pleated, folded, or rolled for securing at the securement point. The end fixture preferably encloses and obscures the top edge of the headgear body.

The end fixture may comprise two side layers between which the top edge of the headgear body is received. The side layers may be fused to each other, for example about a periphery of the end fixture.

The end fixture may comprise an intermediate layer, for example a foam layer. The intermediate layer may be bonded to the side layers.

The base layer may comprise fabric. The fabric may be one that exhibits two-way stretch in a width-wise direction of the headgear. Alternatively, the fabric may comprise 4-way stretch fabric having stretch in both the lengthwise (vertical) and width-wise directions. For example, the fabric may comprise a knit fabric. In an embodiment, the fabric handle is soft or plush to the touch, for comfort.

The outer engagement layer of the headband region may comprise an unbroken looped (UBL) surface to engage with connectors having a complementary hooked surface.

In an embodiment, the areas of fused material exhibit different stretch to the areas of unfused material.

The shape and/or the orientation and/or the position of the fused areas may be selected to reduce or increase the stretch of a respective region of the headband in one or more directions.

The fused areas may be shaped to form a substantially continuous path across a respective region of the headband, in a direction of undesired stretch. Additionally, the unfused areas may be shaped to form a substantially continuous path from a top edge of the headband to a bottom edge of the headband, in a direction of undesired stretch

The headband region may comprise a region having increased stretch in a longitudinal direction of the headband. In some embodiments, a region of increased stretch is provided between the over-ear portions and/or at the side of one or both over-ear portions. The engagement layer may be absent in the region of increased stretch.

In the over ear regions of the headband, the areas of fused material and the areas of unfused material may be configured to reduce stretch in a longitudinal direction of the headband and/or a direction at an angle to or generally diagonal to the longitudinal direction, for example 45 degrees to the longitudinal direction.

The over ear regions may comprise areas of fused material and areas of unfused material.

In another embodiment, the outer engagement layer or the base layer comprises one or more cut-outs and the headband may exhibit different stretch in areas of the headband with cut-outs compared to the surrounding regions.

The shape and/or the orientation and/or the position of the cut-outs may be selected to reduce the stretch of a respective region of the headband in one or more directions compared to the regions without cut-outs.

In an embodiment, the base layer is non-rectangular. The top edge of the base layer may be non-linear.

A height of the base layer may be greater at or near a midline of the base layer than at or near sides of the base layer.

The base layer may have the shape of an irregular pentagon, with two sides of the pentagon forming a top edge of the base layer.

The two sides forming the top edge of the base layer may meet at a central vertex that is aligned with a region between the over ear regions. The two sides may form an angle of about 100 degrees with a side edge of the headgear body.

A top edge of the base layer may comprise one or more cut-outs and optionally the cut-outs may be triangular.

The top edge may be shaped such that when the top edge is gathered and secured at a securement point, the securement point is approximately in line with a spine of the wearer.

In a **second aspect,** the present disclosure relates to Headgear for securing a patient interface to patient, the headgear comprising a headband configured to wrap and secure around the head of the wearer to provide an adjustable fit. The headband has over-ear regions to at least partly cover the patient's ears, the over-ear regions being enlarged regions, and/or the headband comprising a rear bridging portion between the over-ear regions.

The headband region may further comprise at least one extension portion extending from one or both over-ear portions. In an embodiment, the headband region comprises two extension portions, each extending forward from a respective over-ear portion. The extension portion(s) may extend forward from the respective over-ear portion(s). The extension portions may be configured to at least partly overlap when the headgear is in use.

In an embodiment, each ear region comprises a lobe defined by a rounded, downwardly projecting lower edge of the headband.

The bridging portion may be narrower in height than the over-ear regions and configured for placement at or above the nape of the patient's neck. The bridging section may be shaped to reduce pressure on the nape of a patient's neck. The lower edge of the bridging section may be higher than the lower edge of the ear regions and/or the lower edge of a front portion of the headband. In an embodiment, the bridging region has an arcuate lower edge that is highest at a centre of the bridging region such that the bridging region is narrowest at its centre. In an embodiment, the lower edge of the bridging region is continuous with the lower edge of the ear regions, with no abrupt transitions.

The patient interface may be a respiratory interface such as a respiratory mask or nasal cannula. In an embodiment, the headgear and the patient interface is for an infant or neonate.

In an embodiment, the headband comprises an engagement surface for releasably securing connectors to the headgear.

In an embodiment, the headband comprises a plurality of layers, the engagement surface being provided by an outer layer that is fused to one or more inner base layers. The outer engagement layer may be fused to the underlying portion of the fabric body by way of Radiofrequency (RF) welding or high-frequency (HF) welding, or ultrasonic, vibration or friction welding, hot edge welding, hot air welding, or induction welding. The one or more base layers may comprise fabric.

In an embodiment, the outer engagement layer covers substantially all of the headband region, alternatively the outer engagement layer may cover the majority of the headband region. As a further alternative, the outer engagement layer may only cover portions of the headband region, for example the front and sides of the headband.

In an embodiment, the engagement surface is provided by a layer of unbroken looped (UBL) fabric.

The headband may comprise areas of fused material and areas of unfused material, the unfused material at least in part defining connection zones for releasably securing connectors to the headgear.

In an embodiment, the headband comprises areas of fused material and areas of unfused material. The areas of fused material may exhibit different stretch to the areas of unfused material.

The shape and/or the orientation and/or the position of the fused areas may be selected to reduce or increase the stretch of a respective region of the headband in one or more directions.

The fused areas may be shaped such that they form a substantially continuous path from a top edge of the headband to a bottom edge of the headband, in a direction of undesired stretch.

In the over ear regions of the headband, the areas of fused material and areas of unfused material may be configured to reduce stretch in a longitudinal direction of the headband and/or a direction at 45 degrees to the longitudinal direction.

The over ear regions may comprise areas of fused material and areas of unfused material.

The headband may comprise a region having increased stretch in a longitudinal direction of the headband. In some embodiments, a region of increased stretch is provided between the over-ear portions and/or at the side of one or both over-ear portions.

In an embodiment, the headband region comprises an outer layer and one or more inner layers, where the outer layer and/or inner layer may comprise one or more cut-outs and the headband may exhibit different stretch in areas of the headband with cut-outs compared to the surrounding regions.

The shape and/or the orientation and/or the position of the cut-outs may be selected to reduce the stretch of a respective region of the headband in one or more directions compared to the regions without cut-outs.

In an embodiment, the fused or unfused regions form a pattern comprising dots and/or stripes. The stripes may be straight, curved, wavy, or angled, for example, and they may be arranged parallel to each other, forming a grid, or be otherwise orientated. Dots in a pattern may be arranged uniformly or non-uniformly, for example, in rows or grids, radiating from a point, or randomly. Alternatively, or additionally, the fused or unfused regions may form text or decorative or identifying shapes such as a logo.

The pattern formed by the fused or unfused regions may comprise elements having a directional arrangement such as being 'Y' shaped or 'V' shaped, for example. Alternatively, they may comprise any other suitable shape.

The size and/or density of the pattern of the fused and/or unfused regions may vary along the headband. For example, the pattern of the fused and unfused regions may have a higher density pattern at regions where lower stretch is desired. In areas of the headband with larger surface areas such as the over-ear regions, the fused and/or unfused regions may be larger than in other areas of the headband.

In an alternative embodiment, one layer of the headband may comprise cut-outs in addition to or in place of the fused and non-fused regions. The cut-outs may be configured to vary the stretch of the headband in different regions and directions.

In an embodiment, the cut-outs may form a pattern comprising dots and/or stripes. The stripes may be straight, curved, wavy, or angled, for example, and they may be arranged parallel to each other, forming a grid, or be otherwise orientated. Dots in a pattern may be arranged uniformly or non-uniformly, for example, in rows or grids, radiating from a point, or randomly. Alternatively, or additionally, the fused or unfused regions may form text or decorative or identifying shapes such as a logo.

The pattern formed by the cut-outs may comprise elements having a directional arrangement. The elements may be 'Y' shaped or 'V' shaped, for example. Alternatively they may comprise any other suitable shape, for example diamonds, rectangles, ovals, circles, strips, and many other shapes are envisaged.

In an embodiment, unfused regions form a raised engagement surface, while fused regions form depressions.

A patient-contacting underside of the headband may comprise a region of increased friction.

A patient-contacting underside of the headband may comprise a tacky surface. The tacky surface may comprise a tape comprising a polyurethane adhesive film and a polyurethane elastic barrier (e.g., BemisTM tape), neoprene, non-stick silicone, and/or thermoplastic polyurethane. The tacky surface may comprise one suitable for contact with skin and may be provided over substantially all of the underside of the headband, the majority of the underside of the headband, or may only cover portions of the underside of the headband.

In an embodiment, the headgear comprises a body panel with the headband provided along a lower portion of the body. The body panel may comprise a layer of fabric. In one embodiment the headgear comprises a bonnet/beanie.

In a **third aspect,** the present disclosure relates to an adjustment device for adjusting the size of headgear having a flexible body, the device comprising first and second engagement members, and an intervening hinge region. The first and second engagement members may be movable relative to each other to adjust the device between a locking state and a free state, wherein in the locking state the engagement regions grip the body of the headgear to hold the device in position on the headgear, and wherein in the free state the grip is released sufficiently enabling the device to move along the fabric of the headgear to thereby adjust the size of the headgear.

In an embodiment, the device is moved from the locking state to the free state by pressing the first and second engagement members towards each other.

The device may comprise finger grips on opposite sides of the device to facilitate pressing the first and second engagement members towards each other. The first and second engagement members may extend inwardly from respective finger grips.

In an embodiment, the finger grips comprise a region offering increased friction or purchase. For example, the finger grips may comprise a textured, contoured, and/or recessed surface, or alternatively the finger grips may comprise a surface material having increased friction, for example rubber.

In an embodiment, the finger grips are provided by opposite, outward facing surfaces. The surfaces are typically side surfaces, adjacent the respective engagement members. The device may comprise two finger grips, with one finger grip provided on either side of hinge, for example on opposite sides of the hinge region.

In an embodiment, the hinge region is resilient, and device in the locking state in a resting state of the device. The device may be biased towards the locking state.

In an embodiment, the hinge may be provided by a member or region of the device that is shaped to have a hinge point. For example, the hinge may be provided by a bent, curved, or angled member. In an embodiment, the device comprises one or more arched members, defining a hinge point at an apex of the arch. In one embodiment the device comprises two arched members. Alternatively, the hinge may be provided by a narrowed or thinned region or member.

In an embodiment, the first and/or second engagement member may comprise an aperture for receiving the body of the headgear.

The first and/or second engagement members may comprise a loop defining the respective aperture. Alternatively, the first and/or second engagement members may comprise a hook defining an area for receiving the body of the headgear. The defined aperture or area may be any suitable shape, for example round, oval, D-shaped, square, or rectangular.

In an embodiment, both the first and second engagement members define an aperture. These apertures may be substantially the same size and shape.

In an embodiment, the first and second engagement members each comprise an aperture for receiving the body of the headgear, and wherein in the locking position the apertures are misaligned and in the free position the apertures are generally aligned to allow body to slide through the apertures.

In an embodiment, the first and second engagement members each comprise an aperture for receiving the body of the headgear, and wherein in the free state there is substantially more overlap between the apertures of the engagement members compared to in the locking state.

In an embodiment, in the locking state, the body of the headgear follows a tortuous path through the device. The torturous path may comprise one or more bends of the flexible body material. In the locking state, the device may provide more resistance to movement along the fabric compared to the free state.

In an embodiment, the second engagement member comprises a pair of spaced apart loops defining two respective apertures, and wherein the first engagement member comprises a loop that is configured to slide between the two second engagement member loops as the first and second engagement members are moved relative to each other.

In an embodiment, the loops of the second engagement member are parallel, and the spacing between the loops of the second engagement member is the same or greater than a thickness of the loop of the first engagement member.

In an embodiment, a guide aperture to receive and slide along the fabric of the headgear. The hinge may be provided at the sides of the guide aperture.

In an embodiment, a guide aperture is provided between two hinge side members. In an embodiment, the hinge side members extend between the finger grips. The hinge members may comprise two parallel arched members, with the space between the hinge members defining the guide aperture.

In an embodiment, a hinge axis of the device extends through the guide aperture. In an embodiment, in the in the free position of the device, the guide aperture is generally aligned with the engagement member apertures to allow fabric to slide through the device following a generally straight path.

In an embodiment, the device is integrally formed. That is, the hinge, and first and second engagement members are integrally formed.

In an embodiment, the device comprises one or more of acetal, nylon, a suitable polymer, for example a thermoplastic polymer such as acrylonitrile butadiene styrene (ABS), polycarbonates, or a polycarbonate/thermoplastic polymer blend.

In a **fourth aspect,** the present disclosure relates to an adjustable headgear system comprising the headgear according to the first or second aspect, and the adjustment device according to the third aspect, wherein the adjustment device is configured to receive and slide along the body of the headgear, towards and away from the headband region, to thereby adjust the size of the headgear in a vertical direction.

The headgear may have any one or more of the features described above in relation to the first and/or second aspects.

The adjustment device may have any one or more of the features described above in relation to the third aspect.

In a **fifth aspect,** the present disclosure relates to an adjustable headgear system comprising the headgear according to the first or second aspect, and an adjustment device, wherein the adjustment device comprises a flexible body with first and second ends and first and second sides defining an aperture configured to receive and slide along the body of the headgear. The first and second engagement ends may be movable relative to each other to adjust the device between a locking state and a release state, wherein in the locking state the device grips the body of the headgear in the aperture, to hold the device in position relative to the headgear, and wherein in the release state the grip of the device is released sufficiently, enabling the device to move along the body of the headgear towards and away from the headband region, to thereby adjust the size of the headgear in a vertical direction.

In a **sixth aspect,** the present disclosure relates to a patient interface and headgear assembly comprising a patient interface assembly, headgear, and a side connector member for coupling the patient interface assembly to the headgear. The side connector comprises a patient interface connection point for coupling to a side of the patient interface assembly, and spaced apart upper and lower connection points for coupling to the headgear.

The assembly may include two side connector members. In an embodiment, the side connector member is flexible.

The side connector member may be narrower at a first end for attaching to the patient interface assembly, and wider at an opposite second end for connection to the headgear. The spaced apart upper and lower connection points may be proximal the second end, and the patient interface connection point may be proximal the first end.

In an embodiment, the flexible securement member is Y-shaped. For example, the side connector member may be wishbone shaped.

In an embodiment the side connector member comprises a patient attaching portion that bifurcates to upper and lower headgear connecting portions. In alternative embodiments, the securement member may be generally triangular or may have a fan-like shape. The member may comprise one or more cut-outs.

In an embodiment, the side connector member comprises a plurality of layers, at least one of the layers being a fabric layer.

In an embodiment, the flexible side connector member comprises outer fabric layers, an inner patient contacting fabric layer, and an intermediate stiffening sandwiched between the fabric layers
The stiffening layer may comprise a polymer layer. For example, the stiffening layer may comprise a nylon sheet.

The stiffening layer may be bonded to at least one of the fabric layers. The stiffening layer may be fused to at least one of the fabric layers, for example using radio frequency (RF) welding or high-frequency (HF) welding, or ultrasonic, vibration or friction welding, hot edge welding, hot air welding, or induction welding,

In one embodiment the stiffening layer is smaller than the outer and inner fabric layers such that the lower fabric forms a border around the perimeter of the stiffening layer. The upper and lower fabric layers may be bonded together around the border region, for example by way of RF welding.

In an embodiment, the outer fabric layer comprises unbroken looped (UBL) fabric.

In an embodiment, the outer fabric layer is smaller than the inner fabric layer but larger than the stiffening layer such that the inner fabric layer creates a single layer perimeter about the device.

In an embodiment, the inner fabric layer is a comfort layer, and is sized such that the edge of the inner fabric layer extends beyond the perimeter of the stiffening layer.

In an embodiment the inner fabric layer also extends beyond the perimeter of the outer fabric layer, thereby creating a soft, comfort edge about the device.

The layers of the side connector member may be fused together. For example, the layers of the side connector may be fused together using one or more of radio frequency (RF) welding, high-frequency (HF) welding, ultrasonic, vibration or friction welding, hot edge welding, hot air welding, or induction welding.

In an embodiment, the side connector member comprises areas of fused material and areas of unfused material, the unfused material at least in part defining connection zones for releasably securing connectors to the headgear.

In an embodiment, contrast between the fused and unfused regions form a pattern to indicate the correct orientation of the connector.

In an embodiment, the fused or unfused regions form a pattern comprising dots and/or stripes and/or shapes. The stripes may be straight, curved, wavy, or angled, for example, and they may be arranged parallel to each other, forming a grid, or be otherwise orientated. Dots in a pattern may be arranged uniformly or non-uniformly, for example, in rows or grids, radiating from a point, or randomly. Alternatively, or additionally, the fused or unfused regions may form text or decorative or identifying shapes such as a logo.

In an embodiment, the pattern is visible on the outer surface of the connector. The pattern may correspond to a pattern on connector zone of the headgear
In an embodiment, the patient interface connection point and the upper and lower headgear connection points comprise hook or loop connectors.

In an embodiment, the patient interface connection point is provided on an outer surface of the connector and the upper and lower headgear connection points are provided on an inner surface of the connector.

In an embodiment, the assembly comprises a lateral arm coupling the flexible connector to the patient interface. Additionally, or alternatively, the assembly may comprise two side connector members arranged laterally, and two respective lateral arms for coupling the side connector members to the patient interface. Additionally, or alternatively, the assembly may comprise a chin strap for coupling to the headgear.

In an embodiment, the headgear may be the headgear described above in relation to the first or second aspects.

The patient interface may be a respiratory interface such as a respiratory mask or nasal cannula. In an embodiment, the headgear and the patient interface is for an infant or neonate.

In a **seventh aspect,** the present disclosure relates to a patient interface assembly comprising a patient interfacing body, a frame to hold the patient interfacing body, and a pair of lateral arms. A first end of each lateral arm is releasably connecting to a front face of the frame at a respective connection zone of the frame, and each connection zone is positioned between mid-point of the frame and a respective side of the frame
In an embodiment the two lateral arms are separate members, individually connectable and removable from the frame.

In an embodiment, each frame connection zone spaced from a midpoint of the frame, towards a respective side of the frame.

In an embodiment, each frame connection zone is provided closer to the respective side of the frame than to midpoint of the frame.

In an embodiment, each frame connection zone comprises one or more male connectors for receipt by respective apertures or recesses in the respective lateral arm.

In an embodiment, each frame connection zone comprises a first projection having an enlarged end, for receipt through an aperture in the respective lateral arm.

The first projection may comprise a post with the enlarged end at a top of the post. In an embodiment, the enlarged end of the first projection is configured to prevent inadvertent decoupling of the lateral arm from the frame by resisting pull-off of the lateral arm. In an embodiment, the enlarged end is not centred on the post and instead projects towards the respective side of the frame.

In an embodiment, the enlarged end of the projection has an area that is larger than the cross-section of the receiving aperture in the respective lateral arm. For example, in one embodiment, the area of the enlarged end is at least twice the cross-sectional area of the post. The cross-sectional area of the post may substantially correspond to the area of the cross-sectional area of the respective arm aperture.

In an embodiment, the aperture in each arm for receiving the first projection has a recess shaped to receive the enlarged end of the projection. In an embodiment, a top surface of the enlarged end of the projection is substantially flush with a top surface of the arm when the enlarged end is seated in the recess.

In an embodiment, the first projection is provided closer to the respective side of the frame than to midpoint of the frame.

In an embodiment, each frame connection zone comprises a second projection having the form of a hooked connector for receipt in a complementary recess in the respective lateral arm. The connection between the hooked connector and the lateral arm may be concealed by a front surface of the lateral arm.

In an embodiment, the hooked connector comprises a post with a hook portion extending from the top of the post at 90 degrees to the post. In an embodiment, the hook portion is substantially parallel with a surface of the frame. In an embodiment, the hook extends towards the mid-line of the frame.

In an embodiment, the complementary recess in the respective lateral arm for receiving the hooked connector comprises a blind L-shaped hole.

In an embodiment, a top surface of the enlarged end of the hooked connector is positioned below a top surface of the arm when the arm is coupled to the frame.

In an embodiment, first end portions of the lateral arm comprise a flexible, elastic material for pulling and/or pressing into engagement with the frame connector(s).

In an embodiment, each arm is assembled to the frame by seating the hooked connector in the complementary recess, then pulling the flexible arm down over the first projection until the enlarged portion of the first projection is seated against a surface of the arm. In the coupled configuration an under surface of the arm may contact an outer surface of the frame.

In an embodiment, the flexible arm may comprise one or more of silicone, a thermoplastic elastomer, or another suitable plastic such as PET, HDPE, polypropylene, resins, polymers or combination materials. In one embodiment, the flexible arm comprises a thermoplastic elastomer.

In an alternative embodiment, the frame may comprise only a single projection for engaging each arm, the single projection having a post and an enlarged end. Each enlarged end may project beyond the respective post, towards the mid-line of the frame to provide a hook portion.

In an embodiment, each post has a generally triangular cross-section, with an apex of the triangle pointing towards the respective side of the frame. In an embodiment, the enlarged end is rectangular.

In an embodiment, the arm comprises a rigid end portion for attaching to the single connector. Preferably the rigid end portion does not extend past the side of the frame when the arm is engaged with the frame.

In an embodiment, first end portions of the lateral arm each comprise a rigid clip that engages the respective frame projection by way of a two stage, sliding and snap-down, motion.

In an embodiment, the rigid end portion or clip comprises a hard plastic such as polypropylene. In an embodiment, the flexible arm may comprise one or more of silicone, a thermoplastic elastomer, or another suitable plastic such as PET, HDPE, polypropylene, resins, polymers or combination materials. In one embodiment the flexible arm comprises a thermoplastic elastomer bonded to the rigid end portion or clip.

In an embodiment the body of the flexible arm is over-moulded to the end portion or clip.

In an embodiment, a second end of each arm comprises a connection surface for engaging with a headgear or connectors for securing the interface assembly on a patient. The connection surface may comprise a hooked or looped surface.

In an embodiment, the connection surface is provided by a pad, the pad comprising an over-moulded loop pad. The connection pad may be integral with the body of the flexible arm.

In an embodiment, each lateral arm comprises an opening between the first end and a second end of the arm, the opening providing visibility of one or more elements at the respective side of the frame.

In an embodiment, the assembly comprises a patient interface body for coupling to the frame. Sides of the patient interface may include one or more features that are at least partly visible through the openings in the lateral arms. In one embodiment, the assembly comprises O-rings on a side of the patient interface. The O-rings may be coloured to indicate a size or other feature of the patient interface.

The patient interface body may have one or more of the features described in relation to the seventh or nineth aspects.

In an embodiment, the assembly includes a connector for coupling the frame to a gas supply or removal conduit. The assembly may comprise two connectors, for connecting to each side of the frame. In an embodiment, at least a portion of the, or each, connector is visible through the opening in the, or each, lateral arm. The connector(s) may be a collar as described in relation to the twelfth aspect.

In an embodiment, each lateral arm comprises an opening between the first end and a second end of the arm, the opening allowing passage therethrough of a conduit for the supply or removal of gases to/from the frame.

In an **eighth aspect,** the present disclosure relates to an interface body for a patient interface assembly, the interface body comprising a patient contacting portion and a coupling portion for coupling to a frame. The coupling portion comprises top and bottom stiffening regions, each stiffening region having a convex coupling wall to seat against a complementary surface of the frame, and wherein the convex coupling walls define a recess for engagement with the frame.

In an embodiment the interface body comprises a respiratory mask or a nasal cannula.

In an embodiment, the interface body is for an infant or neonate. The interface body may comprise a sealing or non-sealing type of interface.

In an embodiment, the thickness of the stiffening regions, and thereby the height of the coupling walls, varies across a width of the interface body. The height of the coupling wall may be larger towards the sides of the interface body, and smaller towards a midline of the interface body.

In an embodiment, each stiffening region comprises a curved outer surface.

In an embodiment, interface body comprises a breathing chamber, and wherein a cross section of the breathing chamber is generally cylindrical proximal sides of the interface body, and generally D-shaped at a midline of the interface body.

In a **ninth aspect,** the present disclosure relates to a patient interface assembly comprising the interface body as described above in relation to the seventh aspect, and a frame, the frame comprising a coupling portion for engaging with the coupling portion of the interface body, wherein the coupling portion of the frame comprises a pair of opposing concave surfaces for engaging the coupling walls of the interface body.

The coupling walls of the interface body may protrude above the coupling portion of the frame when the interface body is engaged with the frame.

In an embodiment, each coupling wall protrudes a first distance above the frame coupling portion at the side edges of the coupling wall, and a protrude a second distance above the frame coupling portion at a midpoint of the coupling wall, wherein the first distance is greater than the second distance.

In an embodiment, the cross-sectional profile of the frame coupling portion varies in shape along the coupling portion.

In an embodiment, the cross-sectional profile has a thickness that varies across the frame coupling portion.

In an embodiment, the thickness of the cross-sectional profile is larger towards a midline of the interface body and smaller towards the sides of the interface body.

In a **tenth aspect,** the present disclosure relates to an interface body for a patient interface, the interface body comprising a nasal cannula having two nasal prongs, wherein the wall of the interface body has a region of reduced thickness in a philtrum region, for location adjacent to a patient's philtrum in use.

In an embodiment, the philtrum region is configured to provide increased compliance compared to at least one adjacent area of the patient interface, and thereby decreased pressure on the patient's philtrum.

In an embodiment, the philtrum region is positioned below the base of the prongs.

In an embodiment, the philtrum region is a generally oval region.

In an embodiment, the philtrum region extends generally across the interface body.

In an embodiment, the wall thickness of the philtrum region is substantially constant.

In an embodiment, the philtrum region has a wall thickness of between about 0.2mm and about 1.0mm. In one embodiment, the philtrum region has a wall thickness of between about 0.4 mm and about 0.6mm. In one embodiment the philtrum region has a wall thickness of about 0.5mm. In some embodiments the wall thickness may vary across the philtrum region.

The interface body may further comprise a flexing region adjacent the base of the prongs, wherein the wall of the interface body in the flexing region has a reduced thickness compared to the philtrum region.

The flexing region may have a wall thickness of between about 0.1mm and about 1.0mm. In one embodiment the flexing region has a wall thickness of about 0.3 mm.

In an embodiment, the flexing region has a generally reniform shape.

In an embodiment, the wall of the interface body comprises four regions of differing wall thicknesses: the prongs having a first wall thickness, the flexing region having a second wall thickness, the philtrum region having a third wall thickness, and the remaining body having a fourth wall thickness.

In an embodiment, a boundary of the flexing region is shared by the philtrum region. That is, the flexing region and the philtrum region are directly adjacent/contiguous.

In an embodiment, a septal relief recess is provided between the two prongs. The recess may be recessed beyond a base of the prongs and/or a front surface of the interface body at the sides of the prong. In an embodiment the recess is sized to prevent or minimise contact between the nasal septum and the patient interface, when the interface is in use. The recess may be provided by a groove, dip, or channel, for example.

In an embodiment, the septal recess has a depth of between about 1.5 mm and about 2.5mm, preferably between about 1.8 mm and about 2.2mm from the base of the prongs. In one embodiment the septal recess has a depth of about 1.9 mm from the base of the prongs. In another embodiment the septal recess has a depth of about 2.2 mm from the base of the prongs. The patient interface may be a respiratory interface for an infant or neonate.

In an **eleventh aspect,** the present disclosure relates to an assembly for a patient interface, the assembly comprising a frame to hold an interface body, and a stability arm connectable to the frame and connectable to headgear. The stability arm comprises a flexible portion that allows the arm to be flexed away from the patient or headgear during assembly.

In an embodiment, the flexible portion comprises a region of the arm having a reduced wall thickness.

In an embodiment, the thickness of the arm transitions from the reduced wall thickness at the flexible portion to a larger thickness at adjacent portions of the arm.

In an embodiment, wherein a major length of the stability arm is substantially rigid. In an embodiment, the flexible portion is positioned proximal one end of the rigid portion, proximal a first end of the arm. In an embodiment, the substantially rigid length of the stability arm has a constant thickness.

In an embodiment, the stability arm comprises a connection feature for connection to the frame, proximal a first end of the arm, and a headgear connector is provided at an opposite, second end of the arm; wherein the headgear connector comprises a pad having a hook or loop surface.

In one embodiment, the pad has a loop surface. The arm and pad may be configured such that a lower end of the pad overhangs a lower edge of the headgear. A loop surface may reduce risk of skin damage.

The pad may be any suitable shape, for example oval, or round. In one embodiment, the pad is oval with a major axis of the pad aligned with a longitudinal axis of the arm.

In an embodiment, the headgear connector pad is hinged to the stability arm.

In an embodiment, the headgear connector pad is pivotally attached to a second end of the arm about a rotation axis that is perpendicular to the longitudinal axis of the arm. The pivot axis may comprise a pin. In one embodiment the pad is oval and the pivot axis is parallel to a minor axis of the pad.

In an embodiment, the pivot axis is set back from the bottom edge between about one quarter and one third of the length of the support pad.

In an embodiment, the connector pad is a flexible member. For example, the connector pad may comprise a thermoplastic elastomer.

In an embodiment, the stability arm comprises a connection feature for connection to the frame, and wherein the flexible portion is proximal the connection feature.

In an embodiment, the stability arm is transparent or translucent.

In an embodiment, the stability arm comprises a connection feature for connection to the frame and the stability arm may be configured to have an installed position that is substantially aligned with a midline of the frame, and a connection/disconnection position that is at an angle to the midline of the frame.

The connection feature of the stability arm may comprise one or more of the features described below in relation to the eleventh aspect.

The fame may comprise one or more of the features for engagement with the stability arm, as described below in relation to the eleventh aspect.

In an embodiment, the assembly comprises a patient interface body for coupling to the frame. The patient interface body may have one or more of the features described in relation to the seventh or nineth aspects.

In a **twelfth aspect,** the present disclosure relates to an assembly for a patient interface, the assembly comprising a frame to hold an interface body, and a stability arm connectable to the frame and connectable to headgear; wherein the stability arm has an installed position that is substantially aligned with a midline of the frame, and a connection/disconnection position that is at an angle to the midline of the frame.

The connection/disconnection position may be between about 30 degrees and about 90 degrees from the midline of a frame, for example between about 30 degrees and about 60 degrees, or between about 40 degrees and about 50 degrees. In an embodiment the connection/disconnection position is about 45 degrees from the midline of a frame.

In an embodiment, the frame comprises a protrusion for coupling the stability arm to the frame, and the stability arm comprises a connection feature proximal a first end of the arm for connection to the frame protrusion, wherein the connection feature is configured to receive the protrusion.

In an embodiment, the protrusion comprises a post with an enlarged head at an end of the post. The enlarged head may be generally square in shape. In an embodiment, the enlarged head comprises four lobes projecting laterally from a top of the post. The lobes may form a square head.

In an embodiment, the connection feature comprises an aperture. On an under surface of the stability arm, the edge of the aperture may be filleted to create a gradual transition from the under surface to the aperture.

In an embodiment, the under surface of the stability arm is the patient facing surface of the stability arm

In an embodiment, the aperture is generally square in shape, and oriented such that a diagonal of the square is substantially aligned with the midline of the frame in the installed position.

In an embodiment, a top surface of the stability arm comprises recesses positioned at respective sides of the square aperture, wherein the recesses are positioned to receive respective portions of the frame projection in the installed position.

In an embodiment, the recesses comprise four generally triangular recesses, to receive respective corners of a square head of the projection.

In an embodiment, in the connection/disconnection position, the head of the projection is aligned with the connection aperture of the arm, such that the aperture fits over the head of the projection; and wherein in the installed position, the head of the projection is seated in the recesses, with the post of the projection extending through the aperture.

In an embodiment, as the arm moves to its installed position, movement of the projection into the recesses creates tactile feedback.

An under surface of the enlarged head of the projection may move across a top surface of the arm between the connection/disconnection position and the installed position.

In an embodiment, an under surface or side of the enlarged end interferes with corners of the recesses as the stability arm is moved between the connection/disconnection position and the installed position, creating resistance to movement.

In an embodiment, the projection has a height that is substantially the same as the thickness of a first end of the arm such that, in the installed position, a top surface of the enlarged end of the projection is substantially flush with a top surface of the arm, while an under surface of the first end of the arm is in contact with a surface of the frame.

In an embodiment, the frame projection is positioned on a front face of the frame, at a midpoint of the frame.

In an embodiment, the stability arm comprises a headgear connector at a second end of the arm, wherein the headgear connector comprises a pad having a hook or loop surface. The headgear connector pad may be hinged to the stability arm.

In an embodiment, the stability arm may include one or more of the features described above in relation to the tenth embodiment.

In an embodiment to attach the stability arm to the frame, the connection aperture at the first end of the arm is placed over the frame protrusion

In an embodiment, the assembly comprises a patient interface body for coupling to the frame. The patient interface body may have one or more of the features described in relation to the seventh or nineth aspects.

In a **thirteenth aspect,** the present disclosure relates to an assembly for a patient interface, the assembly comprising a frame to hold an interface body, a conduit, and a collar coupling the conduit to the frame. The collar receives an end portion of the conduit, and a length of the end portion of the conduit is fixed to the collar.

In an embodiment, the collar is bonded to frame.

In an embodiment, the collar is overmoulded to the frame.

In an embodiment, the connection between the frame and the collar is a permanent connection, and the connection between the collar and the conduit is a permanent connection.

In an embodiment, the length of the end portion of the conduit attached to the collar is greater than 1 mm.

In an embodiment, the length of the conduit end portion attached to the collar is less than the length of the conduit end portion received in the conduit, such that there is an unbonded length of conduit within the collar.

In an embodiment, the length of the conduit end portion attached to the collar is positioned at the frame end of the conduit.

In an embodiment, the length of the conduit end portion of the conduit attached to the collar is at the end of the conduit.

In an embodiment, the collar comprises a flexible body.

In an embodiment, the collar comprises one of more of a thermoplastic, polyurethane, and/or silicone.

In a **fourteenth aspect,** the present disclosure relates to a patient interface connector for coupling a patient interface assembly to headgear, the connector having an interface attachment point proximal a first end of the connector on an outward facing surface of the connector, and a connection point for coupling to the headgear on a patient facing surface of the connector; wherein the connector increases in width from a first end to a second end. The connector comprises one or more stiffening components, stiffening layers, and/or stiffened regions.

In an embodiment, the connector has two spaced apart headgear attachment points on a patient facing surface of the connector for connecting to headgear.

In an embodiment, the connector is flexible.

The perimeter edge of the connector may comprise curved portions and may be free from angular corners.

In an embodiment, the side connector member is Y-shaped. A bifurcation point of the Y shape may be positioned closer to the second end than to the first end.

In an embodiment, the stiffening component is configured to prevent buckling or twisting of the component in use.

The stiffening component or feature may be positioned at least across a mid-region of the connector and may be configured to prevent buckling or twisting of the mid-region in use.

In an embodiment, the patient interface comprises a relatively rigid stiffening layer, and a comfort layer, where the comfort layer is positioned on a patient facing side of the connector. The comfort layer may be over-moulded or co-moulded with the stiffening layer.

The comfort layer may extend around a perimeter edge of the connector. Additionally, the comfort layer may be rounded or tapered at the perimeter.

The comfort layer may extend above the stiffening layer to form a lip around the perimeter of the connector.

The comfort layer may be shaped to form a compliant, compressible edge of the connector. Additionally, the compressible edge may be configured to deform by deflecting or folding to conform to a wearer's facial contours.

The comfort layer may comprise a fin and/or a recess adjacent the edge of the connector.

The stiffening layer may be more rigid than the comfort layer.

The thickness of the fin may be selected to have sufficient strength to be self-supporting and/or to prevent inadvertent deflection of the fin towards the wearer. The thickness of the fin may also be selected such that the fin does not form a pressure point and/or a hard edge.

The stiffening layer may comprise a thermoplastic material, for example, polypropylene. The thermoplastic material may be inelastic.

The stiffening layer may include one or more features to enhance stiffness in a desired direction and/or to increase flexibility in a desired direction. In some embodiments the stiffening layer includes cut-outs, scored patterns, engravings, thinned regions or other features to selectively decrease the thickness of the stiffening layer at defined points. The cut-outs may include slits or slots, for example, a series of straight, curved, or shaped slots. The slits or slots may be orientated in a generally transverse direction of the connector to enhance flexibility and bending of the connector around a patient's face. The cut-outs or other features may extend to a periphery of the stiffening layer or may terminate at points spaced inwards from the periphery.

The comfort layer may comprise an elastomeric material, for example, silicone.

In one embodiment, the comfort layer extends away from the patient facing surface at an angle of between 0 and about 90 degrees.

In an embodiment, the side connector member is curved to follow or accommodate the contours of a wearer's face.

The patient facing surface of the connector may have a concave curvature.

In an embodiment, the patient interface connector comprises one or more stiffened regions, the stiffened region(s) comprising an area of increased material thickness.

The stiffened region(s) may comprise outward protrusions.

The patient facing surface of the connector may be substantially smooth and/or flat.

In an embodiment, the patient interface connector comprises a hinge region intermediate the first end and a bifurcation point of the connector.

In an embodiment, patient interface connector comprises a hinge region intermediate the stiffened region and the first end.

The hinge region may comprise an area of decreased material thickness. The connector may be configured such that flexing of the connector occurs at the hinge rather than elsewhere in the body of the connector.

In an embodiment, the force required to flex the connector about the hinge region is less than the force required to detach a first attachment point from the patient interface.

The hinge may be configured such that it allows the connector to accommodate face contours.

The interface attachment point may comprise a mechanical fastener. For example, the interface attachment point may comprise a hook or eye type connector pad for engagement with a complementary hook or eye connector surface on the patient interface or a frame for the patient interface. The headgear attachment points may comprise a hook or eye type connector pad for engagement with a complementary hook or eye connector surface on the headgear. The connector may comprise recesses for receipt of the hook and/or eye connector pads.

In a **fifteenth aspect,** the present disclosure relates to a component for use with a patient interface assembly and/or headgear, comprising a relatively rigid stiffening layer, and a comfort layer, wherein the comfort layer is positioned on a patient facing side of the component.

In an embodiment, the comfort layer is over-moulded or co-moulded with the stiffening layer.

The comfort layer may extend around a perimeter edge of the stiffening layer. The comfort layer may be rounded or tapered at its perimeter.

In an embodiment, an edge of the comfort layer extends above the stiffening layer to form a lip around the perimeter of the component.

The comfort layer may be shaped to form a compliant, compressible edge of the component. The compressible edge may be configured to deform by deflecting or folding to conform to a wearer's facial contours.

In an embodiment, the comfort layer comprises a fin and/or a recess adjacent the edge of the component.

The stiffening layer may be more rigid than the comfort layer.

The thickness of the fin may be selected to have sufficient strength to be self-supporting and/or to prevent inadvertent deflection of the fin towards the wearer. The thickness of the fin may also be selected such that the fin does not form a pressure point and/or a hard edge.

The stiffening layer comprises a thermoplastic material, for example, polypropylene. The thermoplastic material may be inelastic.

The comfort layer may comprise an elastomeric material, for example, silicone.

In a **sixteenth aspect,** the present disclosure relates to a patient interface and headgear assembly comprising a patient interface assembly; headgear; and a patient interface connector according to the fourteenth aspect, for coupling the patient interface assembly to the headgear, wherein a first of the two attachment points of the patient interface connector comprises a patient interface connection point for coupling to a side of the patient interface assembly, and a second of the two attachment points of the patient interface connector comprises a connection points for coupling to the headgear. The connector increases in width from a first end to a second end of the connector.

In an embodiment, the connector has two spaced apart headgear attachment points on a patient facing surface of the connector for connecting to headgear.

In an embodiment, the patient interface connection point and the upper and lower headgear connection points may comprise hook or loop connectors.

In an embodiment, the assembly further comprises a lateral arm coupling the connector to the patient interface.

In an embodiment, the assembly comprises two patient interface connectors according to the fourteenth aspect, arranged laterally, and two respective lateral arms for coupling said connectors to the patient interface.

In an embodiment, the assembly further comprises a chin strap for coupling to the headgear.

The patient interface may be a respiratory interface such as a respiratory mask or nasal cannula. In an embodiment, the headgear and the patient interface is for an infant or neonate. In an embodiment, the patient interface is a sealing interface

In a **seventeenth aspect,** the present disclosure relates to an adjustment device for gripping a flexible material; the device having a flexible body with first and second ends and first and second side walls defining an aperture for receiving the material, wherein the first and second ends are movable relative to each other to adjust the device between a locking state and a free state; wherein in the locking state the device grips the material in the aperture, to hold the device in position relative to the material, and wherein in the free state the grip of the device is released sufficiently, enabling the device to move along the material to thereby adjust the position of the device relative to the material.

In an embodiment, the device is moved from the locking state to the free state by pressing the first and second ends of the device towards each other. The device may comprise finger grips on the first and second ends of the device to facilitate pressing the first and second ends towards each other.

In an embodiment, the first and second side walls are positioned closer to each other in the locking state to the release state.

In an embodiment, the first and second side walls each have an inner surface that is convex towards the aperture. The inner surfaces of the first and second side walls may comprise one or more gripping features to enhance the strength of the grip with the received material.

In one embodiment, inner surfaces of the first and second side walls comprise oppositely projecting, complementary steps. In the locking state, the steps may urge the received material towards a bent state.

In an embodiment, the aperture narrows at or near a midline of the device. The aperture may comprise a necked region at or near a midline of the device. In one embodiment, the aperture has an hourglass-like cross-sectional shape.

In an embodiment, the device body comprises a resilient material. The side walls may comprise a resilient material. In an embodiment, in a resting state, the device is in the locking state. Additionally, or alternatively, the device may be biased towards the locking state.

In an embodiment, the device comprises hinge regions where the side walls join the ends. The hinge regions may be resilient.

In an embodiment, a length of the device is smaller at a midline of the device than at the ends of the device. The device may comprise a concave curvature on the base and/or the top of the device.

In an embodiment, the first and second ends of the device may comprise finger grips to assist a user to hold the device. For example, the finger grips may comprise protrusions or depressions.

In an **eighteenth aspect,** the present disclosure relates to a chin strap for a patient interface and/or headgear assembly. The chin strap comprises a first portion and a second portion having two arms. The chin strap is configured to wrap around a patient's head, with the arms of the chin strap engaging with a connector on the first portion of the strap.

The chin strap may comprise a first end and a second end, and a bifurcation point intermediate the first and second ends, with the two arms extending from the bifurcation point to the second end.

In an embodiment, the connector is at or adjacent the first end of the strap.

In an embodiment, the chin strap comprises two or more layers of material fused or otherwise attached together. Alternatively, the chin strap may include only a single material layer.

The chin strap may include a layer of loop-type material. The loop-type material may provide engagement surfaces on the arms by unfused regions of material. The layer of loop-type material may be a patient-facing layer. In some embodiments comprising two or more layers of material fused together, engagement surfaces may be provided on the arms by unfused regions of material.

The chin strap may include a necked region adjacent the bifurcation point and/or a notch between the two securement arms at the bifurcation point.

In an embodiment, the arms are shaped to wrap on either side of a headgear adjustment member or shaped to accommodate another feature of patient headgear. For example, the spacing between the arms may be greater towards the bifurcation point or at a point where the arms will extend around the adjustment member, and less at the second end of the chin strap. The shape of the arms may alternatively or additionally assist to accommodate shape and contours of the patient head, which may assist to stabilise the chin strap when secured to the patient.

The term 'comprising' as used in this specification and claims means 'consisting at least in part of'. When interpreting statements in this specification and claims that include the term 'comprising', other features besides those prefaced by this term can also be present. Related terms such as 'comprise' and 'comprised' are to be interpreted in a similar manner.

It is intended that reference to a range of numbers disclosed herein (for example, 1 to 10) also incorporates reference to all rational numbers within that range and any range of rational numbers within that range (for example, 1 to 6, 1.5 to 5.5 and 3.1 to 10). Therefore, all sub-ranges of all ranges expressly disclosed herein are hereby expressly disclosed.

As used herein the term '(s)' following a noun means the plural and/or singular form of that noun. As used herein the term 'and/or' means 'and' or 'or', or where the context allows, both.

### BRIEF DESCRIPTION OF THE DRAWINGS

Embodiments will now be described by way of example only and with reference to the accompanying drawings in which:
Figure 1 shows an example of an exemplary respiratory system in which embodiments of the patient interface assemblies can be used.
FIG. 2 shows an example of a further exemplary system in which embodiments of the patient interface can be used.
Figures 3A and 3B illustrate exemplary embodiment headgear and patient interface assemblies in position on a neonatal patient, where Figure 3A illustrates a system having a nasal mask with a stabilising arm, and Figure 3B illustrates a system having a nasal cannula;
Figure 4 is a top view of an assembly for a patient interface having a frame, attached conduits, and connectors;
Figure 5 is a top view of an exemplary embodiment frame for a patient interface assembly, having projections on a front face of the frame for coupling stabilising members;
Figure 6 is a rear view of the frame of Figure 5, showing the patient facing side of the frame;
Figure 7 is a front view of the frame of Figures 5 and 6;
Figure 8 is a perspective section view of the frame of Figures 5 to 7, with the projections hidden, showing the cross-sectional profile of the body member of the frame;
Figure 9 is a top view similar to Figure 5, but showing sectioning lines for Figures 10(i) to 10(viii);

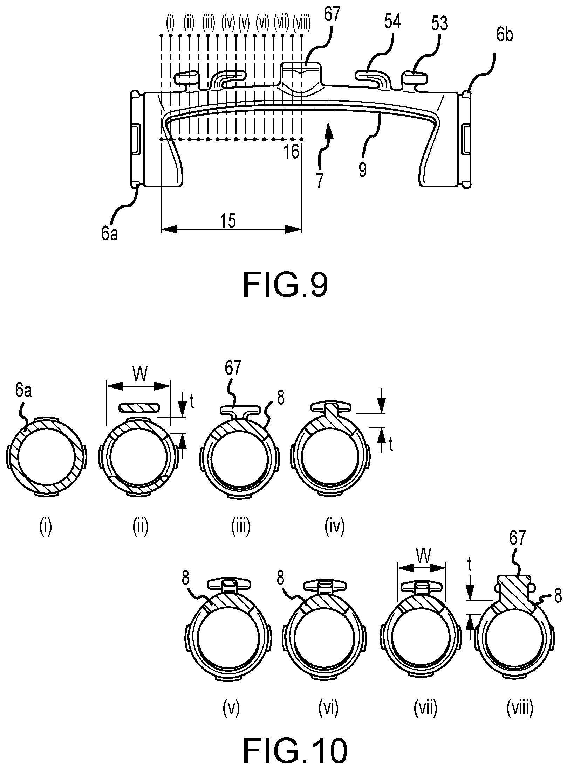

Figures 10(i) to 10(viii) are section views taken through the body member of the frame, where Figure 10(i) is a section view through line (i) of Figure 9; Figure 10(ii) is a section view through line (ii) of Figure 9; Figure 10(iii) is a section view through line (iii) of Figure 9; Figure 10(iv) is a section view through line (iv) of Figure 9; Figure 10(v) is a section view through line (v) of Figure 9; Figure 10(vi) is a section view through line (vi) of Figure 9; Figure 10(vii) is a section view through line (vii) of Figure 9; and Figure 10(viii) is a section view through line (viii) of Figure 9;
Figure 11 is a front perspective view of one embodiment patient interface body in the form of a nasal cannula;
Figure 12 is a front perspective view of one embodiment patient interface body in the form of a nasal mask;
Figure 13 is a perspective view of the mask of Figure 12 showing the different wall thicknesses at different regions of the mask;
Figure 14 is a top section view taken through a plane through a top portion of the mask of Figures 12 and 13, showing the wall profile and shape of the patient contacting portion of the mask;
Figure 15 is a side section taken through a mid-line of the mask of Figures 12 and 13, showing the wall profile and shape of the patient contacting portion of the mask;
Figure 16 is a top section view corresponding to Figure 14, illustrating movement of the mask (broken lines) upon the application of force to the patient contacting surface to one side of the mask;
Figure 17 is a side section view corresponding to Figure 15, illustrating movement of the mask (broken lines) upon the application of force to the patient contacting surface to one side of the mask;
Figure 18 is a top section view taken through a plane through a top portion of the mask of Figures 12 and 13, showing the mask worn by a neonatal patient, and also showing a prior art mask, for comparison;
Figure 19 is a bottom view showing the mask of Figures 12 to 17 being worn by a neonatal patient;
Figure 20 is a rear view of the mask of Figures 12 to 17 illustrating the shape of the patient-contacting seal;
Figure 21 is a top-rear view of the nasal cannula of Figure 11, indicating the position of a flexing region surrounding the base of the prongs;
Figure 22 is a rear-underside view of the nasal cannula of Figure 21, illustrating the position of a philtrum contacting region;
Figure 23 Figure 23 is a view corresponding to Figure 22, illustrating the wall thickness in different regions of the nasal cannula body;
Figure 24 is a front cut-away perspective view of the nasal cannula of Figures 21 to 23, illustrating the shape and location of the philtrum contacting region;
Figure 25 is a view of the nasal cannula of Figures 21 to 24, illustrating the shape of a septal relief feature between the nasal prongs;
Figure 26 is a front view of the nasal cannula of Figures 21 to 25;
Figure 27A and 27B are detail elevation views of the septal relief of Figure 23, where Figure 27A illustrates the feature for a first size of patient interface, and Figure 27B illustrates the feature for an exemplary larger size patient interface;
Figure 28A and 28B are underside views of the nasal cannula of Figures 21 to 25, where Figure 28A illustrates identifying features for a first size of the nasal cannula, and Figure 28B illustrates identifying features for a second size of the nasal cannula;
Figure 29 is a partial front section view of one side of the nasal cannula of Figure 28A, illustrating the identifying O-ring;
Figure 30 is a detail perspective view of an exemplary size identifying feature of a patient interface;
Figure 31 is a rear view of the nasal cannula of Figures 21 to 26, with assembled side O-rings;
Figure 32 is a front view of the nasal cannula of Figure 31, illustrating with broken lines where the edge of a coupling frame is configured to sit, transitioning from a larger offset at the sides of the frame to a smaller offset in the centre;
Figure 33 is a side section view of the nasal cannula of Figures 31 and 32, taken through a mid-plane of the interface;
Figure 34 is a side view of the nasal cannula of Figures 31 to 33;
Figure 35 is a side view of the nasal cannula of Figures 31 to 34 coupled to an interface frame;
Figure 36 is a side section view of the nasal cannula of Figures 31 and 34 coupled to an interface frame, the section taken through a mid-plane of the interface;
Figure 37 is a rear view of the nasal cannula of Figures 31 to 34 coupled to an interface frame, with the frame shown as transparent;
Figure 38 illustrates a first exemplary embodiment frame coupled at its sides to two conduits;
Figure 39 illustrates a collar attached to the end of one conduit, for attaching the conduit to the frame;
Figure 40 is a partial top view showing one side of the frame of Figure 38 attached to the collar and conduit of Figure 39;
Figure 41 is a top view of the connector provided at a distal end of each conduit in the embodiment of Figure 4, showing the connector coupled with the complementary receiving fixture;
Figure 42 is an exploded view of connector of Figure 42 and the receiving fixture;
Figure 43 is a side section view of the body of the connector of Figures 41 and 42;
Figure 44 is a perspective view of the body of the connector of Figures 41 and 42;
Figure 45 is a front view of a second embodiment frame with lateral connecting arms attached for securing the patient interface to headgear;
Figure 46 is a top view corresponding to Figure 45;
Figure 47 is a partial front view of the frame of Figure 45, showing the lateral arm on one side, and with a nasal cannula coupled to the frame;
Figure 48 is a side view of the assembly of Figures 45 and 46;
Figure 49 is a front view of a third embodiment frame with alternative embodiment lateral connecting arms attached for securing the patient interface to headgear;
Figure 50 is a top view corresponding to Figure 49;
Figure 51 is a top view of one of the lateral connecting arms of the embodiment of Figures 49 and 50, having a rigid end clip;
Figure 52 is a front view of the lateral connecting arm of Figure 51;
Figure 53 is a perspective view of the frame of Figures 49 and 50 illustrating the rigid end clips of the lateral arms engaged with the frame, with the other portions of the lateral arms hidden;
Figure 54 is a partial top view of side of the frame of Figures 49 and 50, showing the coupling projection for a lateral arm and illustrating the direction of movement for engaging the clip with the projection;
Figure 55 is a partial front view of one side of the frame of Figures 49 and 50, showing the shaped of the coupling projection for a lateral arm;
Figure 56 is a perspective view of a stabilising arm for coupling to the frames of Figures 4, 6, 7-10, 38, and 45-55;
Figure 57 is a top perspective detail view illustrating an aperture at a first end of the stabilising arm with side recesses for engagement with the coupling projection;
Figure 58 is a top view corresponding to Figure 57;
Figure 59 is an underside detail view illustrating an aperture at a first end of the stabilising arm with a filleted edge to facilitate location of the aperture over the coupling projection;
Figure 60 is a front view of the first embodiment frame, showing the coupling projection for attachment to the stabilising arm;
Figure 61 is a top view corresponding to Figure 59;
Figure 62 is a detail perspective view of the coupling projection of Figures 59 and 60;
Figure 63 is a perspective detail view illustrating the stabilising arm of Figure 56 aligned with the frame coupling projection for attachment;
Figure 64 corresponds to Figure 67 but showing the stabilising arm and projection engaged in the connection/disconnection position;
Figure 65 is an underside view corresponding to Figure 64;
Figure 66 is a front view of the frame of Figure 56, illustrating movement of the stabilising arm between the connection/disconnection and installed positions;
Figure 67 is a front detail view of the connection between the stabilising arm and the frame, with the stabilising arm in a position between the connection/disconnection and installed positions;
Figure 68 is a perspective detail view corresponding to Figure 67;
Figure 69 is a front view of the stabilising arm of Figure 56 coupled to the frame coupling projection in the installed position;
Figure 70 is a front detail view of the connection between the stabilising arm and the frame, with the stabilising arm in the installed position;
Figure 71 is a perspective detail view corresponding to Figure 70;
Figure 72 is a detail perspective view of the pivoting connection between the headgear connection pad and the body of the stabilising arm;
Figure 73 is an exploded perspective view corresponding to Figure 72;
Figure 74 is a detail side view of the hinge of the stabilising arm;
Figure 75 is a side view of the stabilising arm of Figure 56;
Figure 76 is a plan view of one embodiment of headgear having a fabric body with a lower headband region, showing the headgear before assembly into a bonnet and without any adjustment or end fixture components;
Figure 77 is an illustrative side view of the bonnet of Figure 76 assembled with an end fixture and worm by a patient;
Figure 78 is a perspective view of one embodiment end fixture attached to a pleated top edge of the headgear fabric;
Figure 79 is an exploded perspective view of the end fixture of Figure 78;
Figure 80 is a partial side perspective view of the assembled headgear of Figures 76 and 77, illustrating the shape of the ear regions;
Figure 81 is a partial rear-side perspective view of the assembled headgear of Figures 76 and 77, illustrating the shape of a rear portion of the headband;
Figure 82A and 82B illustrate exemplary patterns for fusing two alternative embodiment headbands, where Figure 82A illustrates an embodiment having fused portions in the form of curved and straight lines, and Figure 82A illustrates an embodiment having fused portions in the form of dots and including sizing information;
Figure 83 is a front perspective view of a first embodiment adjustment device for adjusting the size of flexible headgear such as the bonnet of Figures 76-81, showing the adjustment device in a neutral position corresponding to a locking state of the device;
Figure 84 is a rear perspective view corresponding to Figure 83;
Figure 85 is a side perspective view of the adjustment device of Figures 83 and 85, showing the device pressed into a free state;
Figure 86 is a side perspective view of a second embodiment adjustment showing the adjustment device assembled with a flexible body of headgear and in a locking state;
Figure 87 is a top view of one embodiment flexible securement member for coupling a patient interface to headgear;
Figure 88 is an underside view corresponding to Figure 87
Figure 89 is a top view of the securement member of Figure 87, but with a top layer of the member hidden, to illustrate the positioning of the intervening stiffening layer;
Figure 90 is a top view of a further embodiment securement member having a top layer comprising UBL fused to the underlying layers, illustrating one exemplary weld pattern;
Figures 91(i) to 91(iv) illustrate some alternatively shaped securement members;
Figure 92 is a top view of a chin strap for holding a patient's mouth shut;
Figure 93 is an underside view of the chin strap of Figure 92, showing the patient facing surface of the strap;
Figure 94 is a perspective view of an alternative embodiment side connector having a stiffening layer and a comfort layer;
Figure 95 is a front view of the side connector of Figure 94;
Figure 96 is a rear view of the side connector of Figures 94 and 95;
Figure 97 is a front view of an alternative embodiment side connector, showing the shape and position of a stiffening layer;
Figure 98 is a front perspective view of a further embodiment side connector having a curved profile;
Figure 99 is a rear perspective view of the side connector of Figure 98;
Figure 100 is a front and side perspective view of a further embodiment side connector, the connector having stiffening regions provided by areas of increased material thickness;
Figure 101 is a side perspective view of the side connector of Figure 100, showing the patient facing surface of the connector;
Figures 102(i) to (vi) illustrate various exemplary perimeter edge features for various side connector embodiments in which a comfort layer extends around the edge of a stiffening layer, where Figure 102(i) shows a simple rounded lip, Figure 102(ii) shows a slightly tapered lip, Figure 102(iii) shows a deep tapered lip, tapered at a steep angle, Figure 102(iv) shows a wide tapered lip, tapered at a shallow angle, Figure 102(v) shows an edge with a compliant perimeter fin, and Figure 102(vi) shows an edge with an alternatively shaped perimeter fin;
Figures 103(i) and (ii) illustrate exemplary geometry for a further embodiment of perimeter edge features;
Figure 104 illustrates various exemplary sizes and shapes for the side connectors described herein;
Figure 105 is a side perspective view of a third exemplary adjustment device for adjusting the size of the headgear;
Figure 106(i) and (ii) are a front and plan view of the adjustment device of Figure 105 respectively;
Figure 107 is a cut-away perspective view of a further embodiment adjustment device for adjusting the size of the headgear, showing internal shoulders for gripping the material of the headgear;
Figures 108(i) and 108(ii) illustrate a further exemplary adjustment device for adjusting the size of the headgear, where Figure 109(i) is a perspective view and Figure 109(ii) is a plan view;
Figure 109 is a plan view of a second embodiment of headgear having a flexible body with a lower headband region, showing the fabric base layer without any end securement;
Figure 110 illustrates the alignment of the headgear with a wearer when secured on their head, where Figure 110(i) is a side view of a patient wearing the headgear of Figure 76, and Figure 110(ii) is a side view of a wearer wearing the headgear of Figure 109;
Figures 111(i) and (ii) show two further embodiments of the headgear body, where Figure 111(i) shows an embodiment having a triangular cut-out along a square top edge of the headgear body, and Figure 111(ii) shows an embodiment in which the top edge is angled on either side of a triangular cut-out;
Figures 112(i) and (ii) illustrate an exemplary over-ear portion of headgear having a pattern of fused and unfused regions to limit stretch in selected directions;
Figures 113(i) to (iv) illustrate further exemplary patterns of fused and unfused regions to limit stretch in selected directions in the headband;
Figures 114(i) to (iii) illustrate yet further exemplary patterns of fused and unfused regions to limit stretch in selected directions in the headband;
Figures 115(i) and (ii) show a further embodiment bonnet-style headgear showing the headgear before assembly into a bonnet and without any adjustment or end fixture components, the headband region of the headgear having a stretch region provided between the over-ear portions, where Figure 115(i) is a plan view of the exterior side of the headgear and Figure 115(ii) shows the patient facing side of the headgear;
Figure 116(i) and (ii) show a further embodiment bonnet-style headgear showing the headgear before assembly into a bonnet and without any adjustment or end fixture components, the headband region of the headgear downwardly angled side portions, where Figure 116(i) is a plan view of the exterior side of the headgear and Figure 116(ii) shows the patient facing side of the headgear;
Figure 117 is a plan view of the exterior side of yet a further embodiment bonnet-style headgear showing the headgear before assembly into a bonnet and without any adjustment or end fixture components;
Figure 118(i) and (ii) are plan views of a further embodiment chin strap, where Figure 118(i) shows the patient-facing side of the chin strap and Figure 118(ii) shows the outer side of the chin strap;
Figure 119 is a left side view showing the chin strap of Figures 118(i) and 118(ii) assembled with headgear;
Figure 120 is a right side view showing the chin strap of Figures 118(i) and 118(ii) assembled with headgear;
Figure 121 is a perspective view of a further alternative embodiment side connector having a stiffening layer and a comfort layer;
Figure 122 is a front view of the side connector of Figure 121;
Figure 123 is a side view of the side connector of Figures 121 and 122;
Figures 124 (i) to (iii) illustrate various flexing directions of a stiffening layer for a side connector;
Figures 125 (i) to (iii) illustrate three exemplary embodiments for a side connector stiffening layer having slits for flexibility, with the slits extending to the periphery of the stiffening layer;
Figures 126 (i) to (iii) illustrate three exemplary embodiments for a side connector stiffening layer having slits for flexibility, with the slits set in from the periphery of the stiffening layer;
Figure 127 is a plan view of a further embodiment side connector having a stiffening layer;
Figure 128 is a side view of the connector of Figure 127; and
Figure 129 is a section view taken along line L129 of Figure 127.

### DETAILED DESCRIPTION

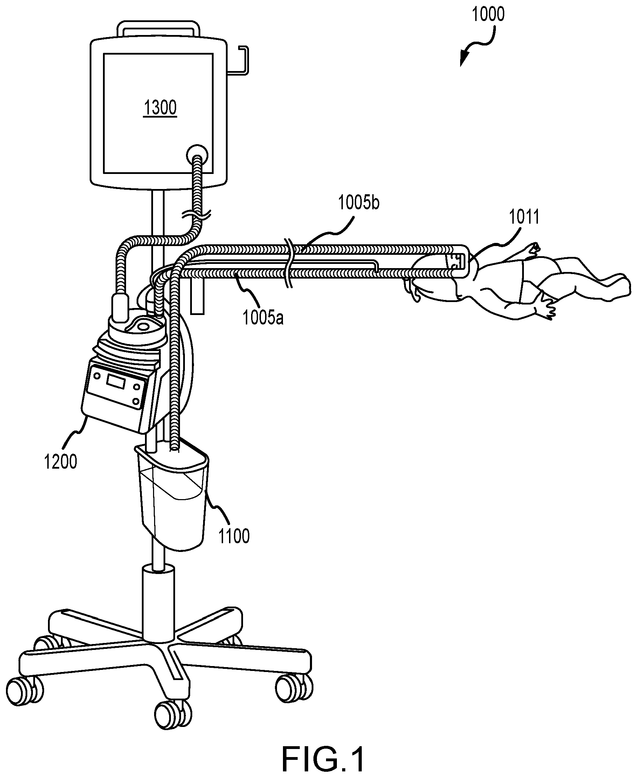

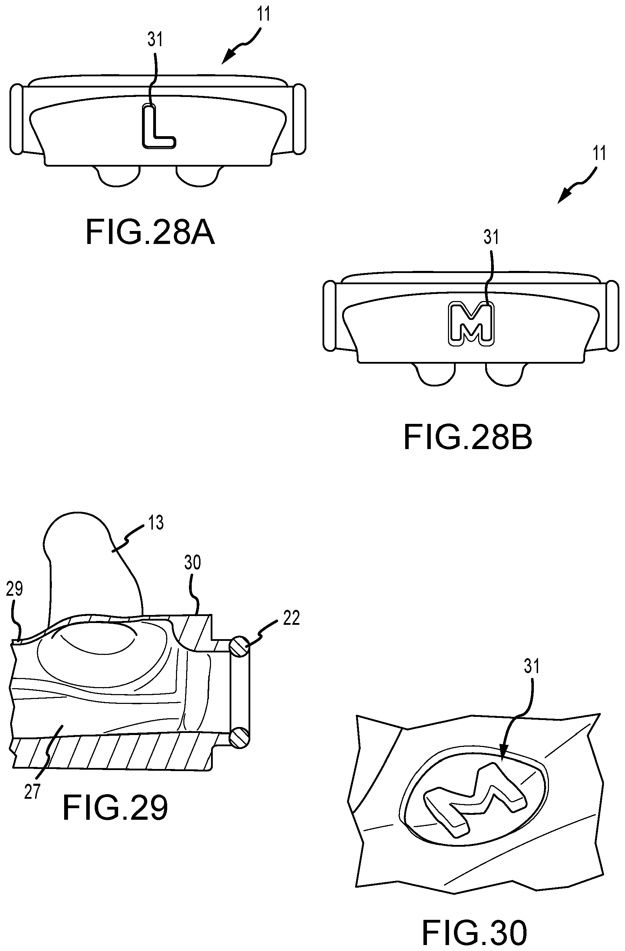

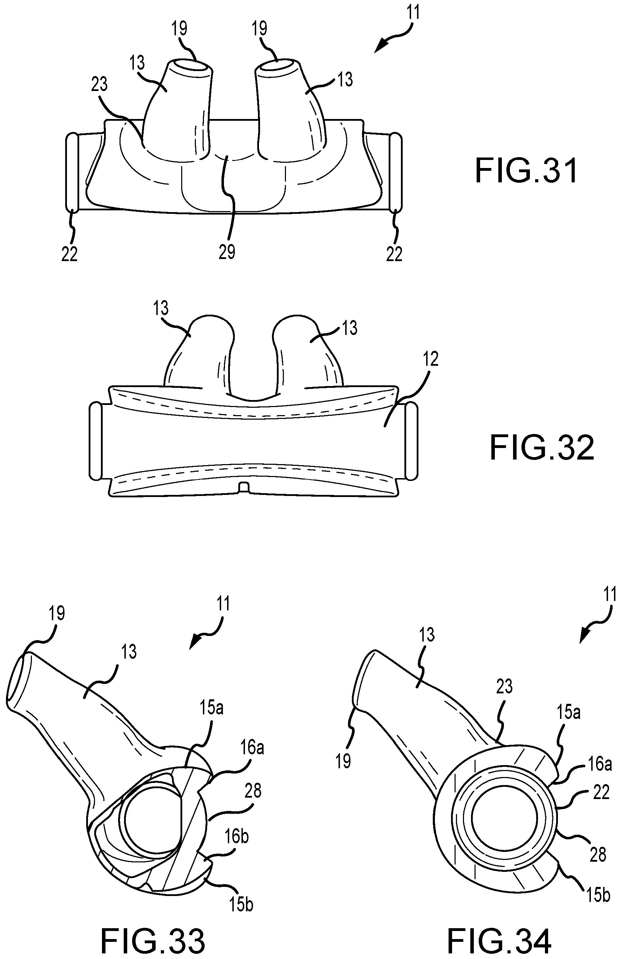

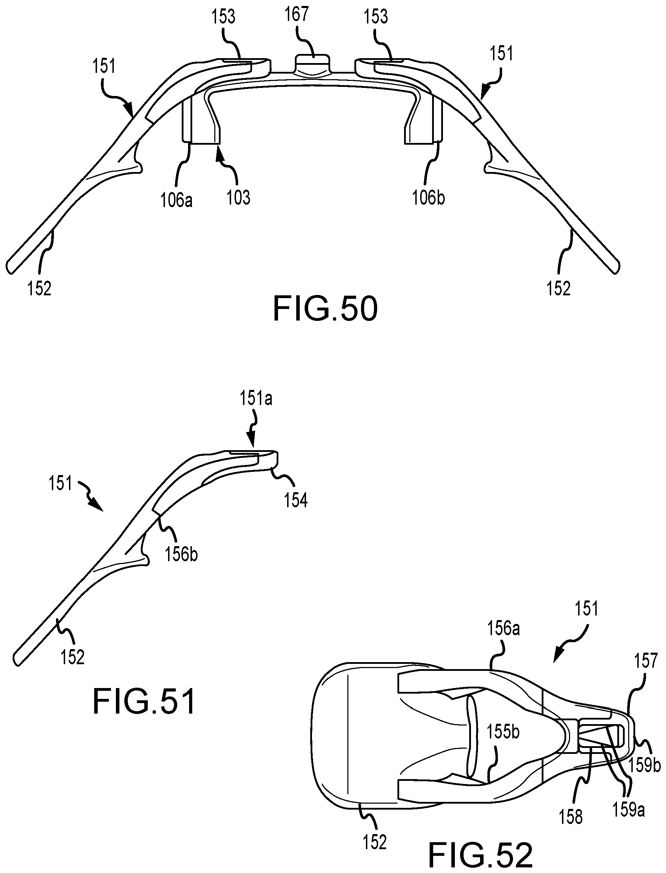

Various embodiments and methods of manufacture will now be described with reference to Figures 1 to 129. In these figures, like reference numbers are used to indicate like features. Where several embodiments are illustrated, like reference numbers may be used for like or similar features in subsequent embodiments but with the addition of a multiple of 100, for example 2, 102, 202, etc.

Directional terminology used in the following description is for ease of description and reference only, it is not intended to be limiting. For example, the terms 'front', 'rear', 'upper', 'lower', and other related terms refer to the location of a part or portion of a respiratory mask relative to a user when the user is wearing the respiratory mask. In this specification, 'rear' refers to a location that is proximal to the user (when the mask is in use) and 'front' refers to a location that is distal to the user by comparison. The terms 'upper' and 'lower' refer to the location of a part or component of a mask relative to the rest of the mask when the mask is in use and the user is sitting in an upright position.

### Respiratory System

Figure 1 shows an example respiratory system 1000 in which embodiments of the patient interface assembly 1001 described herein can be used. In the illustrated arrangement, the patient interface assembly 1001 receives an inspiratory flow of gases via an inspiratory conduit 1005a. A flow of the expiratory gases can be directed from the interface 1011 via an expiratory conduit 105b to a resistance device, which in the illustrated arrangement is a bubbler device 1100. An optional humidifier system 1200 is provided to humidify the inspiratory flow of gases.

The humidifier system 1300 typically includes a chamber sitting atop a heater base, the chamber of which is fed with a source of gases flow from, for example, a hospital or other supply source 1300. The humidified inspiratory flow of gases is delivered to the airway of the patient by inspiratory conduit 1005a and the patient interface 1011. Excess and expired gases are evacuated from the patient interface 1011 by the expiratory conduit 1005b. The resistance device 1100 provides resistance to the expiratory flow of the gases exiting the system 1000 to the atmosphere to provide a desirable peak (or positive) end expiratory pressure (PEEP). One of skill in the art will understand that such a system may include additional and/or replacement components as are known in the art.

In some embodiments, the patient interface assembly 1001 includes a nasal cannula. In other embodiments, the patient interface 1011 includes a mask. For example, the patient interface 1011 may include a nasal mask, an oro-nasal mask, an oral mask or a full-face mask. In some embodiments, the resistance device and/or the humidifier is integrated into the supply source 1300. Although a water-based resistance device is shown in Figure 1 for administering 'bubble CPAP', it should be appreciated by one of skill that the resistance device may be any other mechanical or electrical resistance device as is known in the art.

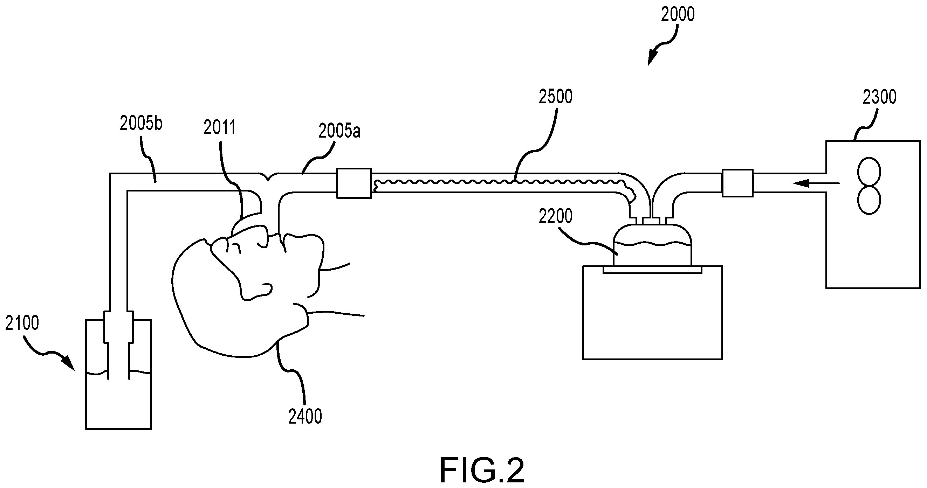

Figure 2 illustrates a further example respiratory system 2000 including a bubbler device and humidifier. A humidified Positive End Expiratory Pressure (PEEP) system is shown in which a patient 2400 is receiving humidified and pressurized gases through the patient interface 2011 connected to an inspiratory or inhalatory conduit 2005a. A flow of gases (for example air) is provided from a gases supply means or blower 2300 to the inlet of a humidifier 2200. An inspiratory conduit 2005a is connected to an outlet of the humidifier 2200 to convey the humidified gasses to the patient interface 2011 being worn by the patient 2400.The inspiratory conduit 2005a may contain heating means or heater wires 2500 that heat the walls of the conduit to reduce condensation of humidified gases within the conduit. The excess gases then flow through an expiratory or exhalatory conduit 2005b to a pressure regulator 2100.

In the embodiment shown, the pressure regulator takes the form of discharging the flow of exhalatory gases into a chamber 2100 containing a column of water, with the exhaled gases bubbling through the water before exiting the chamber 2100. The gases expired by the patient may be channelled through a similar breathing tube to other equipment (valves, ventilators, pressure devices, or the like) or expelled to the patient's surroundings. The breathing gases delivered to a patient may be heated close to body temperature (usually between 33°C and 37°C) and/or humidified to varying levels (commonly up to saturation for medical applications), to improve comfort.

It should be understood that the present disclosure, however, is not limited to the delivery of PEEP gases but is also applicable to other types of gases delivery systems and may not necessarily involve humidification.

### Headgear and patient interface system

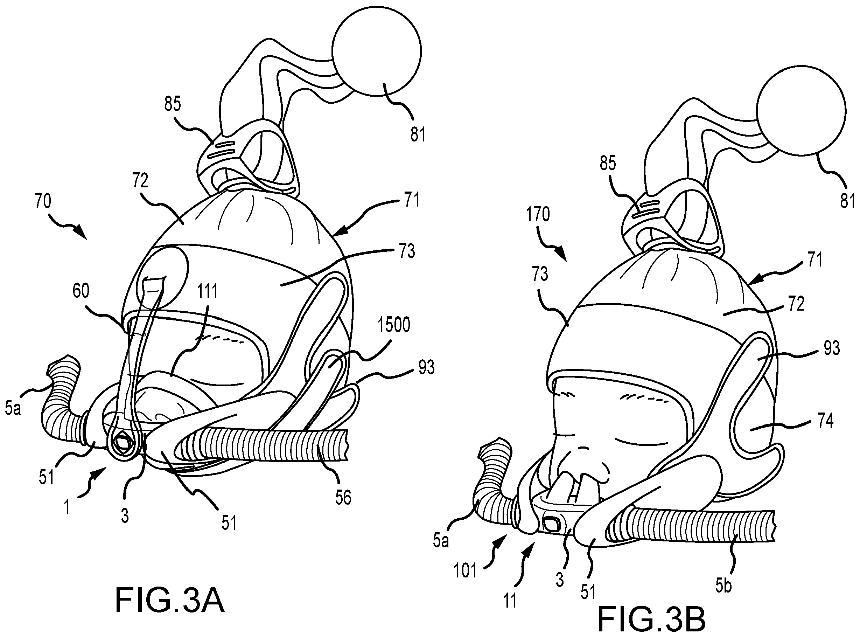

Figures 3A and 3B illustrate two exemplary embodiment patient interface assemblies 1, 101 and headgear assemblies 70, 170 for use in respiratory systems 1000, 2000 such as the systems described above. The patient interface assemblies 1, 101 comprise a frame component 3 for coupling to inspiratory and expiratory conduits 5a, 5b, and holding a patient interface body 11, 111 in fluid communication with the inspiratory and expiratory conduits 5a, 5b. The patient interface assemblies 1, 101 may be coupled to the headgear 71, for example utilising a pair of lateral arms 51 releasably connecting the frame to headgear 71, as illustrated.

The inspiratory and expiratory conduits 5a, 5b may be interchangeable with each other.

In alternative embodiments, the patient interface assemblies 1, 101 may be otherwise secured with respect to the patient's face, for example utilising dermal patches. Dermal patches adhered to a patient's skin may have a non-patient facing side with an engagement surface to releasably fasten with an engagement surface of the lateral arms 51.

In alternative embodiments, the headgear assemblies 70, 170 described herein may be utilised with patient interfaces that differ from those described in this specification.

In the patient interface assembly 1 of Figure 3A, the assembly is provided with an interface body 111 comprising a nasal mask for an infant or neonate. In the patient interface assembly 101 of Figure 3B, the interface body 11 comprises a nasal cannula for an infant or neonate, however other patient interface types are possible. The frame and interface body assembly enables ready assembly and disassembly of the patient interface, and thereby enables a user or a clinician to swap between different types of interface bodies (e.g. between the cannula body and the mask body) or between interface bodies of different sizes to improve the fit of the interface, by interchanging the interface body coupled to the frame.

The headgear in the embodiment shown is in the form of a wrap-around bonnet 71, having an end securement fixture 81 and an adjustment device 85. The headgear includes a headband region 73 with an over-ear portion 74 that extends at least partly over a wearer's ears. The headband 73 includes an engagement surface for attaching connectors 93 to secure the patient interface assembly 1, 101 with respect to the headgear.

The headgear may be used with various types of patient interfaces, including those shown, for varying respiratory therapies. For example, it may be used with interfaces for the delivery of CPAP, or with an interface configured to deliver high flow therapy. The nature of the headgear connectors required will generally depend on the nature of the patient interface. In the embodiment of Figure 3A, two Y-shaped or wishbone-shaped side connectors 93 are provided to couple the lateral arms 51 of the patient interface assembly to the over-ear region 74. These side connectors 93 may be used alongside an optional central securement member 60 that attaches between a front surface of the frame 3 and a point on the front of the headband 73. In the embodiment of Figure 3B having a nasal cannula, only two Y-shaped or wishbone-shaped side connectors 93 are used to couple the patient interface assembly 101 to the headgear. The side connectors may have other shapes than those shown in these Figures, for example as discussed further below.

For some applications, a chin strap may be provided to hold or encourage the patient's mouth closed, for example for the delivery of CPAP.

### Patient interface frame

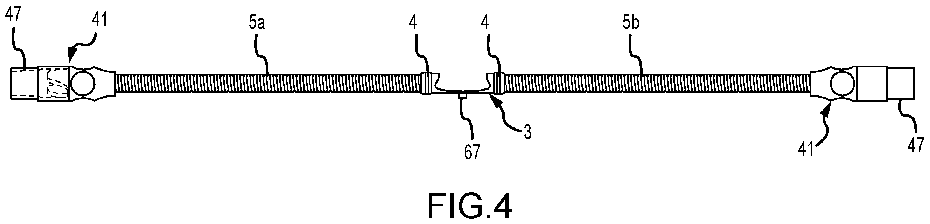

Referring now to Figures 4 to 10(viii) a frame 3 is provided for coupling an interface body 3 to the inspiratory and expiratory conduits 5a, 5b. The frame 3 comprises a coupling portion 7 for engaging with a complementary coupling portion of an interface body, to securely and releasably retain an interface body with respect to the frame and in fluid communication with the conduits 5a, 5b.

The coupling portion 7 of the frame 3 comprises two end regions 6a, 6b for coupling to respective conduits. A body member 8 extends between the end regions 6a, 6b. The body member 8 may be a contoured body member 8. The body member 8 is provided at a front of the frame 3, forming a bridge between the two end regions 6a, 6b. The body member 8 has a contoured inner surface for contacting a complementary coupling portion of an interface body, for example shaped to sit flush against a substantially cylindrical barrel, and concave sides 9 for engaging a complementary convex coupling surfaces on the interface body 11, 111.

With particular reference to Figures 9 and 10, both the width of the body member 8 and the wall thickness of the body member 8 varies along a length of the body member 8, that is, from proximal the frame first end region 6a to proximal the frame second end region 6b. The change in width and thickness along the member 8 is gradual, preferably without abrupt changes.

The width of the body member 8 is widest proximal the body member ends, proximal the first and second end regions 6a, 6b of the frame, and tapers to a narrowest point at a mid-line ML of the frame 3. The tapering is non-linear so as to form two convex sides 9 to the body. The frame member 3 is typically a rigid component. It is desirable to minimise or avoid contact between the frame member 3 and a patient, to reduce the risk of damage to facial tissue. This curved profile of the body member 8 advantageously increases the clearance between the frame components and a patient's face, reducing the chance of inadvertent contact between the frame and a patient. The contoured side may provide additional space for flexing of patient interface bodies for improved fit with a patient. The contoured side may also enable the coupling of patient interface bodies with enhanced relief features. In particular, the contoured side may provide space to enable the frame to couple with a nasal cannula having a septal relief recess, and/or to provide clearance for a patient's septum.

In the embodiment shown, first and second end regions 6a, 6b of the frame are annular in cross section. The width (taken to be the cross-sectional chord length of the body member) at the ends of the body member 8 is less than the diameter of the end regions 6a, 6b of the frame. At a mid-line ML of the body member 8, the width of the body member 8 is about 50% of the diameter of the end regions 6a, 6b of the frame, however other widths are possible, for example between about 35% and about 90% of the diameter of the end regions.

Referring to the section views of Figures 10(i) to 10(viii), the wall thickness, t of the body member 8 varies from a thinnest point adjacent the ends of the body member, where the member is widest (Figure 10(ii)), to a thickest point adjacent or at a mid-line of the frame 3 where the member is narrowest. This increase in wall thickness provides additional stiffening to compensate for the reduction in material from the reduction in width of the member. The projections 53, 54, 67 on the front surface of the frame, for attaching securement members or connectors, are not considered to form part of the wall thickness of the frame body member.

In the embodiment shown, the change in wall thickness is provided by a body member 8 having a different surface curvature on an outer surface of the member compared to an inner surface of the member. As illustrated by Figures 10(ii) to (viii), the cross-sectional profile of the frame body 8 is generally arcuate. In the embodiment shown, the curvature of the inner surface of the body member 8 is substantially unchanged along the length of the body member 8, but the curvature of an outer surface of the body member 8 varies from a largest radius of curvature adjacent the ends of the body member, to a smallest radius of curvature at or near the midline ML, having the effect of increasing the wall thickness.

Figure 10(ii) shows that adjacent the end regions of the frame 6a, 6b, the outer and inner surfaces of the body member 8 are concentric. In comparison, Figure 10(viii) shows that at or near the midline ML, the outer and inner surfaces of the body member 8 have non-concentric curvature such that the thickness of the body member varies along the cross-sectional profile. For example, the thickness of the body member is greater in a middle of the cross-section and tapers outwards towards ends thereof.

In alternative embodiments, the body section may alternatively or additionally comprise one or more stiffening features to compensate for the reduced width in the centre of the body member, for example, one or more ribs or webs on an outer or inner surface of the body member. A suitable rib may be integrally formed with the body member or permanently bonded. The shape, height, and/or width of the rib may vary along the length of the rib, for example, to provide the required strengthening.

### Patient interface body

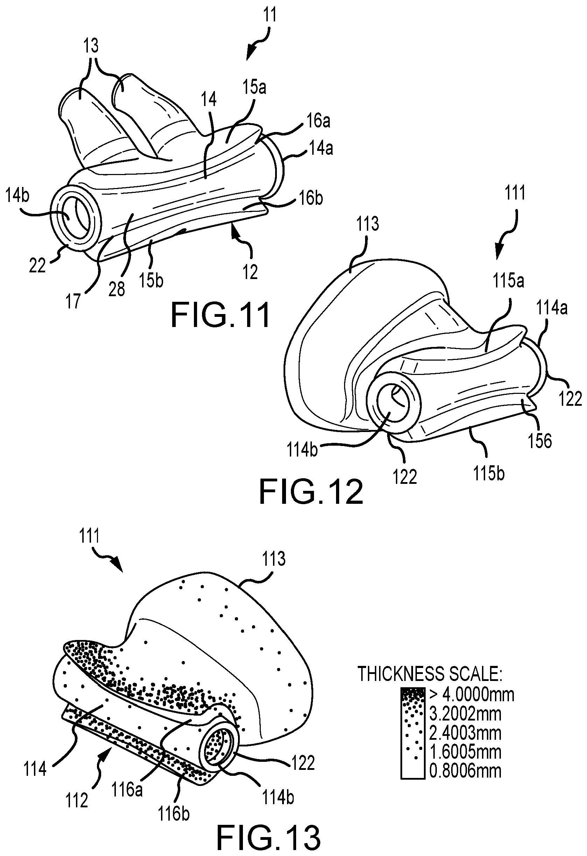

Figures 11 to 13 illustrate two exemplary embodiment patient interface bodies 11, 111. The interface body of Figure 11 has the form of a nasal cannula, while the interface body of Figure 12 has the form of a nasal mask. The interface bodies 11, 111 comprise a patient-contacting portion 13, 113 and a coupling portion 12, 112, shaped to couple with the coupling portion 7 of the frame 3. The patient contacting portion 13, 113 may form a full or partial seal with the face of the patient.

The patient contacting portion 13, 113 extends from the rear (patient facing) side of a manifold 14, 114. The manifold 14, 114 defines a pair of openings 14a, 14b, 114a, 114b for fluid communication with the inspiratory and expiratory conduits 5a, 5b. The manifold openings are provided on opposite sides of the manifold for receipt by the end portions 6a, 6b of the frame 3. The openings 14a, 14b, 114a, 114b allow for lateral flow of fluid into and out of the manifold. The patient contacting portion 13, 113 is fluidly coupled to the manifold, for example by a rear opening in the manifold, to facilitate the flow of the gas to and from the manifold and a patient via the patient contacting portion.

In the embodiment shown, side ends of the manifold are annular for coupling with the annular end portions 6a, 6b of the frame 3. A front portion of the manifold has a front outer surface extending between the end portions shaped to couple with the frame 3, with a generally semi-cylindrical front surface.

Annular members 22, 122 may be provided at the interface body end portions 6a, 6b, for forming a seal with the interface frame 3. The annular members are raised rings with respect to the manifold and configured to provide a friction or interference fit with an interior surface of the frame 3 to provide a fluid-tight seal with the frame.

These annular members 22, 122 may be coloured members for use with a transparent or translucent frame or frame portion to provide a visual indicator of correct assembly between the interface body and the frame. A user or clinician may observe the position and configuration of the coloured ring through the transparent or translucent frame. In the embodiment shown, correct positioning and coupling of the frame and patient interface body is indicated by observing that the coloured ring is in axial alignment with the frame 3 and forms a circle. An incorrect positioning is indicated by observing that the coloured ring is deformed or out of alignment with the frame 3, also indicating that the gas seal between the frame 3 and interface body 11 may be deficient.

Additionally, or alternatively, differently coloured annular members can function as an indication of the size of the interface body. The interface body may alternatively or additionally include a sizing indicator 31 on another portion of the body such as towards a centre of the manifold. The coupling portion 12, 112 of the interface body 11, 111 comprises top and bottom stiffening regions 15a, 15b, 115a, 115b on top and bottom portions of the manifold 12, 112. Each stiffening region 15a, 15b, 115a, 115b has a convex coupling wall 16a, 16b, 116a, 116b that projects up from the front outer surface of the manifold.

The stiffening regions 15a, 15b, 115a, 115b provide stiffness to the coupling portion of the interface body to provide easier handling, assembly, and reduce the likelihood of unintentional disassembly of the patient interface body 11, 111 with the frame 3.

The projecting convex coupling walls of the stiffening regions 15a, 15b together with the front outer surface of the manifold 14 define a recess 17 to receive the body member 8 of the frame 3. The recess has a shape that corresponds to the shape of the frame body member 8 and the two convex sides 9 of the body 8, with a width of the recess varying along the recess, from a first side of the interface body to a second side of the interface body. The width of the recess is widest proximal the sides of the interface body, and tapers to a narrowest point at a mid-line ML of the interface body. The tapering created by the two facing convex coupling walls of the stiffening regions 15a, 15b is non-linear.

The complementary shapes of the recess and frame body member assists with correct assembly of the interface body with the frame and reduces the chance of rotational misalignment. In the present embodiment, the frame is symmetrical about a transverse plane such that the interface may be correctly installed in the frame facing up or down. In alternative embodiments where the frame requires a particular orientation of the interface body for correct operation, top and bottom edges of the frame body may have different curvatures, and the same asymmetrical curvatures provided on the coupling portion of the interface body, to ensure the interface can only be installed one way.

With the frame 3 and interface body 11, 111 engaged, the inner surface of the frame body member 8 may generally lie against the front outer surface of the manifold 14, or may generally follow the curvature of the front outer surface of the manifold 14 with a small spacing between the components for clearance. In one embodiment, the clearance may be 0.1mm to 0.2mm. However, at least a portion of each coupling wall 16a, 16b, 116a, 116b is configured to sit proud of the front surface of the frame body member 8 when assembled. The coupling walls may sit proud of the front surface of the frame body member 8 by a distance that remains generally constant across the frame body member. Alternatively, the coupling walls may sit proud of the front surface of the frame body member 8 by a distance that varies across the frame body member 8.

Figures 13 to 17 illustrate the varying wall thicknesses over the body for the nasal mask interface body 111. The coupling walls 116a, 116b of this embodiment 111 are shaped to have a height of between about 2 mm and about 3.5 mm from the front surface of the manifold 114 such that when coupled with the frame 3, they sit proud of the front surface of the frame body member 8 by a constant distance of about 0.9 mm across the frame body member. In alternative embodiments, the coupling walls 116a, 116b may sit between about 0 mm and about 2 mm proud of the front surface of the frame body member 8. They may be proud of the body member 8 by a constant distance, or the distance may vary across the body member.

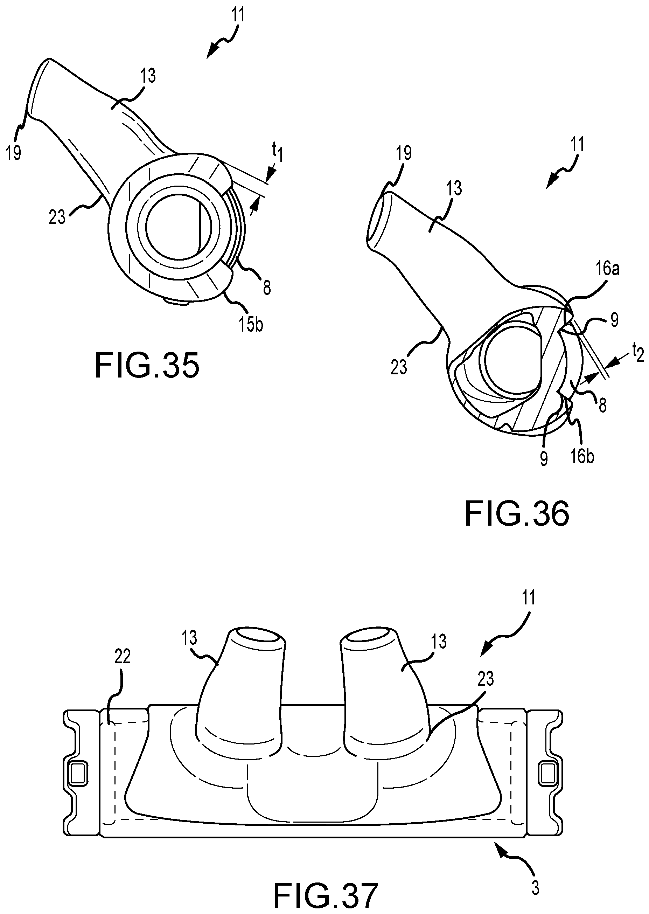

In contrast, Figures 35 and 36 illustrate the frame body member 8 seated in the coupling portion recess of the exemplary nasal cannula 11, showing the fit at a midpoint of the frame and interface body (Figure 36) and at one side of the interface body (Figure 35). The coupling walls 16a, 16b in this embodiment are configured to sit proud of the front surface of the frame body member 8 by a distance that varies across the frame body member, transitioning from about 0.9 mm proud proximal the sides of the frame 3 to about 0.3 mm proud at the center. In alternative embodiments, the coupling walls 16a, 16b may sit proud of the front surface of the frame body member 8 by a distance that varies across the frame body member, transitioning from up to about 2 mm proud proximal the sides of the frame 3 to at least 0 mm proud at the center. In alternative embodiments, the coupling walls 16a, 16b may sit proud of the front surface of the frame body by a constant distance across the body. It will be understood that these are exemplary dimensions and not intended to be prescriptive and that many variations to these dimensions are envisaged.

The portion of the patient interface coupling portion that is proud of the frame body 8 helps to reduce the risk of mis-assembly and aids in retention of the connection between the interface coupling portion and the frame.

The thickness of the stiffening regions 15a, 15b, in the cannula interface body 11, and thereby the height of the coupling walls 16a, 16b, may vary across a width of the interface body 11. In the interface body 11, exemplified, the height of each coupling wall 16a, 16b, is larger towards the sides of the interface body, and smaller towards a midline of the interface body.

The thickness of the stiffening regions 115a, 115b, in the mask interface body 111, and thereby the height of the coupling walls 116a, 116b, is substantially constant or may vary slightly across a width of the interface body 111.

### Nasal mask

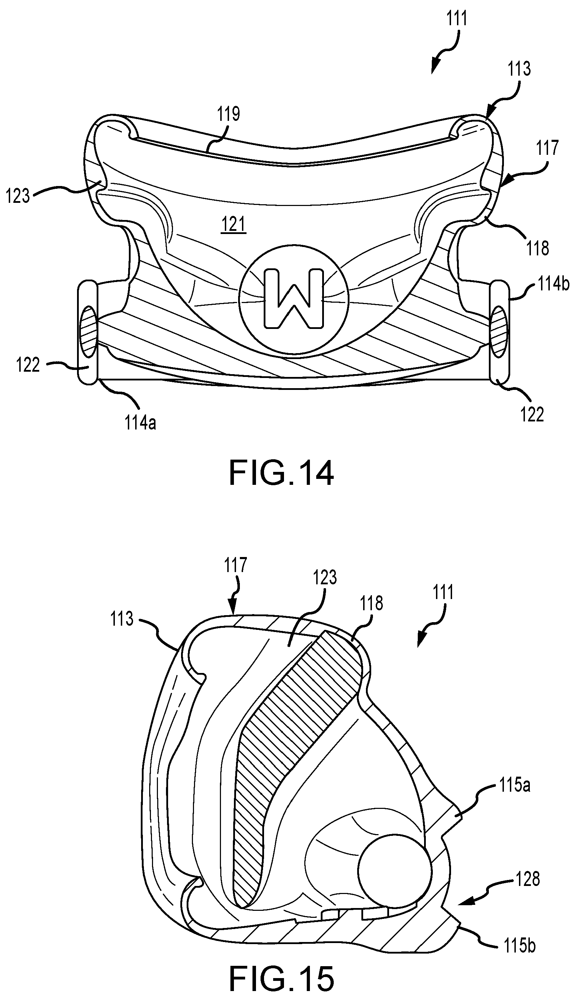

Figures 14 to 20 further illustrate the exemplary nasal mask embodiment of the interface body 111. The interface body 111 includes a mask cushion, extending rearward from the frame coupling portion 112 and manifold. The mask 111 may be applied on the face of a patient, forming a seal on or about the nose surrounding the nares of the patient. In alternative embodiments, the mask 111 could cover both a nose and a mouth of the patient.

The mask cushion includes a substantially hollow body defining a mask cavity 121, and a face-contacting surface 113 located generally opposite the frame coupling portion 112, with a peripheral edge 119 face-contacting surface 113 defining an opening. The mask cavity 121 is configured to receive the flow of gases from the manifold such that these gasses can flow through the opening to the patient.

This face contacting surface 113 sits against a wearer's face, surrounding a portion of the patient's nose, including the nares. The contacting surface 113 may sealingly engage about the user's nose such as against one or more of the cheek surfaces and/or lateral side surfaces of the user's nose, the upper lip region below the user's nose, and across the nasal bridge region or nasal tip region of the user's nose. Alternatively, the sealing region at the apex of the mask may be positioned at or near the transition from nasal bone to nasal cartilage such that at least part of the sealing region is over the nasal bone.

The mask cushion includes a "rolling region" 117. The rolling region 117 is configured to roll or bend over onto an outer surface of the mask to allow the mask to accommodate facial movement or application of forces on the mask, and to accommodate different nose geometries. Rolling of the rolling region 117 assists to relieve pressure applied to the users nose and/or face, by rolling more in regions of increased pressure. The rolling region can also assist to maintain the integrity of the seal between the mask and the patient by enabling the mask to conform to a patient's face.

In the embodiment shown, the rolling region 117 extends along upper and side portions of the mask 111. When the mask 111 is seated on the face of the user in use, the contacting surface 113 may lie over the bridge of the nose, sides of the nose and above the upper lip of the user. With a supply of positive pressure air, the contacting surface 113 may balloon and seal against the face of the user.

The wall thickness and/or stiffness of the mask 111 in and adjacent to the rolling region 117 of the mask is selected to provide the desired rolling and deflection characteristics. To assist with the rolling or bending, the region 117 can have a varying thickness or a varying stiffness. For example, the thickness selected may determine the degree of movement offered by the mask. Further, varying stiffness of the regions can create hinge points or pivot points which may direct movement of the mask.

In the embodiment shown, the rolling region 111 comprises thin regions at and adjacent the patient contacting surface 113, and at a front region 118 of the rolling region, adjacent the manifold, along the top and sides of the mask. These thin regions facilitate bending or folding of the mask 111.

The reduced stiffness at the patient contact surface enables the surface to conform to the patient's face. In addition, the reduced stiffness and/or thickness at the front region (top and sides of the mask) enables a rolling motion of the mask, with a substantially flat force-displacement curve. When the mask cushion is compressed, such as when the mask 111 is pushed towards the patient face in use, the rolling region 117 may roll forwards from a pivot or hinge point at or adjacent the base of the mask.

The mask 111 comprises a stiffened structural portion 123 in between the two regions 119 and 118. The stiffened structural portion 123 can be stiffer or formed of a thicker material than the adjacent regions 118, 119. The structural portion 123 may be continuous or unbroken around the circumference of the mask or may extend partially around the circumference of the mask. In the embodiment shown, the structural portion 123 extends around the sides and top of the mask. In alternative embodiments, the structural portion 123 may extend around the sides, top and base of the mask. The structural portion 123 may be a band.

In the embodiment shown, the wall thickness of the patient contacting surface 113 is thinner than the wall thickness at the strengthening region 123. The transition from the strengthening region 123 to the patient contacting surface 113 is a gradual transition comprising a gradual tapering of the wall thickness. There is preferably no abrupt change in wall thickness from the strengthening region 123 to the patient contacting surface 113. A smoother transition between these regions reduces the likelihood of an edge of the strengthening region from contacting the user's skin and/or nasal bridge as the mask deforms to seal on the patient.

For the transition from the strengthening region 123 to the front rolling region 118, it may be less necessary that this is gradual, as the front edge of the strengthening region is not patient-facing.

The structural portion 123 can be positioned to affect the force-displacement profile of the rolling region at the top and/or side. For example, the force profile may be relatively flat as the thin material of the reduced stiffness section rolls until the thickness of the rolling material begins to increase (either abruptly or gradually). The increase in the thickness of the rolling material can then ramp up the force-displacement profile. The gradual thinning or gradual reduction in stiffness of the structural portion 310 can be used to allow the rolling region to roll for a certain distance before gently resisting displacement rather than going from a low force to a high force when the rolling region stops rolling.

In some cases, such as at higher pressures, the structural portion 123 may help prevent the mask from inflating. The structural portion 123 can also help to prevent folds or creases in the thin material sections of the regions 118, 119.

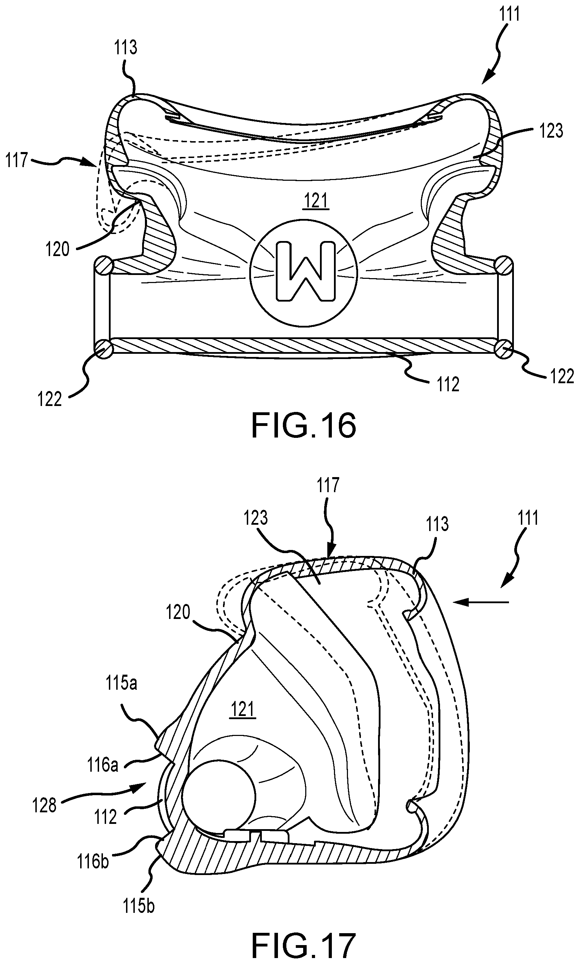

The mask can roll at each side, independently or together, and pivot about a point at or adjacent the base of the mask. As shown by the broken lines in Figures 16 and 17, the rolling region 117 can deflect around a front edge 120 of the rolling region 117. As shown in Figure 17, the mask 111 is configured to hinge at the base of the mask such that there is higher displacement towards a top of the mask.

The material thickness of the mask wall can thicken or stiffen as it moves forward of the front edge 120 of the rolling region. In some embodiments, the mask can have a thickened or stiffened base that can add structure to for example, prevent the mask from folding in half laterally.

In some embodiments, the reduced stiffness section of material can have a thickness of approximately 0.5mm or less. The reduced stiffness section of material can have a thickness of 0.8 mm or less, 0.7mm or less, 0.6mm or less, 0.5mm or less, 0.4mm or less, or 0.3mm or less. In some embodiments, the ratio of thickness of the structural portion 123 to the reduced stiffness section can be about 20:1, 15:1, 10:1, 5:1, 2:1, or 1.5:1.

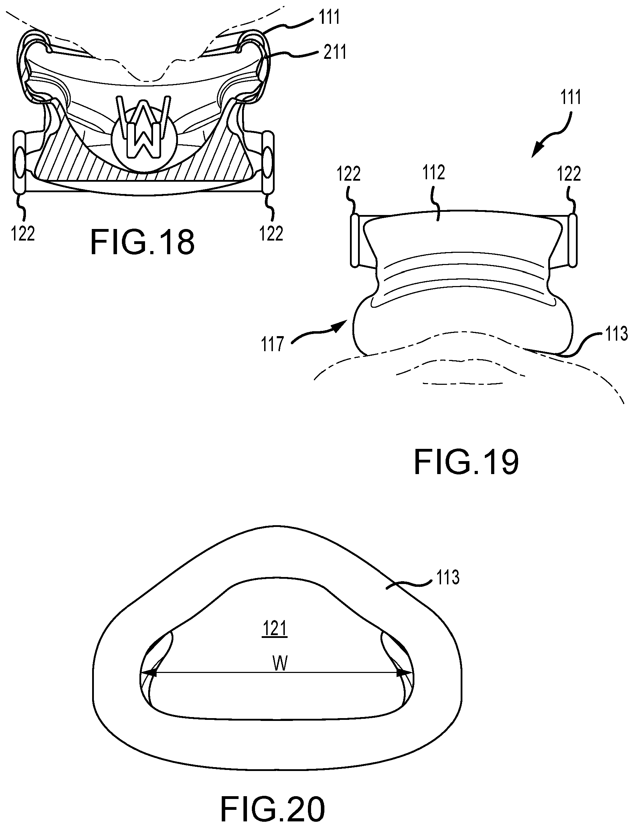

Referring to Figure 18, the patient contacting surface 113 may have a curvature in a transverse plane to provide good conformity with the transverse curvature of a patient's face, in particular to the facial curvature of an infant or neonate. Figure 18 compares the curvature of two example embodiments - the presently described embodiment 111 and an alternative mask 211, which is described in WO2021176338.

A greater transverse curvature of the mask may improve the contact of the mask with the patient, particularly over the patient's cheeks. In the present embodiment 111, the curvature is such that the forwards-rearwards depth of the patient contacting surface 113, measured from the forward most point of the surface 113 at the midline of the mask, to the rearmost points at the lateral regions of the surface 113 mask is between about 0.5 mm and about 2mm more than the depth of the contact surface in the embodiment exemplified in WO2021176338. In one embodiment the forwards-rearwards depth of the patient contacting surface 113 is between about 1 mm and about 1.6 mm more than the depth of the contact surface in the embodiment exemplified in WO20211763. Referring to Figure 20, the opening of the mask chamber 121 has a width W of between about 10 mm to about 40 mm, depending on the mask size. However, other sizes are envisioned.

The mask interface body 111 can be constructed from a single material or from two or more different materials. The mask body 111 can be constructed from silicone.

### Nasal cannula

Figures 11 and 21 to 34 illustrate a further embodiment patient interface body 11 in the form of a nasal cannula. The cannula 11 comprises a pair of prongs 13 that extend from a rear portion of the frame coupling portion 12 and manifold 14, towards the patient. Each nasal prong 13 may be shaped to extend from the manifold 14, generally upwardly and rearwardly into a user's nares. Each nasal prong 13 may have a curvature that includes one or more inflection points.

The nasal prongs 13 are shaped to form a seal with nares of a patient's nose and allow gas flow from and to the user. For example, the curvature, wall thickness and/or the cross-sectional shape of the prongs may vary from a base of each prong 13 to the tip 19. In some embodiments, the nasal prongs are shaped for sealing in an infant or neonate's nares. The prongs 13 can be shaped and formed to minimize tissue compression and kinking of one or more of the prongs 13 during insertion into a patient's nares. In the embodiment shown, the wall thickness of the prongs is substantially constant from a base of the prongs 13 to the tip 19.

The prongs 13 may taper inwardly from or a base of each prong 13 to the tip 19. The prong cross-sectional area may gradually diminish from or between a base of each prong 13 to the tip 19. This prong tapering may aid in the sealing function of the prongs 13. When the prongs 13 are pushed into the patient's nares, they may seal somewhere along the length of the prong 13 due to tapering in which the prongs 13 widen towards the base 23. The tapering may aid in insertion of the prongs 13, as opposed to prongs which are of a constant cross-sectional area, or prongs which widen towards the outlet.

Each nasal prong 13 defines a lumen, the internal cross-section of which varies along the length of the nasal prong 13. In the embodiment shown, the outlet of the prong lumen at the prong tip 19 is substantially circular. In some embodiments, the outlet of the prong lumen can be substantially elliptical. In the embodiment shown, the cross-sectional dimensions generally decrease along the trajectory from a base of each prong 23 to the tip 19. The cross-sectional shape may also change.

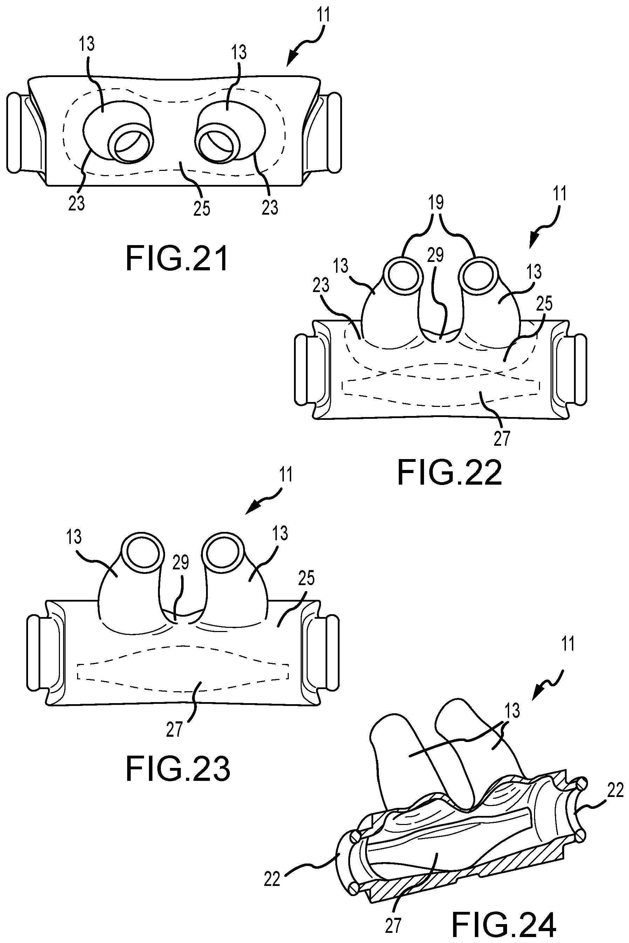

The cannula body 11 comprises a flexing base region 25 (indicated by the broken lines in Figure 21) at and/or adjacent the base 23 of the nasal prongs 13. The base region 25 can form a portion of the cannula body 11 where the nasal prongs 13 meet the manifold 14. The base region 25 may assist to provide comfort and conformability to the patient while also ensuring the flow path is maintained between the manifold and nasal prongs.

In the embodiment shown, the flexing base region 25 comprises a region having reduced wall thickness relative to the base of the prongs 13 and the rest of the manifold 14. The reduced wall thickness region 25 may fully or partially surround the base 23 of each nasal prong and may extend between the nasal prongs 13. In the embodiment shown, the base region 25 has a generally reniform shape as illustrated in Figure 21, forming a band of reduced thickness material around the base of each prong and between the prongs.

The reduced wall thickness region creates a spring effect at the base of the prongs 135. The thinner region of material may be partly or wholly formed in the base of the nasal prongs.

In some embodiments, the wall section of the prongs 13 transitions towards the thinner wall of the base region 25 around the prongs. This base region 25 can have a substantially constant thickness throughout the base region. In some embodiments, the thickness of the prong 13 adjacent the base 23 can be between 0.3 mm - 2.0 mm, 0.5 mm - 2.0 mm, 0.6 mm - 2.0 mm, 0.3 mm-1.8 mm, 0.5 mm - 1.8 mm, 0.6 mm - 1.8 mm, 0.7 mm - 1.8 mm, 0.6 mm - 1.5 mm, or 0.7 mm - 1.5 mm. The thickness of the base region 25 can be between 0.1 mm-1.5 mm, 0.2 mm-1.5mm, 0.2mm-1.3mm, 0.3 mm-1.3 mm, 0.5 mm- 1.2 mm, or 0.5 mm - 1.0 mm. For example, in one embodiment, the thickness of the prong 13 can be about 0.6 mm and the thickness of the base region 25 can be about 0.3 mm. In such an embodiment, the ratio of the thickness between the prongs 13 to the base region 25 can be 1:0.5. The ratio of thickness between the prongs 13 to the base region 25 can be any other ratio that provides the desired transition therebetween.

The flexing base region 25 may provide a spring action or mechanism at the base of the prongs 13. The base region 25 can assist to decouple movement of the housing and/or manifold 14 from the movement of the nasal prongs 13. The base region 25 may provide the ability for the prongs 13 to flex about the base region 25 whilst avoiding kinking of the prongs 13. The prongs 13 may flex whilst maintaining position in the nares. For example, the base region 25 can provide a spring-like movement at the base of the prongs 13 and/or the area surrounding the base of the prongs 13. The base region 25 can apply a spring load to the prongs to absorb some interface movement whilst maintaining the prongs 13 in position in the nares. As an example, the base region may initially be in a neutral/relaxed position (i.e. natural state of the cannula body) without any forces applied to any part. When inserted in the nares, the action of pushing the prongs into position, (and sealing) can cause the base region 25 to flex or deflect downwardly into the manifold. Prong position in the nares may remain constant, particularly once the prongs are in a sealed position. If, in use, the housing and/or manifold is pulled downwards and/or away from the face (e.g. by pulling of tubes), the base region 25 may move towards the neutral position. There can still be enough elasticity in the base region 25 to maintain the prong position in the nares. For example, the spring load from the base region 25 can absorb interface movement such as cheek movement, patient movement, or headgear movement. The base region 25 can assist movement of the or each prong 13 in multiple directions to accommodate movement of the interface, whilst also maintaining a seal sufficient to maintain delivery of therapy.

The flexing base region 25 may assist to accommodate varying septum spacings. The base region 25 may allow one or both prongs 13 to flex back and forth, towards and away from the face, to accommodate different facial geometries. The base region 25 may alternatively or additionally provide an upwards push into the nares to maintain a constant seal.

In some embodiments, the base region 25 may be shaped to define a recess at a septal region 29 between the prongs 15. This recess 29 is configured to provide for clearance of a patient's septum to avoid or reduce pressure on the septum and thereby minimise the risk of skin or tissue damage. The recess 29 may also improve visibility of the patient's nares and/or septum.

The septal recess 29 is defined by a surface of the base region 25 between the prongs 15 that extends forward of the base 23 of the prongs, and forward of the patient contacting or cheek contacting surfaces 30 to the sides of the interface body. This recess 29 has a curved profile, formed by a concave surface of the base region between the prongs 15. The wall thickness of the base region 25 at the septal recess 29 may be substantially constant, or it may vary.

In use, due to the flexing base region, the prongs 13 may depress towards or into the manifold 14. In the absence of an adequate septal recess 29, this may cause contact and/or increased pressure between a patient's septum and the interface. Therefore, it is desirable for the depth t1 of the septal recess to be as large as possible without adversely impacting resistance to flow through the gas path of the prongs and without compromising the stiffness of the coupling portion 12 of the interface body 11.

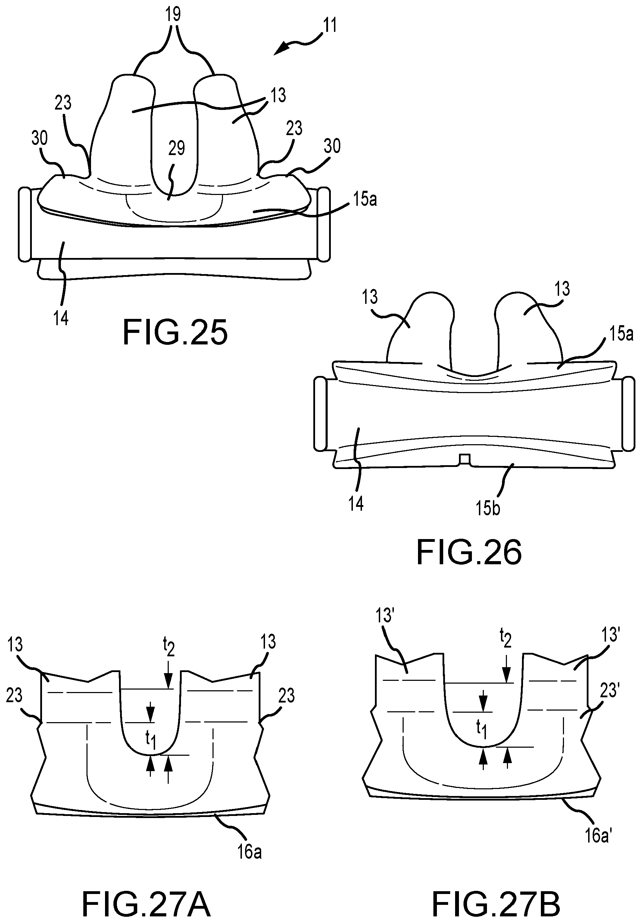

Figures 27A and 27B illustrate indicative geometries for the septal recess 29 in two different sizes of interface bodies. In the smaller size illustrated in Figure 27A, the septal recess 29 has a depth t1 of about 1.9mm forward from a base 23 of the prongs. This equates to a distance t2 of about 3.8mm from a lower sealing portion of the prong. In the larger size illustrated in Figure 27B, the septal recess 29 has a depth t1 of about 2.2mm forward from a base 23 of the prongs. This equates to a distance t2 of about 4.1mm from a lower sealing portion of the prong 13'. It will be understood that these dimensions are provided as examples, and that the size of the septal recess may vary for different sized prongs and/or different patient interface body 11 sizes.

The cannula body 11 may comprise a further region 27 (indicated by the broken lines in Figure 22) positioned generally below the base region 25 on the patient facing side of the cannula body. This region 27 is configured to provide comfort and conformability where the interface body 11 contacts a patient's philtrum, reducing pressure on the skin and minimising risk of skin damage.

This philtrum-contacting region 27 may comprise a region having reduced wall thickness relative to the base of the prongs 13 and/or the rest of the manifold 14. The philtrum-contacting region may comprise a region having a thicker wall thickness compared to the base region 25. In some embodiments, the philtrum-contacting region 27 may comprise a region having a wall thickness that is the same or larger than the wall thickness at the base of the prongs 13. The wall thickness may be substantially constant across the region 27. In the exemplary embodiment shown and described above, the philtrum-contacting region 27 has a constant wall thickness of about 0.5 mm. In another embodiment, the wall thickness is about 0.6mm at the base 23 of the prongs and the philtrum-contacting region 27 has a constant wall thickness of about 0.6mm.

The philtrum-contacting region 27 is contiguous with the flexing base region 25 of the prongs, with an upper boundary of the philtrum-contacting region 27 forming a lower boundary of the base region 25. Therefore, the shape of an upper boundary of the philtrum-contacting region 27 at least partly corresponds to the shape of a lower boundary of the base region 25. The flexing base region 25 may transition into the philtrum contacting region 27 gradually, or in some embodiments, the transition may be quite abrupt.

The philtrum-contacting region 27 may extend across a majority of the width of the body. For example, philtrum-contacting region 27 may extend at least the width of the two prongs, from the lateral side of a first prong to the lateral side of a second prong 15. Alternatively, the philtrum-contacting region 27 may extend at least the width of the base region 25.

In the embodiment shown, the philtrum-contacting region 27 is shaped to be broadest at a middle of the region, at a mid-line of the interface body 11, and in-line with the septal region 29 between the prongs 13. The philtrum-contacting region 27 may taper to be narrowest towards the lateral sides of the region 27. In one embodiment, the philtrum-contacting region 27 has a generally oval shape.

In an embodiment, the wall of the cannula interface body 11 comprises at least four regions of differing wall thicknesses: the prongs 15 having a first wall thickness, the flexing base region 25 having a second wall thickness, the philtrum region 27 having a third wall thickness, and the remaining body having a variable wall thickness that includes a fourth wall thickness. The fourth wall thickness may be greater than the first, second and third wall thicknesses. The fourth wall thickness may be the maximum wall thickness of the interface body 11. The first wall thickness may be greater than the second wall thickness and greater than or equal to the third wall thickness. The third wall thickness may be greater than, or equal to, the second wall thickness. The second wall thickness may be less than the first, third and fourth wall thicknesses.

### Conduit-frame coupling

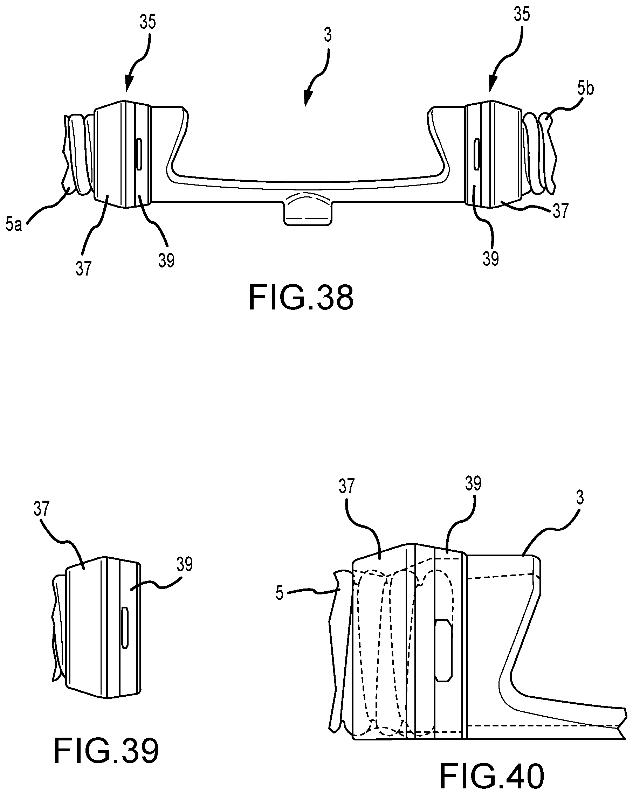

Figures 38 to 40 illustrate an arrangement for coupling the inspiratory and/or expiratory conduits 5a, 5b, to the patient interface frame 3. The arrangement comprises a collar 35.

The connection between the collar 35 and the frame 3 may be permanent. For example, at least part of the collar 35 may be fused or overmoulded to the frame 3.

The collar 35 may comprise a fusing portion 39 that acts to couple the collar 35 and the conduit 5 to the patient interface frame 3. Optionally an entirety or substantially all of the collar 35 may comprise the fusing portion.

The conduit 5 may be attached to the collar 35 about an annular portion of the collar and conduit. The connection between the conduit 5 and the collar 35 may be a permanent one, for example, a fused connection. The attached annular portion of the collar and conduit 5 are positioned adjacent the frame 3.

The length of the annular portion of the conduit 5 that is attached or fused to the collar 35 is a length of at least 1mm, for example, between about 1mm and about 5 mm. However, this length may vary depending on the size of the conduit. For a helically wound or corrugated conduits, at least a single wind or corrugation is fused with the collar 35. In some embodiments, one or more of the winds or corrugations may be fused with the collar 35. For example, up to three winds or corrugations may be fused with the collar, or alternatively more than three winds or corrugations. The fused length of the conduit is positioned at or proximal the side of the frame end of the collar 35.

In addition to the fused length, the collar 35 additionally houses a free length of the conduit. That is, at least a portion of the conduit is free to move within the collar 35, within the bounds of the collar. The free length may be longer than the attached/fused length of the conduit.

The free portion within the collar ensures that any bending of the conduit is not reacted at a rigid connection at the conduit end of the collar, which may compromise or cause failure of the connection. Instead, bending forces are reacted by the length of unbonded tube in the collar flexing and contacting an inner wall of the collar 35.

The inner diameter of the collar 35 may be constant, or alternatively may vary along the collar, for example the diameter may be greater at the end of the collar adjacent the free conduit portion.

The collar 35 may comprise a flexible or rigid body. For example, the collar may comprise one of more of a thermoplastic, polyurethane, and/or silicone. In an exemplary embodiment, the collar 35 comprises pre-moulded Thermoplastic polyurethane (TPU). In an exemplary embodiment, the TPU collar 35 is overmoulded with the patient interface frame 3

### Conduit connector

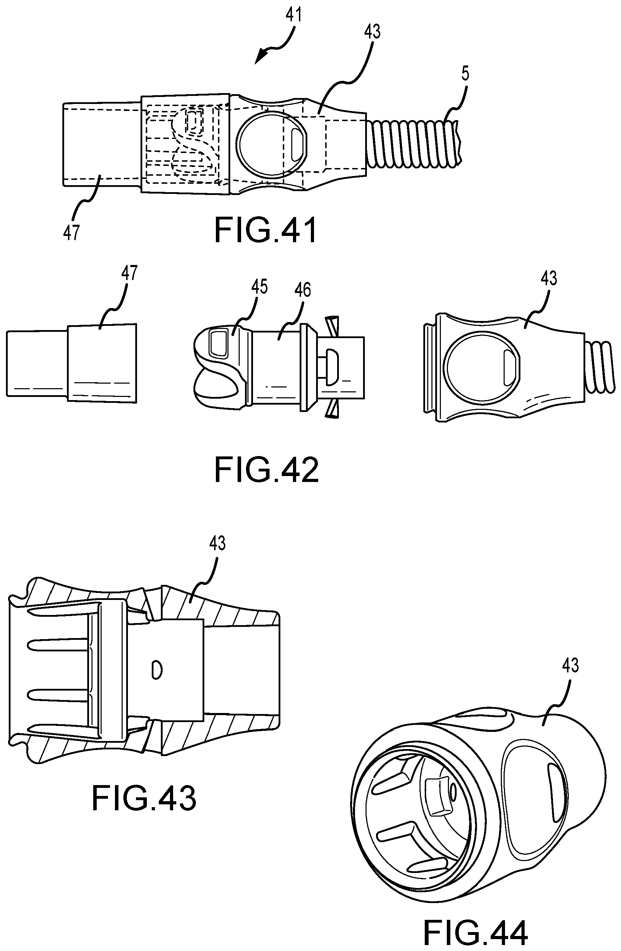

The conduits 5a, 5b and connectors of the patient interface assembly 1, 11 allow fluid communication to and from the patient from an external device or gas flow source. Figures 41 to 44 illustrate an exemplary connector 41 for use at the end of the conduits 5a, 5b. The connector 41 can be any type of interlocking or mating connector to couple the tubing to another device 47 or to additional tubing. The connector 41 can have locking fingers 45 extending from a connector body 43, to connect and secure the tubing to another device or an additional tubing. In some embodiments, the locking fingers 45 can extend away from the connector body 43. The locking fingers 45 can be spaced apart and narrowing along their length away from the connector. The locking fingers can have locking recesses or detents that are formed at least on outer surfaces of each of the locking fingers. The locking recesses can lock with portions of a gas delivery tube connector or other connector 47. The locking fingers can interact with recesses of the gas delivery tube connector or other connector to align the connector 41 and the gas delivery tube connector or other connector.

An outer body of the connector 43 may house a base portion 46 supporting the locking fingers of the connector and comprise grip features to facilitate grasping of the connector. In the embodiment shown, the body comprises four grip indents spaced 90 degrees to each other.

In some embodiments, a first end of one conduit 5a may be coupled to the body of the connector as illustrated in Figure 41, and the first end of a second conduit 5b may include a female connector 47 for receipt of a complementary connector.

Example of the connector and/or the connector assembly can be found in International Application No. PCT/NZ2012/000142, filed August 10, 2012 entitled CONDUIT CONNECTOR FOR A PATIENT BREATHING DEVICE. In other embodiments, the connector can have a tapered fit to connect to another device or an additional tubing.

### Lateral arms

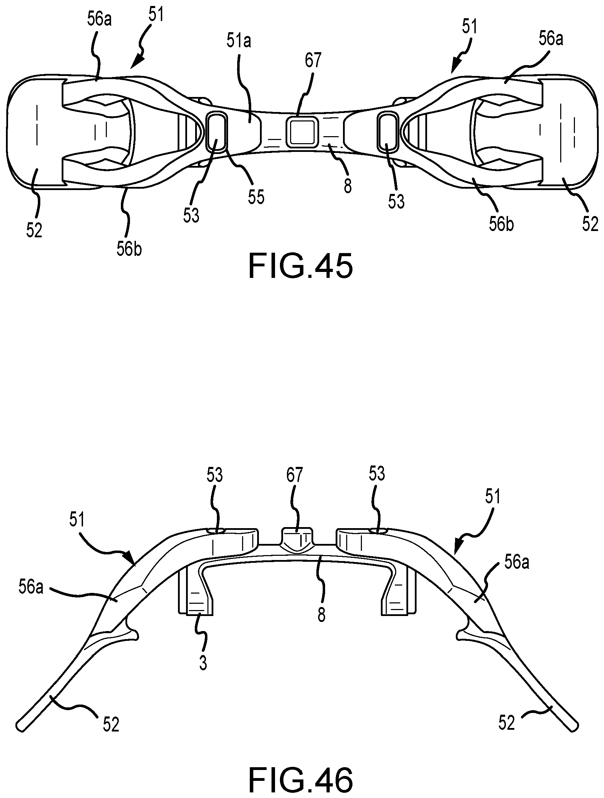

Referring to Figures 45 to 47, a pair of lateral arms 51 is provided for releasably connecting the patient interface frame 3 to supporting headgear and/or connectors to secure or support the patient interface body relative to the patient. In some embodiments the connectors may comprise dermal pads adhered to the patient's face and having an engagement surface for engagement of the lateral arms.

In some embodiments, the lateral arms 51 may secure the interface body to the user's face to help the interface body to form and maintain a seal with the patient's face. In alternative embodiments, such as an interface for nasal high flow therapy, the lateral arms may secure the interface to face in an operational position to deliver gases to patient airways.

Each lateral arm 51 is a separate member having a first end 51a for connection to a front face of the frame 3. Each first end 51a may be releasably or permanently connected to a front face of the frame 3.

The provision of two separate arms advantageously enables a region at a centre of the frame 3 to be uninhibited by the lateral arms and free for attachment to other components such as the securement member described in more detail below. A second end of each arm 51 comprises a connection pad 52 having a connection surface for engaging with headgear and/or connectors to secure or support the patient interface relative to the patient.

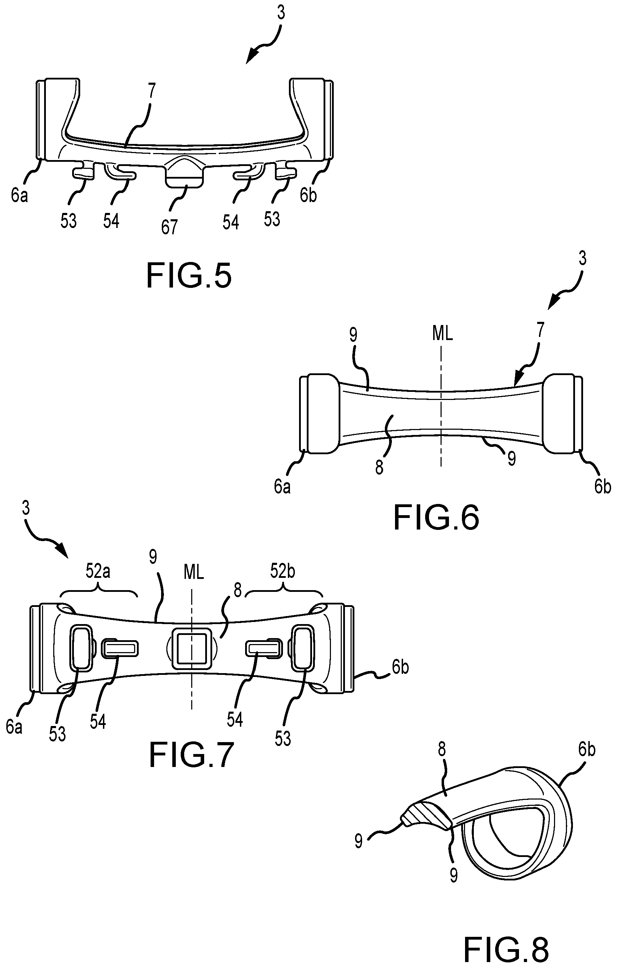

Referring also to Figures 5 and 7, the front face of the frame 3 comprises a left connection zone 52a and a right connection zone 52b for coupling to a respective lateral arm 52. Each connection zone is positioned between a mid-point of the frame and a respective side of the frame.

The connection zones may include one or more connection features to engage with a respective lateral arm 51. The connection features may comprise any suitable feature that facilitates a fixed, optionally releasable, connection with the first end of the arm 51. The connection preferably substantially prevents movement of the first end of the lateral arm with respect to the frame, including relative pivoting of the components.

In the embodiments exemplified, the connection zones each comprise one or more projections for receipt by complementary apertures or recesses on the lateral arms. Each projection 53, 54 for engaging a lateral arm 51 is positioned between a mid-point of the frame and a respective side of the frame 3. In some embodiments some or all of the projections may be positioned to be closer to the respective side of the frame than to midpoint of the frame.

In the example embodiment of Figures 45 to 47, two projections 53, 54 are provided in each frame connection zone. A first projection 53 comprises a post having an enlarged end. The post of the projection 53 projects forward and orthogonally from the front surface of the frame 3 such that the enlarged end of the first projection 53 is spaced from the front surface of the frame with a front face that is generally parallel with the frame front surface. The enlarged end may be centred on the post, or may be off-centre, for example projecting towards one side of the frame 3. In the embodiment shown, the enlarged end of the first projection 53 is generally rectangular in shape, oriented transversely on the frame body member 8, and projects from the post towards the respective side of the frame. However, it will be appreciated that other shaped projections may be utilised.

The first projection 53 is received through an aperture 55 proximal the first end of the lateral arm 51. A recess having a shape and depth corresponding to that of the enlarged end of the projection is provided on a front surface of the lateral arm, in line with the aperture, for seating of the projection when the lateral arm is connected with the frame 3. The height of the first projection 53 is substantially the same as the thickness of the arm at the first end 51a such that the enlarged end of the first projection 53 sits generally flush with the front surface of the lateral arm 51. In other embodiments, the first projection may be sized to sit recessed behind a front surface of the lateral arm 51, or to protrude forward of the front surface.

A second projection 54 has the form of a hooked connector for receipt in a complementary recess in the respective lateral arm. In the embodiment shown, the hooked connector 54 comprises a post with a hook projecting laterally from the post. The hook is preferably oriented to project towards the midpoint of the frame, away from the side edge of the frame, such that it engages with the arm upon the application of tensile forces to the arm (rather than risk disengagement). Preferably the hook portion forms an angle of about 90 degrees with the post, but alternatively may be otherwise angled, for example, depending on the rearward angle of the lateral arms.

The hook portion and the post of the second projection may have a constant width. Alternatively, the width may vary along the projection, and/or the hook portion may have a different dimension to the post.

In the embodiment shown, the second projection 54 projects forward from the frame 3 a smaller distance than the first projection 53, and a distance that is shorter than the thickness of the first end 51a of the lateral arm such that the hook does not extend through the full thickness of the lateral arm 51 and is instead concealed by a front surface of the lateral arm. However, in alternative embodiments, the second projection may extend through the full thickness of the lateral arm 51 to be visible from the front.

The lateral arms 51, including the first ends 51a may comprise a resilient material such as an elastomer. In some embodiments the arms 51 comprise a thermoplastic elastomer such as, but not limited to, those marketed by KRAIBURG^{™}. The use of a flexible material for at least a portion of the lateral arms 51 enables the arms to flex or bend to better accommodate variations in individual patient facial geometry and/or facial movement.

To assemble the lateral arms 51 with the frame 3, the recess (not shown) in the rear surface of the arm is engaged with the second projection 54. The arm is then pulled laterally outward, stretching the aperture 55 over the enlarged head of the first projection 53, before pulling the arm into contact with the surface of the frame and releasing the arm to allow the apertures to return to their un-stretched state. The head of the first aperture prevents movement of the arm out of contact with the front surface of the frame.

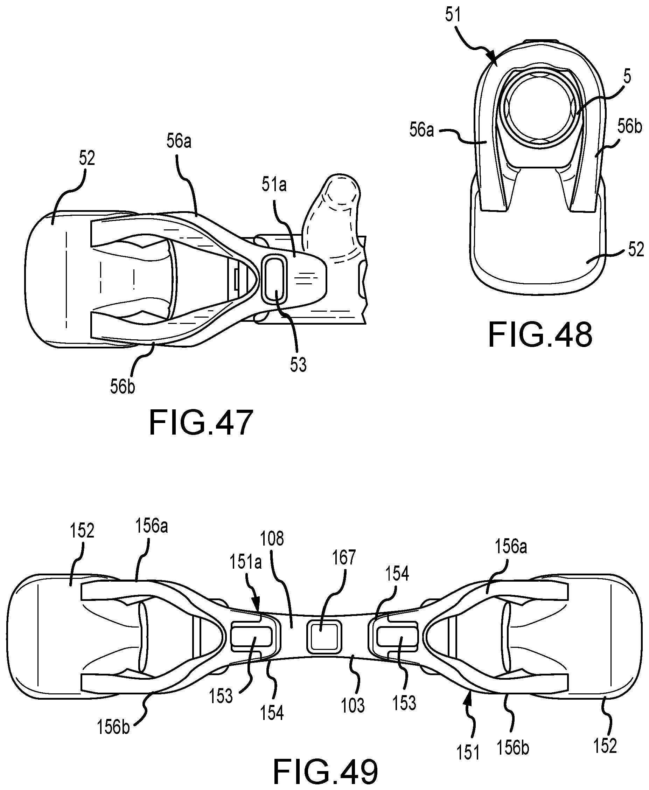

As illustrated in Figure 46, the lateral arms 51 are shaped to extend rearward from the frame 3. From the first end of the lateral arm 51a, the arm bifurcates into upper and lower members 56a, 56b. These two members diverge to define an intervening space that allows the passage of the inspiratory and expiratory conduits 5a, 5b, as illustrated in Figure 48. The space also provides at least partial visibility of the side portions 6a, 6b of the frame and/or the sides 14a, 14b of the patient interface body. This allows for inspection of the coupling between the frame and conduit, and also for viewing identifying features in the vicinity such as the colour-coded annular members 22 described above. It is also advantageous during assembly of the interface body 1 to the frame 3 as, for embodiments where the frame is transparent or translucent, it enables a user to view whether the interface body has been correctly located within the frame 3. At the point where the upper and lower members 56a, 56b diverge, a U-shaped cut-out may be provided to improve visibility of the side of the frame.

The upper and lower members 56a, 56b are twisted along their length about a quarter turn. The upper and lower members 56a, 56b are twisted in opposing directions, away from each other. The surface of the upper member 56a that is parallel with the front face of the frame twists to be upward facing adjacent the connection pad 52. Conversely, the surface of the lower member 56b that is parallel with the front face of the frame twists to be downward facing adjacent the connection pad 52. This twisting stabilises the space between the members to reduce the likelihood of interference with the conduits 5a, 5b.

The upper and lower members 56a, 56b attach to a front surface of the connection pad 52 at spaced apart points at a medial side of the connection pad 52. The opposite, patient facing surface of the connection pad 52 is configured for attaching to headgear or other connectors. In the exemplary embodiments, the patient facing surface of the connection pad 52 comprises a hooked or looped surface to engage with a complementary hooked or looped surface. The hook or loop surface can be overmoulded to the respective connection pad 52 or otherwise connected. Overmoulding may advantageously provide a non-patient-facing surface of the connection pad 52 that is more readily wiped or otherwise cleaned. In preferred embodiments, particularly where there is a risk of a portion of the connection pad contacting a wearer's skin, the connection pad 52 comprises a looped surface.

Figures 49 to 55 illustrate an alternative embodiment lateral arm 151 in which the first end of the arm 151a comprises a rigid portion 154 to facilitate a snap-fit type engagement with the frame. The rigid portion 154 may comprise a hard plastic clip such as a polypropylene clip. The rigid portion is permanently attached to the remainder of the lateral arm 151, for example by way of the elastomer of the remaining lateral arm being over-moulded to the rigid portion.

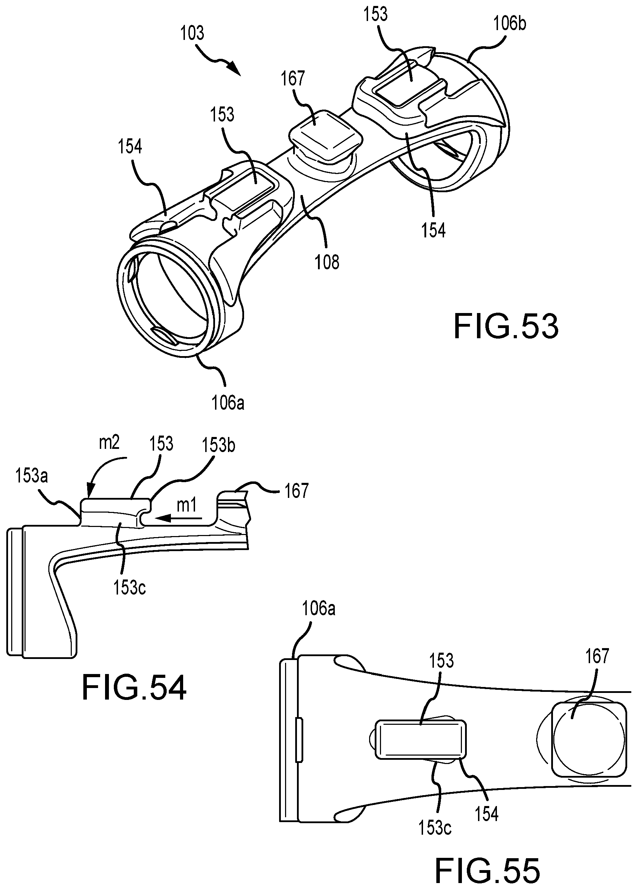

Figures 53 to 55 illustrate a further embodiment frame for use with a snap-fit type clip. In this embodiment frame 103, a single projection 53, is provided in each frame connection zone. The frame projection 53 is shaped to have a plurality of engagement surfaces. For example, the projection 153 includes a hook portion 153 comprising a projection that extends towards the midpoint of the frame, spaced from the front face of the frame. The projection 153 also includes a lip 153a for catching a portion of the clip between the lip and the front face of the frame. In the embodiment shown, the projection has a generally anvil shape.

The clip 157 has an aperture 158 for receiving the projection, with lips 159a, 159b for engaging with those on the projection 153. To assemble the lateral arm 151 with the frame, the clip 157 is slipped underneath the hooked portion of the projection 153, as illustrated by m1 in Figure 54, then pressed over the projection (m2) causing the clip to flex and snap down around the projection. The lip 153a catches a respective lip 159 a on the clip to secure the connection. The base of the projection 153 comprises angled surfaces 153c that diverge towards the hook portion 153b. These surfaces abut complementary angled surfaces 159a of the clip and act to prevent movement of the clip towards the mid-point of the frame 103.

### Stability arm

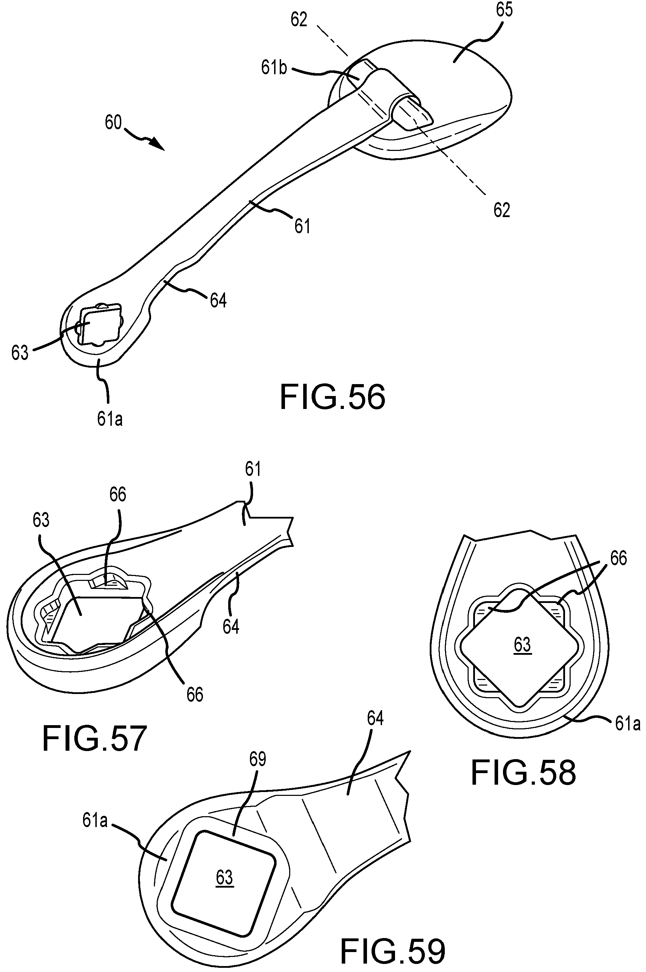

Figures 56 to 75 illustrate an embodiment of a stability arm 60 connectable to the frame 3 and connectable to headgear 71 to help to secure or support the patient interface body relative to the patient. In some embodiments, the stability arm 60 may secure the interface body to the user's face to help the interface body to form and maintain a seal with the patient's face. The length or size of the stability arm may vary such that a suitably sized stability arm can be selected to best fit a given patient and/or the selected mask or cannula. A stability arm is envisaged to primarily be used with mask-type patient interfaces, but in some embodiments it may be used with nasal cannula-type interfaces.

In the illustrated embodiment, the stability arm 60 includes a coupling portion 61a proximal a first end of the arm with a connection feature 63 for connection to the frame 3. A support pad 65 for connection to headgear is provided at an opposite, second end of the stability arm 60, with a bridging potion 61 extending between the coupling portion 61a and the support pad 65.

The support pad 65 may be a forehead support pad for connection to headgear adjacent the forehead of a patient. An end 61b of the bridging portion 61 is pivotally attached to a front surface of the support pad 65 at a pivot axis 62 that is generally perpendicular to the longitudinal direction of the stability arm 60, thereby enabling the support pad to rotate/pitch relative to the bridging portion 61. In the embodiment shown, the support pad 65 may rotate/pitch relative to the bridging portion 61 almost 180 degrees (limited by the front face of the support pad 65 contacting the front face of the arm 61). When the support pad 65 is connected to the headgear, this enables the stability arm 60 and any attached components to be flipped out of the way as required, and then flipped back. This allows a caregiver to move components out of the way for performing caregiving tasks such as cleaning of skin, without necessitating a complete removal and set-up of the components before and after such a task.

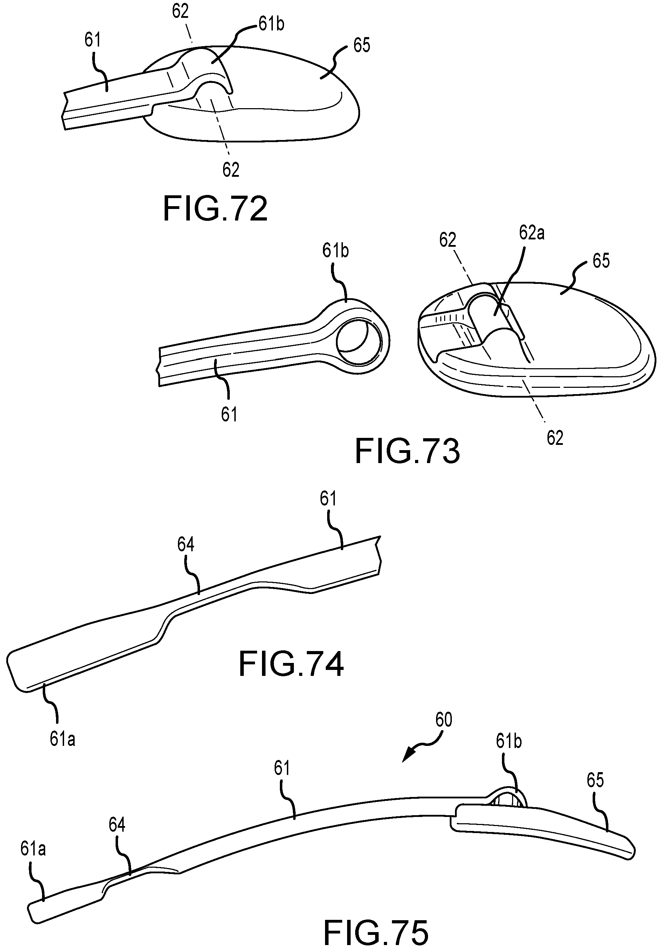

Referring to Figures 72 and 73, in the example shown, a pin 62a for extending along the pivot axis 62a, is received by an aperture at the connecting end 61b of the bridging portion. The pin 62a may be overmoulded therewith.

The interfacing surfaces of the pin 62a and the connecting end 61b may comprise different materials, which enhances the pivoting of the two components relative to each other and prevents the components from fusing together. The two materials may have a different Shore hardness value. For example, the Shore hardness value of the connecting end 61b may be greater than that of pivot pin 62a.

In the embodiment shown, the pivot axis 62 is provided on a lower portion of the support pad 65. In the embodiment shown, the pivot axis 62 is set back from the bottom edge between about one quarter and one third of the length of the support pad 65. This positioning ensures there is a sufficient connection area on both sides of the pivot axis and so reduces the likelihood of the front of the support pad from lifting out of engagement under applied forces.

The patient facing surface of the support pad 65 is configured for attaching to headgear or other connectors. In the exemplary embodiments, the patient facing surface of the support pad 65 comprises a hooked or looped surface to engage with a complementary hooked or looped surface. In exemplary embodiment, the support pad 65 comprises a hooked surface and the stability arm 60 has sufficient length to ensure that none of the hooked surface will overhang the headgear and contact the patient when the stability arm is installed. The stability arm may be available in several sizes to best ensure the hooked surface is always correctly aligned with the headgear. The patient facing surface of the support pad 65 may be contoured or angled to conform to the curvature of a patient's head. The curvature may be slight as illustrated in the embodiments of Figure 56 and 75, or it may be more pronounced.

The support pad 65 may comprise a compliant material such as TPE for patient comfort and to facilitate connection to differently contoured surfaces. The hooked or looped surface is preferably integral with the support pad 65, for example, the body of the support pad 65 may be over-moulded with the looped surface.

In contrast, the bridging portion 61 and the coupling portion 61a comprise a generally rigid material such as nylon. A flexible region 64 is provided at the base of the bridging portion, adjacent the coupling portion, to allow the arm to be bent away from the patient or headgear, about the flexible region during assembly or disassembly to prevent the pad 65 catching on the headgear. The flexible region 64 also allows flexing/movement of the coupling region 61a relative to the rest of the arm, which aids with assembly of the stability arm onto the frame 8. This relative flexing allows the coupling region 61a to 'self-align' with the frame 3, making it easier for the user to engage the stability arm with the frame 3.

The flexible region comprises a length of the bridging portion of the arm having a reduced wall thickness. This flexible region 64 comprises a non-zero length of constant thickness, for example a length of between about 2mm and about 10mm long. This region flexes along this length with a substantially even bend radius, rather than bending at an inflection point, and thereby avoiding the presence of stress concentration associated with an inflection point. This allows a material other than polypropylene or polyethylene to be used if desired.

The thickness of the flexible region may be between about 15% and about 60% of the thickness of the main portion of the arm. In some embodiments the thickness of the flexible region may be between about 25% and about 40% of the thickness of the main portion of the arm 60. In one embodiment, this equates to a thickness of between about 0.3mm and about 1.2mm.

The thickness of the arm transitions from the reduced wall thickness at the ends of the flexible portion to a larger thickness at the adjacent portions of the arm. In some embodiments, the stability arm is transparent or translucent to reduce the visual impact of the arm, making the patient interface assembly appear less intrusive.

The coupling portion 61a for connection to the frame 3 is proximal a first end of the arm and includes a connection feature 63 for engagement with a complementary engagement feature on the frame body 8. In some embodiments, the connection feature 63 includes an aperture to receive a central protrusion 67 on a front face of the frame, such that the coupling portion is attached to frame 3.

The protrusion 67 is positioned at a mid-point of the frame. The protrusion 67 comprises a post with an enlarged head at an end of the post. In the embodiment shown, the enlarged head has a square shape, formed by four triangular lobes 68 projecting outwards from a top of the post. In alternative embodiments, the enlarged head may comprise alternative multi-sided shapes.

The aperture 63 on the coupling portion of the stability arm has a square shape corresponding to the shape of the protrusion 67. However, the aperture 63 is oriented such that a diagonal of the square is substantially aligned with the longitudinal direction of the stability arm, and also with the midline of the frame when the stability arm is in the installed position. That is, in the installed position, the shape of the protrusion head 68 and the shape of the stability arm aperture 63 are angularly misaligned by about 45 degrees.

A top surface of the stability arm coupling portion comprises four generally triangular recesses 66 positioned at respective sides of the square aperture 63. These recesses are shaped and positioned to receive respective lobes of the frame projection 67 when the stability arm is in the installed position.

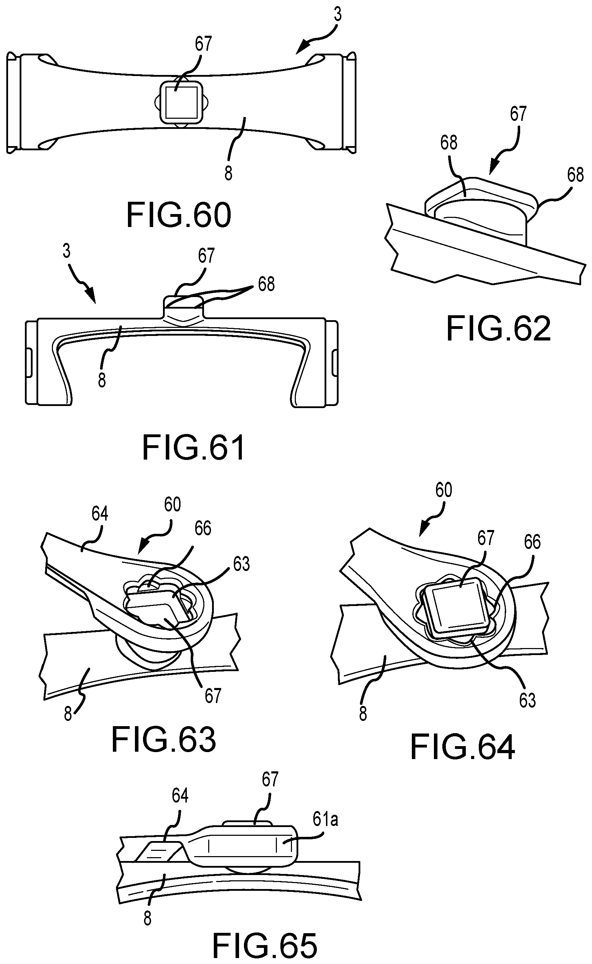

Connection and disconnection of the stability arm 60 to and from the frame 3 will now be described with respect to Figures 63 to 71. To connect (or disconnect) the stability arm 60 to the frame 3, the connection aperture 63 of the arm is aligned with the head of the projection 67, such that the aperture fits over the head of the projection, then pressed onto the projection until an under surface of the arm 60 contacts the frame 3 (see Figures 63 and 4). A large radius fillet 69 (Figure 59) may be provided around the edge of the aperture 63, at least on an under surface (patient-facing side) of the stability arm 60 to help to guide the stability arm 60 into alignment with the protrusion 63.

In the connection/disconnection position for the present embodiment, the stability arm is oriented about 45 degrees to a mid-line of the frame. In alternative embodiments, the connection/disconnection position of the stability arm may be more or less than 45 degrees depending on the shape, number, and/or configuration of the lobes 68. For example, in the connection/disconnection position, the stability arm may by oriented between about 30 and about 60 degrees to a mid-line of the frame.

A front surface of the protrusion 67 may sit flush with or proud of the front surface of the stability arm 60.

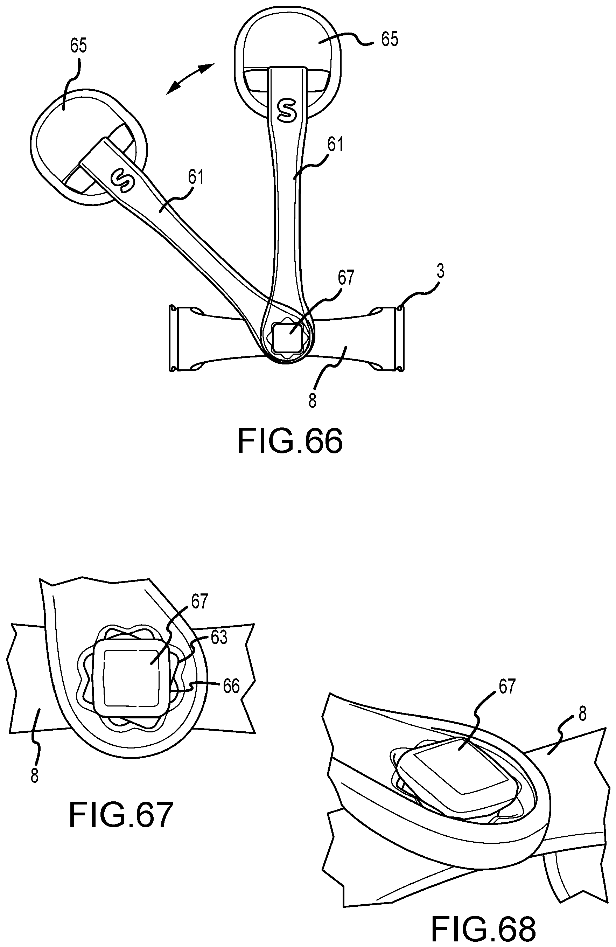

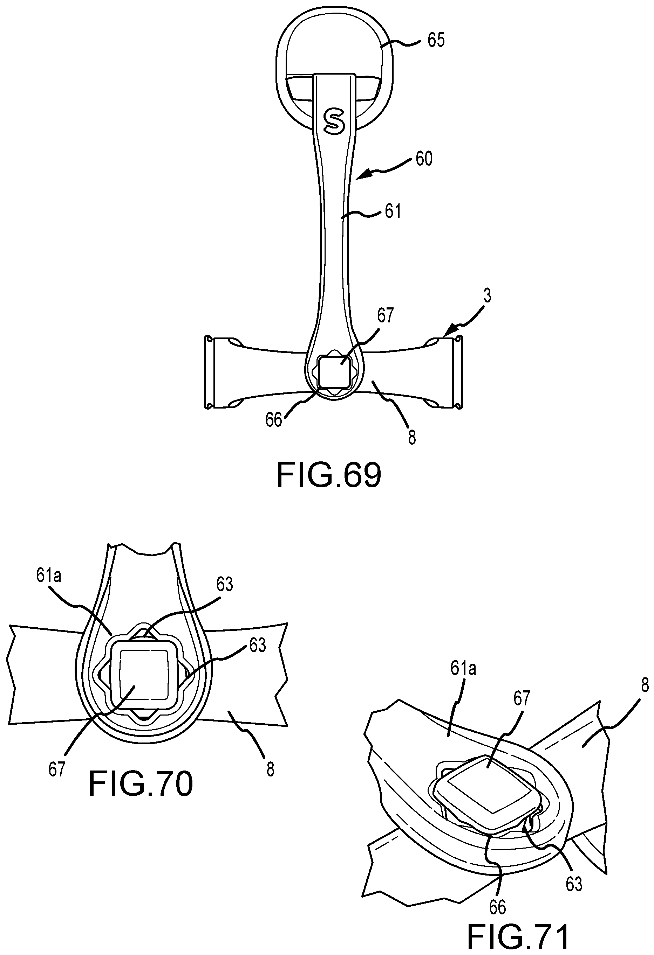

Referring now to figures 66 to 68, the stability arm is rotated towards an installed position in which it is aligned with the midline of the frame and 'locked' in place. As the stability arm 60 is rotated between the connection/disconnection position and the installed position, the corners of the enlarged head of the projection 67 press against the edges of the aperture and recesses. These edges provide surmountable resistance to the rotation. The application of further force by a user can overcome this resistance, snapping the arm to the installed position shown in Figures 69-71. This movement of the projection past the interfering edges creates tactile feedback for the user.

In the installed position, the lobes 68 of the projection 67 are seated in the respective recesses 66, with the post of the projection extending through the aperture 63. In this installed position, there is some play between the stability arm and the frame. That is, the protrusion 67 of the frame 3 can move within the bounds of the recesses 66 in the stability arm. Some play between components is advantageous for easier assembly of the stability arm to the headgear, and to enable micro-adjustments, for example tilting of the arm, for appropriate positioning of the connection pad 65 on the headgear to accommodate different head geometries.

### Headgear

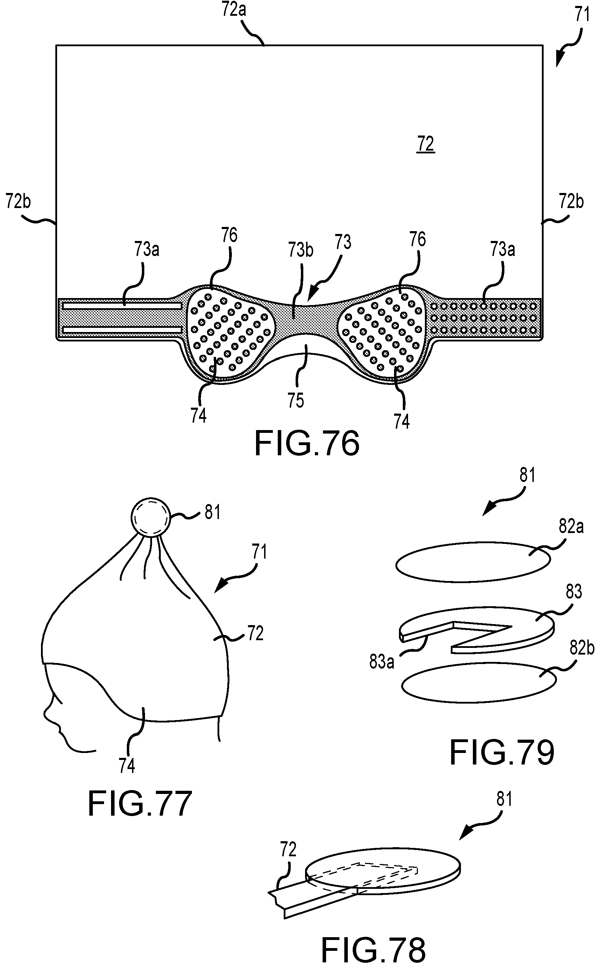

Figures 76 to 90 illustrate components of a headgear assembly for securing a patient interface assembly to a patient. The patient interface may include those described herein but may also include other forms of interfaces. In an embodiment, the headgear and the patient interface is for an infant or neonate.

Figure 76 illustrates one embodiment headgear 71 in an un-assembled form. The headgear comprises a base layer 72 forming a body of the headgear, and a headband region 73. The headband region comprises an outer engagement layer that at least partly overlaps and is fused to a lower portion of the base layer 71.

The overall length of the headband 73 is selected such that the headgear fits a target range of head circumference sizes. For neonatal and infant applications, the headband 73 may have a length between about 15 cm and about 60 cm. The headgear and headband may be available in a number of sizes, with each size intended to fit a target range of head circumference sizes. In some different sized embodiments, the headband 73 may have a length to accommodate head circumference sizes between about 17cm and about 22cm, between about 20cm and about 26, between about 24cm and about 31, between about 29cm and about 36, or between about 34cm and about 45cm.

The headband has over-ear regions 74 shaped and positioned to at least partly cover the patient's ears to protect wearer's ears from the attached components and connectors. The over-ear regions 74 also advantageously provide a larger surface area for more secure connection with coupling components and for enhanced pressure distribution of forces from the patient interface components by way of the larger connection area.

The over-ear regions 74 may be defined by enlarged regions relative to the adjacent headband regions. These over-ear regions are sized to provide sufficient surface area for attaching and appropriately positioning connectors such as those described in more detail below for coupling to and supporting a patient interface.

In the embodiment shown, each ear region 74 comprises a lobe defined by a rounded, downwardly projecting lower edge of the headband. The lower edge of the headband may be arcuate, or generally crescent-shaped. The lower lobes project lower than a lower edge of adjoining front portions 73a of the headband region.

Each ear region 74 may additionally (or alternatively in some embodiments) comprise an upwardly extending lobe defined by a rounded, upwardly projecting upper edge of the headband. These upper lobes project up from a top edge of adjoining front portions 73a of the headband region. The upper edge of the headband may be arcuate, or generally crescent-shaped. In the exemplary embodiment, the upper lobes 76 are smaller than the lower lobes.

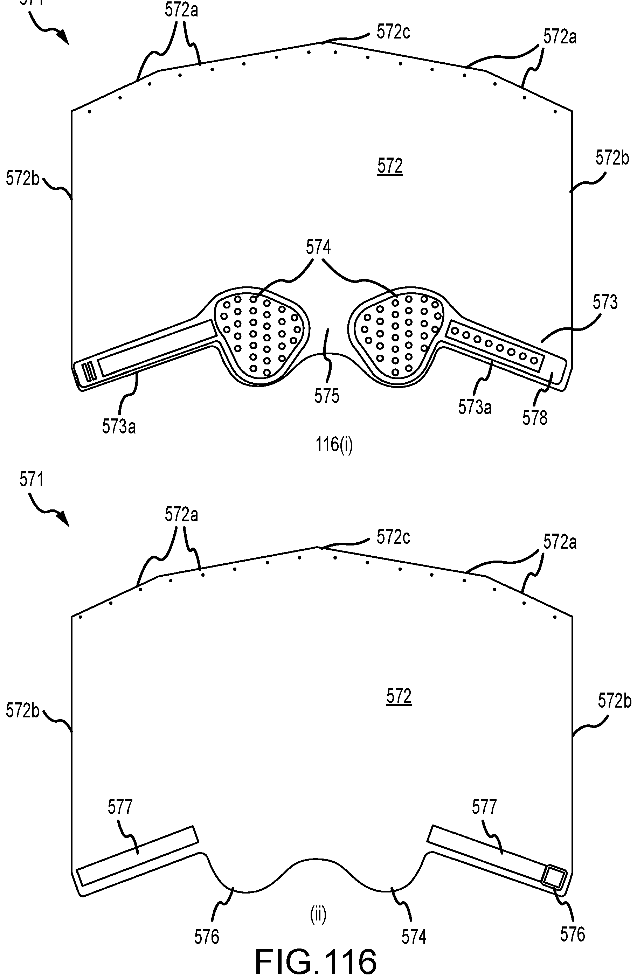

The height of the enlarged ear region may be 1.5 times or more the height of the adjoining front portions 73a. For example, in the embodiment shown, the height of the enlarged ear region is about two times the height of the adjoining front portions 73a. Figures 116(i) to 117 show exemplary embodiments in which the height of the front portions 573a, 673a of the headband 573, 673 is reduced such that the height difference between the front portions of the headband and the enlarged ear regions 574, 674 is more pronounced.

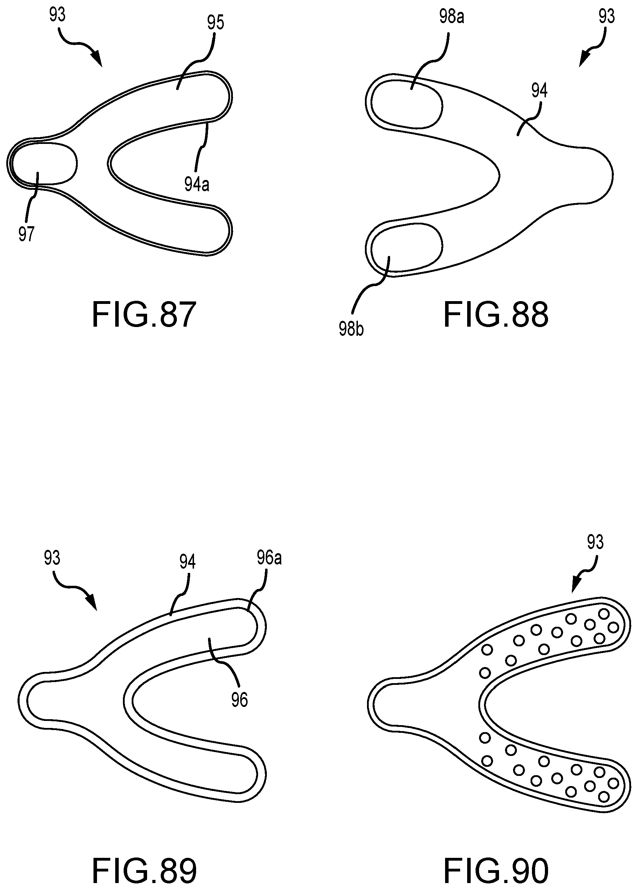

The over-ear regions 74 may be sized to provide sufficient purchase area for the attachment of the connectors illustrated in Figures 87-91, 94-104, 121-123, and 179-129 and which are described below. The over-ear regions 74 may have a height that is at least as big as the width of the respective connector at it's second end. For example, between about 1 and 2 times the width of the respective connector at its second end. In some embodiments, the over-ear regions 74 may have a height that is between about 1 and 1.5 times the width of the respective connector at its second end.

The front portions 73a of the headband 73 may extend directly forward of the over-ear portions, with the two front portions 73a being colinear, as illustrated in the embodiments 71, 171, 471 of Figures 76, 109, and 115(i). Alternatively, the front portions of the headband may be angled relative to horizontal and thereby to each other. Figures 116(i) and (ii) and 117 show alternative embodiments of the headgear in which the front portions 573a of the headband region extend at a downward angle forward of the over-ear regions 574, 674. This angling of the forward portions of the headband region may improve the conformity of the headgear with a patient's head, reducing inadvertent lifting of the headband from a patient's head during use.

The headband front portions 573a, 673a may be angled downwards at an angle of between 0 degrees and about 45 degrees, for example between about 15 degrees and about 30 degrees. In the example embodiment shown in Figures 116(i) to 117, the front portions 573a, 673 are angled downwards at an angle α of about 20 degrees from horizontal. In alternative embodiments, the right portion 473a, 573a of the headband and the left portion 473a, 573a are angled downwards at different angles to each other. For example, the right portion 473a, 573a of the headband may be angled downwards at an angle which is greater than the angle of the left portion 473a, 573a.

The front portion of the headband 73a on the left side of the headgear may be the same length or a different length to the portion 73a on the right side of the headgear. In the embodiments 71, 171, 471 of Figures 76, 109, and 115(i), the left and right portions 73a, 173a, 473a are substantially the same length. In contrast, Figures 116(i) and (ii) and 117 show alternative embodiments in which the headband is asymmetrical, with one front headband portion 573a, 673a being longer than the other 573a, 673a.

Generally, the longer portion may be the front headband portion 573a, 673a that is configured to be wrapped over and engage the outer surface of underlying headband portion. The front headband portion configured to sit against the patient may be the shorter portion. In the embodiments shown, the right portion 473a, 573a of the headband is shorter than the left front portion 473a, 573a. It will be appreciated that this arrangement may be reversed in alternative embodiments.

A rear bridging section 73b may be provided between the over-ear regions 74 for positioning at or above the nape of the patient's neck. This bridging section comprises a lower edge that is contiguous with a lower edge of the over-ear regions 74 and an upper edge that is contiguous with an upper edge of the over-ear regions 74. The upper and lower edges are shaped such that the rear bridging section 73b is narrower in height than the over-ear regions, typically with a shaped lower edge to provide clearance for the base of the neck and thereby to reduce pressure on a wearers neck. This shape also helps the headband to conform around the patient's head as the shape minimises fabric creasing helping the headband to lay flat against the patient and sit comfortably at the nape of the neck. In the exemplary embodiment, the lower edge of the rear bridging section is concave such that the narrowest height of rear bridging section 73b is at a centre point of the section.

The rear bridging section 73b may have a height dimension at its narrowest point that is equal to or less than the height of height of the headband front portions 73a. For example, the height of the narrowest point of the rear bridging section 73b may be between about 0.5 and about 1 times the height of the headband front portions 73a, for example, between about 0.6 and about 0.8 times the height of the headband front portions 73a. In one example the height of the narrowest point of the rear bridging section 73b is 0.75 times the height of the headband front portions 73a.

The rear bridging portion may span a distance between the over-ear regions 74 that is between about 0.2 and about 0.8 times the length of the front headband portions 73a or a shorter one of the headband portions. In some embodiments, the rear bridging portion may span a distance between the over-ear regions 74 that is between about 0.35 and about 0.55 times the length of the front headband portions 73a or a shorter one of the headband portions. The headband region 73 may partially or wholly overlap a lower portion of the base layer. In the embodiment shown, a lower edge of the base layer 72 extends beyond a lower edge of the headband region. This unlapped lower portion forms a lower lip that may soften the edge of the headgear for more comfortable skin contact. In the embodiment shown, a larger depth of unlapped base layer 75 is provided between the over-ear regions 74, in an area configured to sit across the nape of a patient's neck. This portion 75 assists the headgear in conforming with a patient's head as well as providing enhanced comfort.

The base layer 72 comprises a panel of a flexible material, for example a fabric panel.

The flexible material may be a fabric that exhibits at least two-way stretch, with the stretch direction in the width/circumferential direction of the headgear. The base layer preferably comprises only a single panel to thereby avoid the presence of seams that would be created on joining multiple panels. The base layer may be a singly-ply or multi-ply material.

In some embodiments the base layer 72 may comprise a fabric having an unbroken looped (UBL) surface or similar to engage with connectors having a complementary hooked surface.

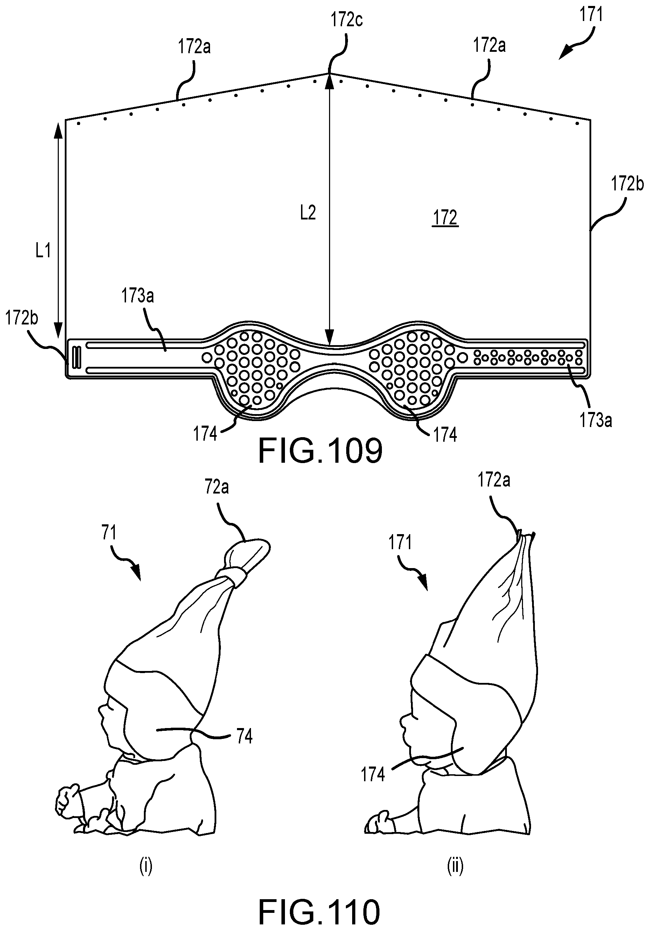

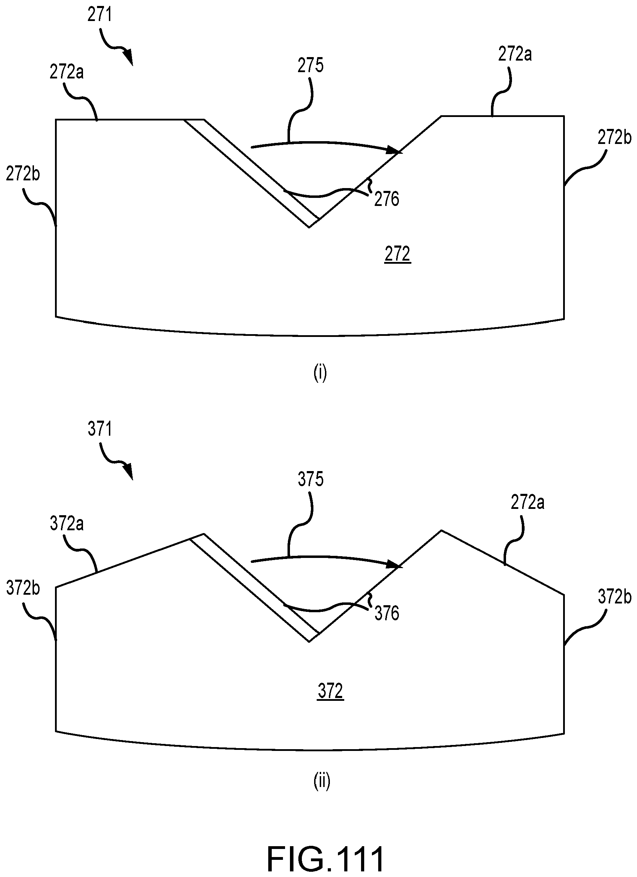

The panel in the embodiment of Figure 76 is generally rectangular, but other shapes are envisaged, for example, trapezoidal. Figures 109, and 111(i) and (ii) illustrate alternative shapes for the body of the headgear. In these embodiments 171, 271, 371, the panel (excluding the over-ear portions), is nonrectangular. In particular, the top edge of the headgear body is nonlinear and comprises one or more portions that are not orthogonal to the side edges. In contrast to the first embodiment 71, the length of the headgear body 172, 272 changes across the width of the headgear.

In the embodiment shown in Figure 109, the headgear body has the shape of an irregular pentagon. The top edge comprises two angled portions 172a, that each form an obtuse angle with the respective side edge 172b of the body. In the embodiment shown, each top edge angled portion 172a forms an angle of about 100 degrees with the respective side edge 172b, however in alternative embodiments other angles are envisaged, for example between 90 degrees and about 120 degrees.

The two angled top edge portions 172a meet at a central vertex 172c that is aligned with a point between the two over ear portions 174. The length L2 of the headgear body at the apex 172c is greater than its length L1 at the side edges.

The angled top edge portions 172a have the effect that when the top edge is gathered to a point and secured, such as with an end fixture, as described in more detail below, the position of the end fixture is moved forward with respect to the patient. Figure 110(i) illustrates the first embodiment headgear 71 (Figure 76) worn by a patient and contrasts this with the present embodiment headgear 171 in Figure 110(ii). The body of the first embodiment headgear has a length that is about the same as the length L2 of the apex 172c in the present embodiment headgear 171. The securement point for the second embodiment headgear 171 is approximately aligned with the spine of the patient, rather than posterior to the spine. This alignment may assist with ease of fitting the headgear onto a patient lying supine, and ease of moving an adjustment mechanism along the body of the headgear. It may also reduce the risk for the adjustment mechanism and/or the end fixture to move under the patient's head to cause discomfort.

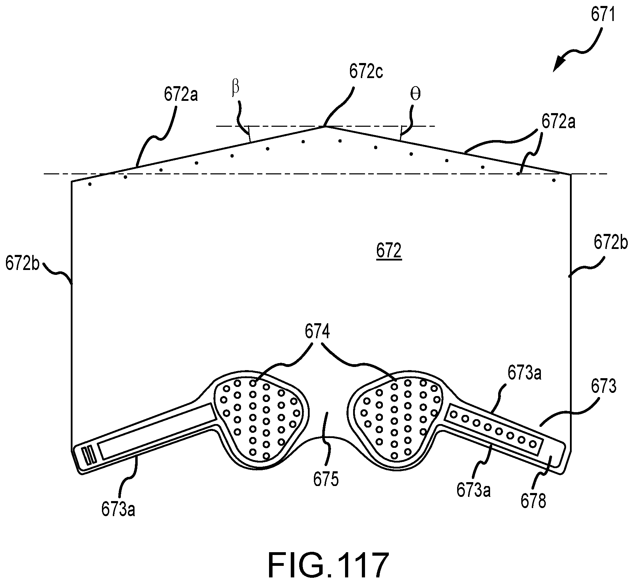

Figure 117 illustrates an alternative embodiment in which the headgear body has a shape approximating an irregular pentagon. In this embodiment, due to the asymmetry of the headband, the vertex 672c at the top edge may not be centrally positioned.

In this embodiment 671, the side edges 672b are of equal lengths to each other, and the two angled top edge portions 672a are also of equal lengths to each other. Due to the different lengths of the front portions 673a, and the angled orientation of these portions 673a, the top edge portions 672a of the base 672 are asymmetrical, with different slope angles β, θ. The slope angles β, θ may vary relative to each other between about 0.2 degrees and about 5 degrees. In some embodiments, the slope angles β, θ may vary relative to each other between about 0.5 degrees and about 2 degrees. In the example embodiment shown, the slope angles β, θ vary by 1.5 degrees - the top edge associated with the longer headband front portion 673a has a slope angle β of 11.5 degrees, the other top edge 672a has a slope angle θ of 10 degrees. However, other combinations of angles are envisaged.

Such a configuration in which the top edges 672a of the headgear base 672 have differing angles β,θ but in which the side edges 672b are the same length may better enable the overlapping portions and top edge of the base layer to align and be gathered when the headgear is assembled.

The headgear body may have other shapes that also provide the function of positioning the securement point in line with the wearer's spine. Additionally, or alternatively, it may also be desirable to remove some bulk from the body of the headgear to make the headgear less cumbersome, and/or to allow an adjustment member to slide along the headgear more readily.

Figures 111(i) and 111(ii) illustrate two alternative embodiments 271, 371 that utilize a cut-out 275, 375 in the top edge of the headgear body 272, 372 to provide these functions. Both embodiments include a triangular cut-out 275, 375 centrally positioned on the top edge. In the embodiment 271 of Figure 111(i), the top edge 272a (other than the cut-out) is orthogonal with the side edges 272b. In the embodiment 371 of Figure 111(ii), the top edge 372a (other than the cut-out) is non-orthogonal with the side edges 372b, forming an obtuse angle therewith.

When assembling the headgear 271, 371, the top edge 272a, 372a of the headgear body 271, 371 excluding the cut-out portion is gathered and secured at a securement point. In this process, the two edges 276, 376 of each triangular cut-out 275, 375 are drawn together. In some embodiments these edges 276, 376 may be joined, for example by sewing or welding or using an adhesive, alternatively the edges 276, 376 could be left un-joined.

The embodiments shown illustrate headgear with a single triangular cut-out, however, alternative embodiments may include more cut-outs and/or cut-outs of different shapes having a similar effect of reducing the material in at least an upper part of the headgear, and positioning the securement point further forward relative to the wearer.

Figures 116(i) and 116(ii) illustrate a further example shape of the headgear base layer 572 having a top edge with four angled portions 572a. This top edge shape, which generally reflects the downward angle of the front headband portions 573a in this embodiment, may produce less bunching then the top 572a is gathered compared to a rectangular body. In some embodiments, cut-outs may be provided along the top surface to further reduce fabric bunching.

The headband region and lower portion of the base layer are configured to wrap and secure around the head of the wearer to provide an adjustable fit. A fixture such as a hooked connector is provided on a patient-facing underside of the headgear proximal a side edge 72b of the headgear, in the headband region, for securing the headgear to itself when it is wrapped around a patient's head. One edge 72b of the headgear is overlapped over the other edge, to whatever extent is necessary to fit a patient's head circumference, and the fixture attached to the engagement surface of the headband region.

The headband regions 73, 173, 473 comprise a flexible outer engagement layer having an engagement surface for releasably securing connectors to the headgear. The outer engagement layer most commonly comprises fabric having an unbroken looped (UBL) surface to engage with connectors having a complementary hooked surface. The presence of such loops may be desirable to act as the loop portion of a hook and loop fastener system. By this configuration a hook and loop fastener system may be provided without having to attach any additional loop-providing component to the panel. This may go at least some way to providing a headgear which is of reduced thickness.

In an embodiment, the outer engagement layer covers substantially all of the headband region, alternatively the outer engagement layer may cover the majority of the headband region. As a further alternative, the outer engagement layer may only cover portions of the headband region, for example the front and sides of the headband. The outer engagement layer may be in the form of a single panel, but alternatively may be provided in two or more pieces.

In manufacturing of the headgear, the base panel and the engagement layer may be cut to their respective desired shapes, then lapped with each other and joined together. Such a configuration may differ from conventional methods such as in the case of a laminate material such as Breathe-o-preen^{®} where a headgear is built up by cutting already laminated panel sections, which are then joined together to form the headgear or parts of the headgear. However, in some forms at least some parts of the headgear may additionally or alternatively be cut once they are lapped together and potentially once one or more lapped parts are joined together.

The outer engagement layer is fused to an underlying portion of the inner base layer 72, an inner surface of the outer engagement layer being fused to an adjacent facing outer surface of the base layer 72. Fusing defines a melting of a layer or at least a constituent material of the layer. In relation to fusing together of two panels, fusing refers to the melting of one or both of the panels into the other or each other, respectively. Thus, two panels may be fused by either a) melting only one or primarily one panel into the other, or b) a simultaneous melting of both panels into each other.

The fusing may be non-additive, in that the fusion involves a treatment applied to the panel or panels and does not involve the use of any additional material such as an adhesive interposed between two panels to fuse them together. Fusing may involve one or both of the application of heat and pressure to the panel or panels.

The layers may be fused by welding including forms of plastic welding such as radio frequency (RF) or high-frequency (HF) welding, or ultrasonic, vibration or friction welding, hot edge welding, hot air welding, or induction welding. The welding process is selected to be suitable for joining the materials of the base layer and the engagement layer. A panel which is to be fused should include a meltable material, such as an artificial fibre. Similarly, where two panels which are to be fused together at least one of the two panels should include a meltable material, such as an artificial fibre. For example, materials such as PVC, CPVC, polyurethane, EVA, PVDC, PET and nylons are suitable for RF or HF welding.

Conventional headgear laminates which include foam layers are traditionally not fused by welding, as welding will degrade the cell characteristics of the foam, compressing it and causing it to become stiffer. Accordingly, welding of conventional headgear is confined to welding peripheral parts or edges of a lapped area of panels. As the headgear according to the disclosure may exclude a foam layer or layers, the panels may be fused together across substantial parts or even all of their overlapped areas. For example, at least a relatively wider boundary around the perimeter of a lapped region may be fused than in a conventional laminated headgear.

In contrast to conventional stitched joins where the desire is to minimise the size of seams to reduce their impact on comfort or their visibility, panels which are joined by fusing may be fused together across large areas without the corresponding seam bulk or change to the surface finish that a large, stitched area would cause.

Panels, and particularly textile or fabric panels, which are fused across large portions of the panels may provide firm yet flexible properties to the headgear. The fused areas may provide a visually clean and uniform surface to the panels. Selectively fusing and not fusing parts of the panels may allow for different material properties within regions of the same panel or panels. It may also provide differences in the surface features of regions made up of the same panel or panels.

Fusing panels may present advantages over other additive methods of joining panels together, such as by stitching or the use of an adhesive. In particular, the weight and potentially also thickness of the joined panels may be relatively reduced. Alternatively or additionally, the outer engagement layer may be attached to the underlying portion of the base layer by other means such as stitching or adhesion.

The layers may be fused together at a substantial entirety of the overlapping region. A substantial entirety of the lapped surfaces may be about 90% of the lapped area, or even about 95% of the lapped area. However, preferably the headband region comprises areas of fused material and areas of non-fused material. The non-fused areas generally provide more purchase for connectors compared to the fused areas. However, the fused areas provide some reduced purchase. The non-fused area at least in part define connection zones for releasably securing connectors to the headgear.

The contrast in the height or surface texture of the fused and non-fused regions form a visual contrast between the fused and non-fused regions. In an embodiment, the fused regions form a pattern comprising dots and/or stripes. Alternatively, or additionally, the non-fused regions form a pattern comprising dots and/or stripes.

The stripes may be straight, curved, wavy, or angled, for example, and they may be arranged parallel to each other, forming a grid, or be otherwise orientated. Dots in a pattern may be arranged uniformly or non-uniformly, for example, in rows or grids, radiating from a point, or randomly. Alternatively, or additionally, the fused or unfused regions may form text or decorative or identifying shapes such as a logo, for example the sizing detail displayed on the headband portion 73a of the headgear in Figure 76.

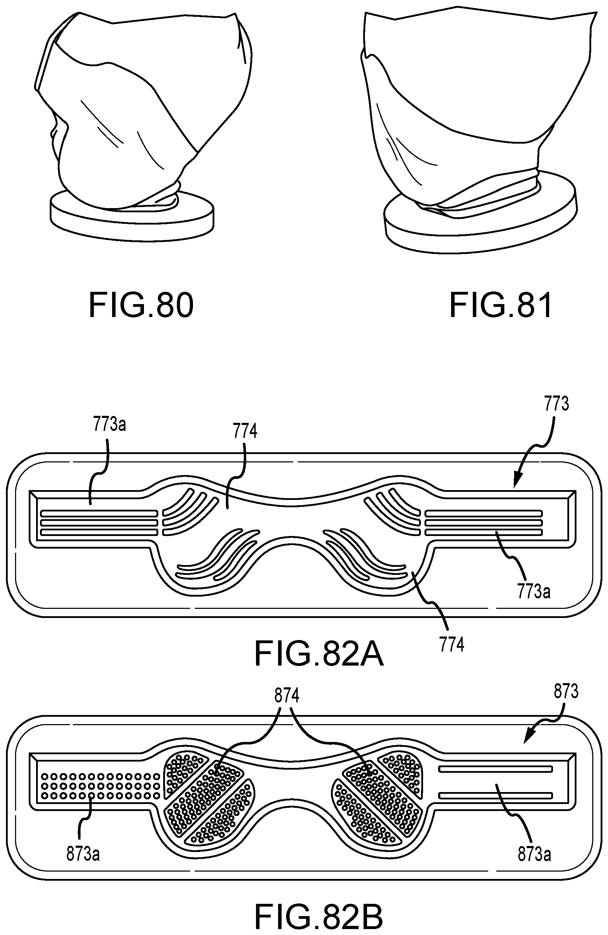

Figures 82A and 82B illustrate two exemplary patterns for welding an engagement layer to a base layer. In the embodiment of Figure 82A, the majority of the surface area of the headband 773 is fused, with parallel lines of unfused material in front portions 773a of the headband, and wavy lines of unfused material in the over-ear regions 774, to provide connection regions. In the embodiment of Figure 82B, the pattern includes a grid of dots of non-fused material on a first front portion 873a of the headband 873, regions of non-fused material defining fused dots in the over-ear regions 874, and non-fused size identifying data on a second front portion 873a of the headband. It will be understood that these exemplary embodiment weld patterns are just two of countless possible patterns, many other patterns are envisaged.

The fusing of the layers may change one or more properties of the panel or panels. For example, melting and re-solidification of a material of a panel may cause the panel to be one or more of thinner, denser, more stiff, less stretchable, less recoverable, or of a greater yield strength in stretching. Accordingly, in addition to joining panels together, selectively fusing the headgear at different lapped and non-lapped regions may allow control of the performance of the headgear. The fusing of the panels, for example where one or both of the panels are melted or partially melted into each other by welding, may result in the thinning of the panel layup at fused zones. This may particularly be the case where the fusing is provided by the application of pressure at the parts of the panels which are to be fused.

In the exemplary embodiment, the base layer 72 comprises a stretch fabric having stretch in a width-wise direction of the headgear. Optionally the fabric may additionally have stretch in a vertical direction of the headgear. The fabric may comprise a knit fabric.

The base layer may comprise a single panel, with the base layer and preferably the headgear being free from internal and external seams. The potential reduction in the number of panels and thickness at both lapped regions and non-lapped regions of the headgear of the present disclosure when compared to a conventional headgear made from joined laminate or multi-ply materials may provide either location-specific or overall reductions in the thickness of the headgear. Reduced thicknesses, whether in particular locations or across the whole headgear, may provide visual and/or physical perceptions to a patient of reduced bulk. Reduced thicknesses may also provide increased comfort for a patient wearing the headgear.

Any such reduction in the number of panels present at different points of the headgear and its thickness may provide corresponding decreases in the overall weight of the headgear.

In contrast, the engagement layer of the headband region may comprise a material with similar or reduced stretch compared to the base layer 72. The engagement layer may comprise substantially inelastic material. The regions of fused material in the headband region may have increased stiffness compared to the non-fused regions. Additionally or alternatively, the regions of fused material in the headband region may have decreased stretch compared to the non-fused regions. Particular patterns of fusing may therefore result in the headband region 73 having reduced overall stretch compared to the base layer 72. The difference in the stretch properties of the fused and non-fused regions may be utilised to reduce or increase the stretch in selected areas of the headband and/or in selected directions. That is, the shape, orientation and/or the position of the fused areas and the unfused areas may be selected to control the way regions of the headband stretch or to limit their stretch under loading.

For example, it has been found that in some cases, limiting stretch in the headband or regions of the headband, particularly in the generally longitudinal 'horizontal' direction of the headband and/or in an angled or generally diagonal direction (around 45 degrees to longitudinal) may be desirable for a correct and secure fit of the headband. Longitudinal stretching of portions of the headband may contribute to incorrect placement of the over-ear portions during fitting of the headgear to the patient, for example such that the over-ear portions don't locate over the ears or don't locate for best fit over the patient's ears. Similarly, angular, diagonal and/or longitudinal stretching in the over-ear portions may also inhibit correct placement of the over-ear portions and limiting stretch in these regions in one or more directions can prevent the over-ear portions migrating away from the ears under patient movement.

Figures 76 and 113(i) to 114 (iii)illustrate some exemplary configurations of the fused and unfused areas in the headband region 73 to reduce longitudinal stretching of those regions. Referring to Figure, 76, the darker regions represent the areas of fused material and the lighter regions represent areas of unfused material. The left side of the headband (as viewed) in Figure 76 contains two continuous lines of unfused material, with the fused material on the remaining left side of the headband forming a continuous line extending from adjacent the over-ear portions, to adjacent the edge of the headgear 72b. In other embodiments, at least one generally continuous strip or band material extends generally in the longitudinal direction across substantially the full width of the headband between the side edges 72b.

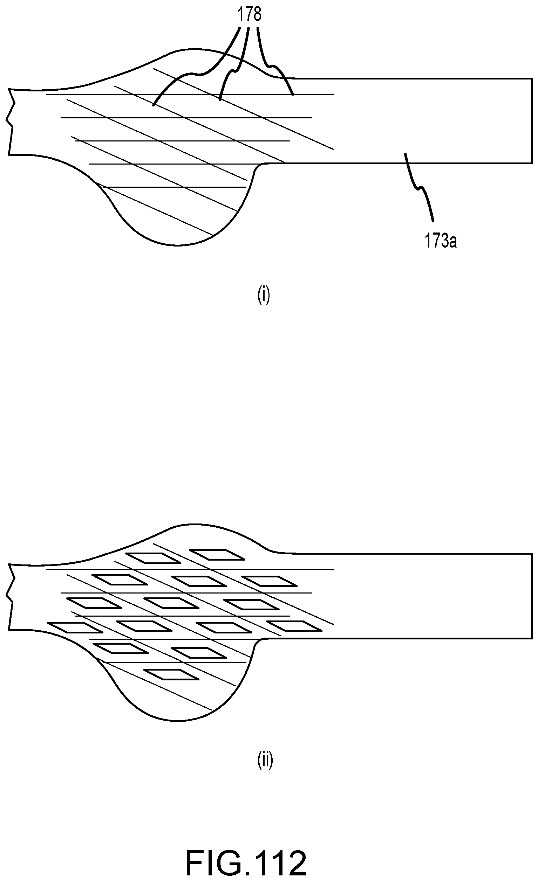

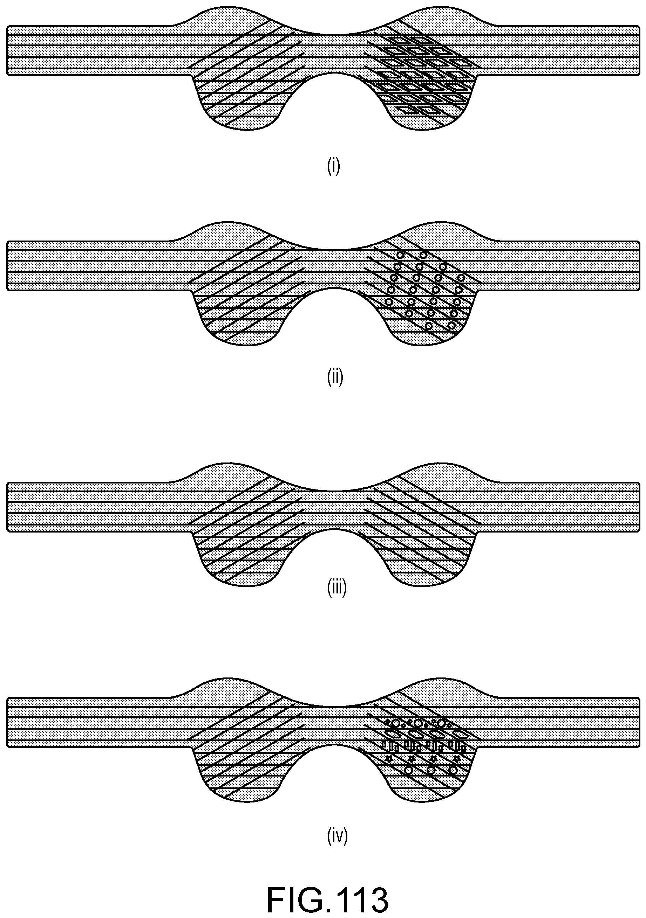

Figures 112(i) to 114(iii) provide further examples of fusing patterns, that is the shape, orientation and/or the position of the fused and unfused areas, illustrating how fusing patterns may be utilised to modify stretch properties of the headband. Referring to Figures 112(i) and to 113(iii), the headband may be fused along lines 178 that generally extend in the direction where little or no stretch is desired. The fused lines may be straight or may curve or have curved portions. The fused lines may be continuous or discontinuous. In the embodiment shown, generally 'horizontal' and generally 'diagonal' weld lines 178 are provided on the over-ear region 174 to limit stretch in those directions. These fused regions may be substantially continuous across the region or regions where limited stretch is desired. For example, a fused region may extend across the full width of the over-ear portion, and along the headband, as shown in Figures 113(i) to (iii), and/or a fused diagonal region may extend between two edges of the over-ear regions.

The headband will be able to stretch at any breaks in the fusing, therefore, where minimal or reduced stretch is required, it is desirable to minimise the size of any breaks in the fusing along the direction of reduced stretch, or ensure that the size of the breaks does not permit stretch beyond a permissible amount. The continuous lines of non-stretch do not need to be perfectly linear.

In the embodiment of Figures 112(i) and (ii), the intersecting diagonal and horizontal fused lines create diamond-shaped pockets of unwelded material 179. These pockets 179 are still free to stretch within the bounds of the encompassing welded regions. This ensures the headband is still able to flex to conform to the patient's head to obtain a secure fit. Other shapes for the unwelded region are envisaged depending on the configuration of the fused regions and any requirements for the surface to receive connectors. Figures 113(i) to 113(iv) illustrate some further non limiting example shapes for the unwelded pockets, including parallelograms (Figure 113(i)), circular pockets (Figure 113(ii)), star shaped and other irregular shapes or groups of shapes (Figure 113(iv)). As illustrated in Figure 113(iv), it is not necessary that all of the pockets have the same shape, one region may contain pockets or areas of different shapes.

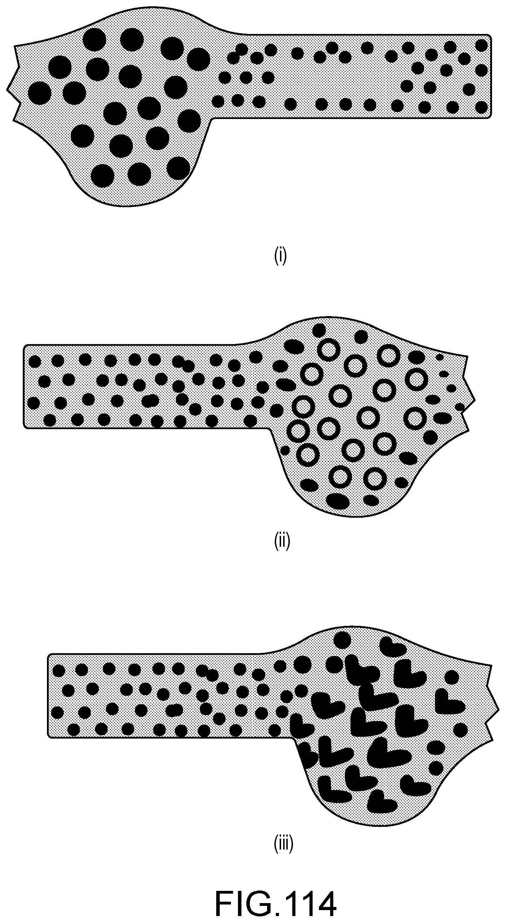

Figures 114(i) to (iii) illustrate alternative weld patterns that limit the stretch of the headband in the same horizontal and diagonal directions. In these images, the light shaded regions indicate fused material and the darker shapes indicate regions of unfused material. The fused material falls along lines where stretch is not desired, in this case, approximately along lines that run generally horizontally across the width of the headband and additionally diagonally in the over-ear regions.

The fused material in the embodiment of Figures 114(i) to (iii) is substantially continuous along the lines of desired reduced strength. The circular, dot, or otherwise shaped areas of unfused material may be placed to appear random and to obscure the lines of limited stretch.

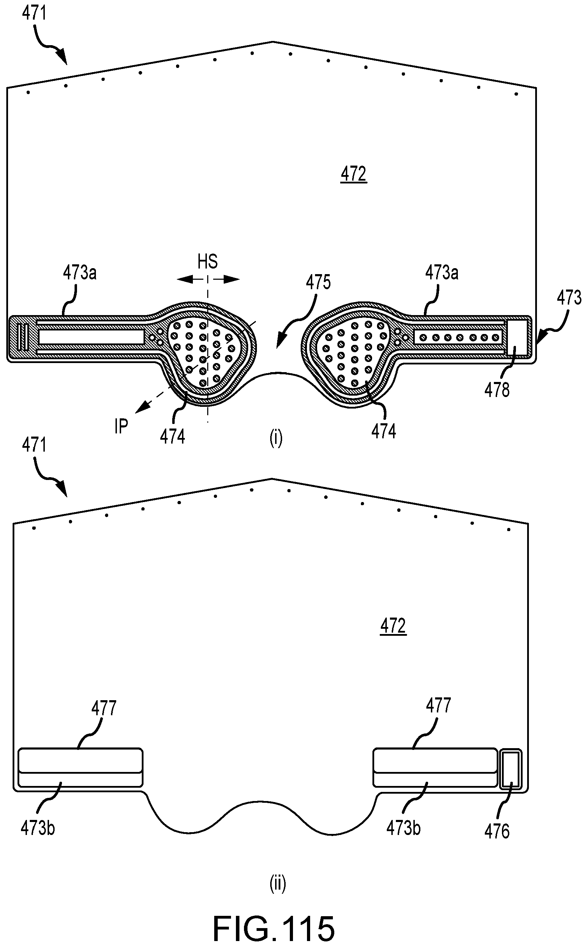

In some embodiments, increased stretch may be desired in at least a region of the headband. For example, increased stretch in the longitudinal (horizontal/circumferential) direction of the headband may be desirable to obtain a tighter fit or better conformance of the headband to the patient's head. Figures 115(i) to 117 illustrate further embodiment bonnets 471, 571, 671 in which the headband 473, 573, 673 has a region of increased stretch 475, 575, 675 at the rear of the headgear, between the over-ear portions 474, 574, 674.

In alternative embodiments, the region of increased stretch may be provided at a different part of the headband 473, 573, 673 and/or there may be more than one region of increased stretch 475, 575, 675. For example, a region of increased stretch may be provided forward of one or both over-ear portions 474, 574, 674. For example, on a front portion 473a, 573a, 673a of the headband, adjacent one of the over-ear portions 474, 574, 674. In such an embodiment where the region(s) of increased stretch are provided on only one front portion 473a, 573a, 673a, the region of increased stretch may be provided on the front portion 473a that is configured to be the overlying portion when the headgear is worn, i.e. the front portion having the hook-type attachment 476, 576, 676 (for securing the headband) on the underside.

In the embodiments of Figures 115(i) to 117, the region of increased stretch 475, 575, 675 comprises a region of the headband in which the base layer 472, 572, 672 of the headgear is exposed by a break or gap in the outer engagement layer. That is, there is no outer-engagement layer in the region of increased stretch 475, 575, 675. Alternatively, or additionally, a region of increased stretch may be provided by a region in which the outer engagement layer is unfused with the base layer 472, 572, 672.

The width of the region of increased stretch 475, 575, 675 is selected depending on the amount of stretch desired and the stretch properties of the base layer 472, 572, 672. A wider region generally provides more horizontal stretch to the headband than a narrower region. The region of increased stretch 475, 575, 675 may also provide increased stretch in other directions, particularly in embodiments where the base layer 472, 572, 672 comprises a material having four-way stretch. The width of the region of increased stretch 475, 575, 675 may be selected to provide the desired stretch while still ensuring the headband has sufficient stability to interface with the respiratory interface components while the over-ear regions remain aligned with the patient's ears. Depending on the properties of the base layer 472, 572, 672 too wide of a gap in the outer engagement layer may result in curling of the lower edge of the base layer upon stretching and/or may make the headgear susceptible to fatigue in the base layer in the region of increased stretch.

The break or gap in the outer engagement layer and/or the length of the region of increased stretch may be between about 5 % and about 50% of the length of the headband, for example between about 10 % and about 35%. In one embodiment, a gap in the outer engagement layer of about 20% of the length of the headband is provided between the over-ear regions 474, 574, 674.

In one embodiment, the break or gap in the outer engagement layer and/or the length of the region of increased stretch may be between about 5 mm and about 35 mm, for example between about 20 mm and about 30mm. In one embodiment shown in Figure 115(i) and (ii), a gap in the outer engagement layer of about 28 mm is provided between the over-ear regions 474.

Additionally, or alternatively, the pattern of the regions of fused and unfused material in the headband region may be configured to provide increased stretch in the horizontal direction in selected areas of the headband. That is, the shape, orientation and/or the position of the fused areas and the unfused areas may be selected to control the way regions of the headband stretch or to provide increased stretch in the horizontal direction under loading.

Referring to Figure 115(i), the darker regions in the headband region denote fused regions, and the lighter areas unfused regions. In the over-ear regions 474 of this embodiment, fused dots are provided in a pattern such that there are parallel columns of unfused material in a direction HS that is substantially perpendicular to the desired horizontal stretch direction. These columns of unfused material allow some stretch in the over-ear portions 474 in the horizontal direction. The pattern of the dots is also configured to reduce or minimise stretch along the direction of interface pull. This is achieved by eliminating or minimising any continuous lines of unwelded material in a direction perpendicular to the direction of interface pull IP. In other embodiments other shapes of fused regions may be used in place of or in addition to the dots shown in Figure 115(i).

It is envisaged that many other shapes and configurations may be utilised for the fused and unfused regions. As one example, Figure 114(iii) illustrates an embodiment having 'V' shaped unfused regions on the over-ear portion, and circular unfused regions elsewhere. The shape of the unfused regions may be selected to best modify the stretch, and/or may be selected to provide a labelling or visual feature on the respective surface. For example, the shape may indicate which component attaches to that region, and/or may indicate a correct attachment orientation.

The size of the fused and unfused regions may vary across the regions of the headband. For example, the enlarged over-ear regions may contain larger regions of unwelded material at a lower density. The headband straps forward of the ear regions may comprise a higher density of smaller unwelded regions. Differences in weld sizes between the over-ear portion and the rest of the headband may help to visually distinguish the regions.

In alternative embodiments, a similar limiting of stretch in certain directions or regions may be obtained by a multi-layer headband comprising a non-stretch layer with one or more cut-outs. The shape and/or the orientation and/or the position of the cut-outs is selected in a similar manner as the non-fused regions of the embodiments described above, to reduce the stretch of a respective region of the headband in one or more directions compared to the regions without cut-outs.

A patient-contacting underside of the headband region may include a tacky surface to provide a region of increased friction between the headgear and the wearer to reduce the incidence of movement of the headgear on the patient's head. The tacky surface may prevent sideways or forward/rearward rotation of the headgear. Such movement of the headgear may cause the patient interface to move out of position and/or may result in reduced sealing between the interface and the patient.

Figures 115(ii) and 116(ii) illustrates an exemplary placement of a tacky surface 477, 577 on an underside of the headband region 473, 573. The height of this tacky surface may be between about 20 % and about 100% of the height of the headband in the overlapping region. In the embodiment shown in Figure 115(ii), a region 473b adjacent the bottom edge of the headband 473 is left free from the material providing the tacky surface. The tacky surface may increase the stiffness of the headgear, so too large a region may decrease conformance to a patient's head. Further, it is envisaged that a clinician may hold one half of the bonnet at the lower edge while pulling the other half of the bonnet around the head to secure. A region at the lower edge of the headgear that is free of the tacky surface may prevent the tacky surface inadvertently sticking to the fingernail or glove of the clinician during this process.

Optionally, a small grip region 478, 578 may be provided on an outer surface of the headband 473, 573. This grip region 478, 578 is positioned on the front portion of the headband that is configured to wrap under and underly the other (overlying) front portion. This grip region 478, 578 is positioned to grip the tacky surface 477, 577 of the underside of an overlying portion of the headband as the headband is wrapped around a patient's head and over the grip region. In addition to providing additional anchoring between the underlying and overlying portions of the headband, the grip region may assist in obtaining a secure fit of the headgear by preventing or reducing slip between these surfaces when securing the hook attachment 476, 576 to the headband engagement layer.

The tacky surface 477, 577 and/or the grip region 478, 578 may comprise an adhesive film or coating, or may comprise a friction layer adhered or bonded to an inner surface of the base layer at the headband region. For example, a material such as Bemis^{™} tape. The tacky surface may be continuous along the length of the headband region, or may comprise discrete regions. The discrete regions may be provided at areas where increased friction is desired. The tacky surface may comprise a linear strip, or may be applied to form a pattern, for example including curved lines and/or dots.

The region of the headband where the small grip region 478, 578 is provided may be an unfused region.

In one embodiment the tacky surface is provided by a polyurethane elastic, neoprene, non-stick silicone, and/or thermoplastic polyurethane layer. The tacky surface may comprise a material suitable for contact with skin and may be provided over substantially all of the underside of the headband, the majority of the underside of the headband, or may only cover portions of the underside of the headband.

### End Fixture

Referring to Figures 76 to 78, Figure 109, and Figures 111(i) and 111(ii), a top edge 72a, 172a, 272a, 372a of the base layer 72, 172, 272, 372 is secured together at a securement point. The top edge 72a, 172a, 272a, 372a of the base layer may be gathered, pleated, folded, rolled or otherwise gathered to reduce the width of the headgear along the top edge 72a, 172a, 272a, 372a.

An end fixture 81 may be provided to mechanically hold the top edge in this gathered configuration.

In one embodiment shown in Figures 78 and 79, the end fixture is a bobble-like component with two side members 82a, 82b that are configured to engage with each other to sandwich the gathered, pleated, folded, of rolled upper edge of the base layer between the two sides 82a, 82b. The two side members 82a 82b may comprise a soft, flexible material such as a fabric. The fabric may be one having a synthetic component to enable the two side members to be fused at a periphery using the techniques described above. In the exemplary embodiment the side members 82a 82b comprise a knitted nylon, optionally an unbroken loop (UBL) material, but other materials are envisaged. Alternatively, the side members may be attached by stitching, adhered together, or otherwise attached, particularly where the material does not include a synthetic or fusible component.

A compliant intermediate layer 83 may be provided between the two side layers. In the embodiment shown, the intermediate layer 83 comprises a foam layer having a cut-out 83a to accommodate the gathered, pleated, folded, or rolled upper edge 72a of the base layer. The intermediate layer 83 may be attached to the two side members 82a, 82b, for example, a first surface of the intermediate layer 83 may be adhered the first side member 82a and the opposite second surface of the intermediate layer 83 may be adhered the second side member 82b. Alternatively, the intermediate layer 83 may be fused to the side members 82a, 82b or may not be attached to the side members 82a, 82b and simply be held between the side members 82a, 82b.

In an embodiment, the adhesion with the intermediate layer may be provided by a polyurethane elastic, and/or thermoplastic polyurethane, or other suitable adhesive or tacky material, for example a copolyamide hot-melt adhesive film or a copolyester hot-melt adhesive film". The adhesive or tacky material may be provided over substantially all of, a majority of, or only a portion of the respective surface of the intermediate member and/or of the respective side member 82a, 82b. The intermediate layer 83 may be heat pressed with the side members 82a, 82b to fuse the layers together.

### Adjustment device

An adjustment device 85 is provided for assembly with the headgear 71 to adjust the wearable length or depth of the headgear. The adjustment device is assembled to be positioned between the headband region and the end fixture 81. The flexible base layer 72 of the headgear passes through the adjustment device 85 and the adjustment device is selectively slidable along the body of the headgear, towards and away from the headband region. The end fixture 81 limits travel of the adjustment device along the fabric thereby prevent inadvertent removal of the adjustment device from the headgear.

The adjustment device 85 comprises a first engagement member 86 and a second engagement member 87, for engaging the body of the headgear at two spaced engagement points. The first and second engagement members 86, 87 are provided on opposite sides of a hinge region 89 such that the first and second engagement members 86, 87 can move towards and away from each other.

The first and second engagement members 86, 87 comprise looped members extending inwards from respective sides of the device. The looped members define first and second apertures 88a, 88b for receiving the body of the headgear. The apertures are sufficiently sized that the body of the headgear can comfortably fit and slide through the apertures, but also sufficiently small that they cause gathering the body member.

The apertures 88a, 88b may have any suitable shape, for example round, oblong, square, D-shaped, keyhole shaped, or tear-drop shaped.

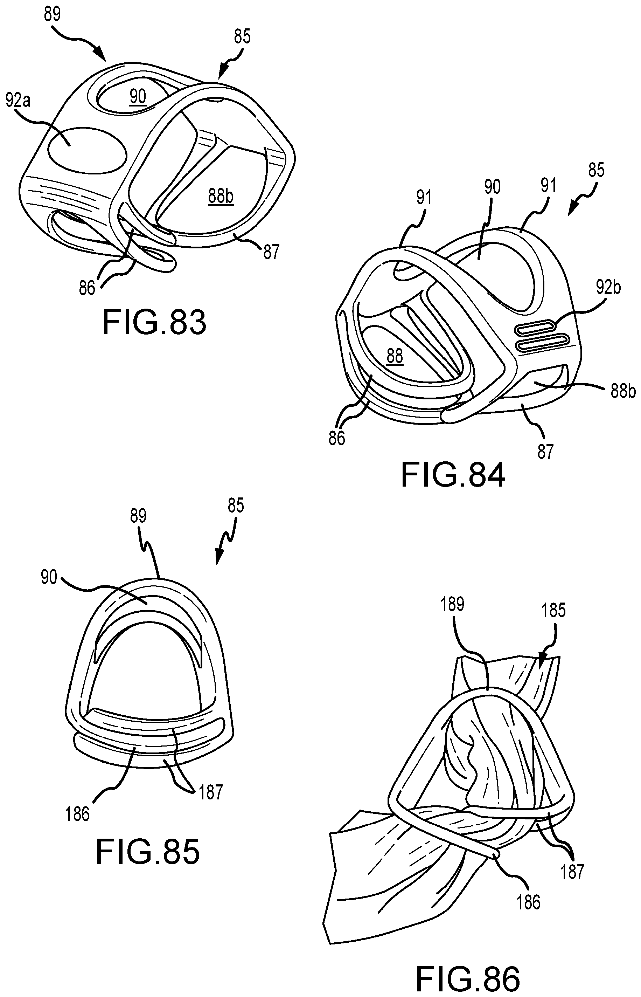

In the embodiment of Figures 83 to 84, the first engagement member 86 comprises a pair of spaced apart loops defining two respective colinear apertures. The two loops may be parallel, as shown, or may converge or diverge. However, alternatively, the first engagement member may comprise only a single member 186, as illustrated for the embodiment 185 of Figure 86.

The device 85 further includes a guide aperture 90 to receive and slide along the body of the headgear. The aperture 90 may have any suitable shape, for example oblong, rectangular, square or round, but is preferably symmetrical about the hinge axis 89. The aperture 90 is sufficiently sized that the body of the headgear can comfortably fit and slide through the apertures.

The guide member 90 is defined between two hinge members 91. In the embodiment shown, the hinge members are arcuate members extending between sides of the device, with an upper apex of the arch aligned with a midline of the device.

The hinge members are integral with the sides of the device but the reduced cross-sectional area at the mid-line of the device enables the device to hinge about the apex of the hinge members 91.

The first and second engagement members 86, 87 are movable towards each other by pressing them towards each other, causing the device 85 to flex about the hinge region. Finger grips 91a, 91b on opposite sides of the device 85, either side of hinge, may provide improved purchase for a user to facilitate pressing the first and second engagement members 86, 87 towards each other. The finger grips 92a, 92b face outward on the side surfaces from which the engagement members extent.

The finger grips 92a, 92b are regions that are spaced from the hinge 89 and may comprise a textured or contoured surface, or may comprise a layer or coating of a material offering improved friction or compliance compared to the body of the device. As an example, in the embodiment of Figures 83 to 85, a first one of the finger grips 92a comprises an oblong, concave recess. A second one of the finger grips 92b comprises two raised transverse ribs. Many other configurations of finger grips are possible.

Referring to Figure 85, the first and second engagement members 86, 87 can be moved towards each other by pressing the sides of the device towards each other at the finger grips 92a, 92b. The first and second engagement members 86, 87 can be moved to a position in which there is substantial overlap between the first and second apertures 88a, 88b. This is a free state of the device 85, in which the device can be moved freely along the body of the headgear towards and away from the headband region.

In the embodiment of Figures 83 to 85 the device 85 has two parallel first engagement members 86. The second engagement member 87 is configured to slide between the two first engagement members as the first and second engagement members are moved relative to each other.

In the free state, the first and second apertures 88a, 88b may be generally aligned with the guide aperture 90 such that the material passing through the device follows a generally linear path.

The hinge region 89 is resilient, and wherein upon release of pressure from the sides of the device, the first and second engagement members 86, 87 move away from each other into a locking state illustrated in Figures 83, 84, and 86. This locking state is the resting state of the device, with the hinge biasing the device into this state.

In the locking position, the first and second apertures are misaligned with each other. The first and second apertures 88a, 88b may also be misaligned with the guide aperture 90. For example, only a small portion of the first and second apertures 88a, 88b, may overlap of there may be no overlay. This misalignment causes engagement regions of the first and second engagement members 86, 87 to grip the body of the headgear to hold the device in position with respect to the material. In this state the body of the headgear to follows a convoluted or torturous path through the device 85 to provide resistance to the headgear body being pulled through the device.

The device 85 can be adjusted from the locking state to the free state by pressing the first and second engagement members towards each other.

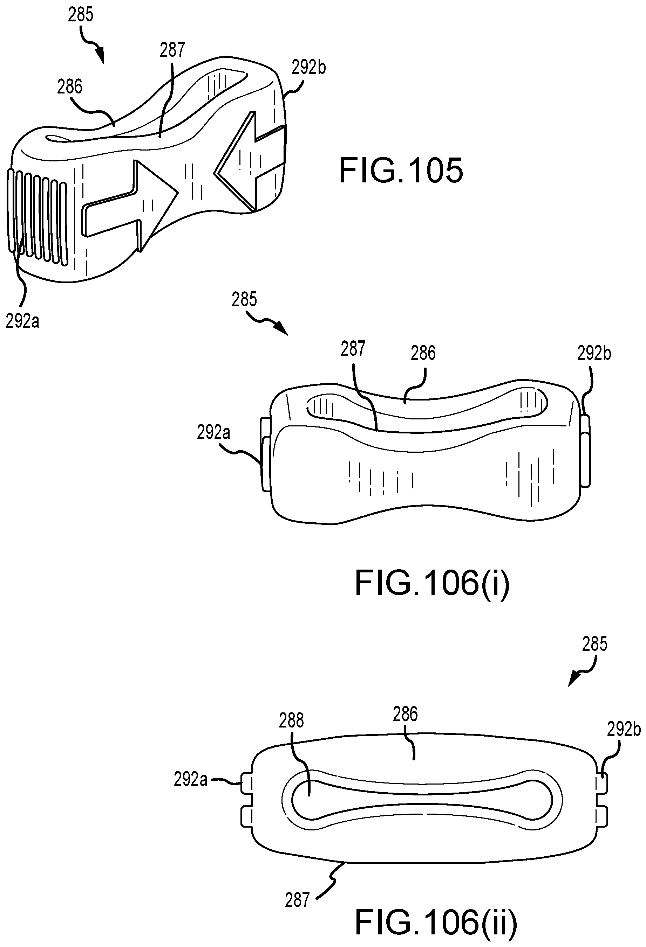

Figures 105 to 107 illustrate an alternative embodiment adjustment device 285 for gripping the flexible body of the headgear. This adjustment device 285 is smaller and less visually intrusive than the embodiments of Figures 83-86. This adjustment device 285 comprises a flexible body having opposite first and second sides 286, 287 and first and second ends 292a, 292b that together define an aperture 288 therebetween to receive the flexible body of the headgear.

The opposite first and second ends 292a, 292b of the device 285 are movable towards and away from each other to adjust the device 285 between a locking state and a free state. In the locking state the device 285 grips the flexible body of the headgear in the aperture 288, to hold the device in position relative to the material. In the free state, the grip of the device is released sufficiently to enable the device to move along the material and to thereby adjust the position of the device relative to the material.

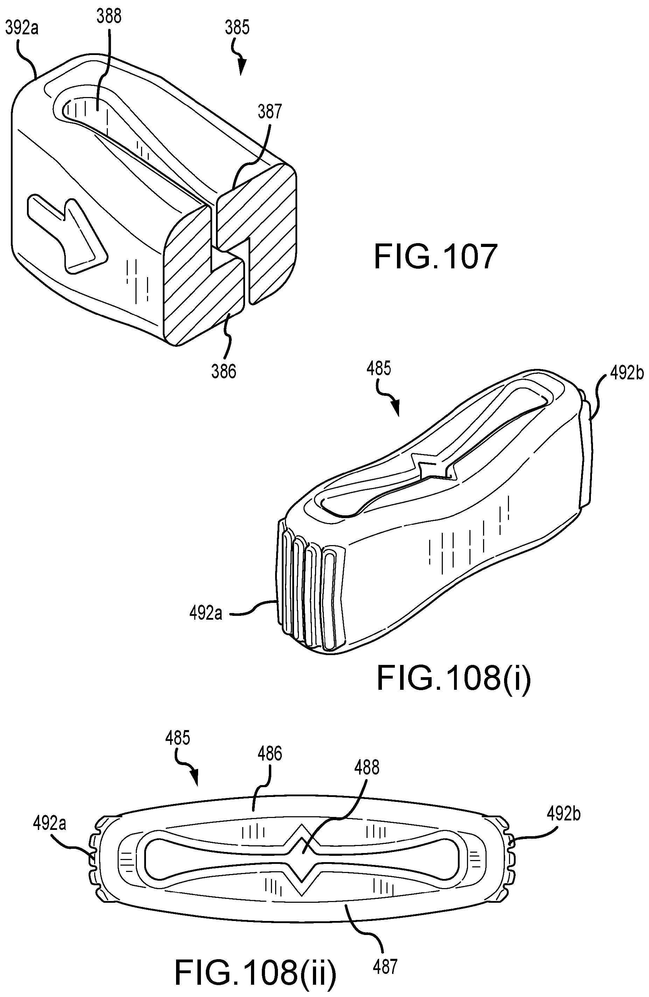

The side walls 286, 287 each have convexly curved inner surface, curved such that the aperture 288 is narrowest at or near a centre of the aperture. This occurs at a midline of the device, intermediate the first and second ends 292a, 292b. Referring to Figure 107, in the embodiment shown, the aperture 288 has an hourglass-like shape, with a necked region at its centre, and is widest nearest the two ends of the device 292a, 292b. The headgear is pinched between the convex side walls and held tightly by the necked region of the device when the device is in the locking state.

The device 285 is resiliently biased towards the locking state such that in a resting state, in the absence of applied forces, the device grips the headgear material in the aperture 288. To release the material, a user must press the first and second ends 292a, 292b towards each other, thereby causing the side walls 286, 287 to bow outwards about end hinge points, widening the central (necked) portion of the aperture 288. The first and second ends 292a, 292b may comprise finger grips to facilitate pressing the ends towards each other. For example, finger grips may be provided by protrusions, depressions, or a textured surface.

Optionally, the adjustment device may include an internal grip feature such as a protrusion to strengthen the grip of the device on the received material. Figure 107 illustrates one embodiment device 386 in which the inner surfaces of the first and second side walls comprise protrusions in the form of oppositely projecting, complementary steps 386, 387. With one step 387 positioned at or towards a top of the device, and the opposite step positioned at or near a base of the device. The headgear is pinched between the side walls and held tightly between the two steps 386, 387 when the device is in the locking state. In the locking state shown in Figure 107, the two steps 386, 387 overlap, such that when the device is in the locking state, material passing through the device is bent, following a non-linear path, creating resistance to the material being pulled through the locked device.

When a user presses the device to the free state, the two steps move from each other, enabling the device to be slid along the headgear to thereby adjust the wearable length of the headgear. In other embodiments, other forms of internal gripping features may be provided, for example, alternatively shaped projections or steps, notches, or textured surfaces.

In other embodiments, the internal grip feature may be provided by an alternative protrusion, or a notch or other recess to assist with gripping the fabric body on one or both side walls. Figures 108(i) and (ii) illustrate a further embodiment comprising a notch in both side walls 486, 487 at the necked region of the aperture 488. In the embodiment shown, a pair of notches located opposite each other, approximately at a mid-point of the device 485 and opposite each other. These notches may assist to grip the flexible body of the headgear. The notches may assist the side walls 486, 487 to bend or bow outwards when the first and second ends 492a, 492b are pressed towards each other to widen the central portion of the aperture 488. In the embodiment shown, the notches have a triangular profile, but other shapes are possible.

The top and/or bottom surfaces of the device 285, 385, 485 may also be contoured to sit closely against the curved surface of a wearer's head. That is, the height of the device may decrease towards a midline of the device. In the embodiments shown 285, 385, both the top and bottom surfaces are contoured. This means the device can be used in either orientation and cannot be inadvertently used 'upside down'.

The device comprises a resilient material, preferably a relatively soft material. Non-limiting examples include a thermoplastic elastomer, silicone, or a thermoset elastomer. The device may be a unitary component, formed from a single material. Alternatively, the device may be integrally formed from two or more materials.

### Side connectors

Flexible connectors 93 may be provided to couple interface components such as the lateral arms of the above-described patient interface assembly to the over-ear regions of the headgear. Figures 87 to 90, illustrate one embodiment of the flexible connector 93 where the connector is a multi-layer connector.

The connector comprises a patient interface connection area 97 adjacent a first end of the connector, on a front surface of the connector, for connecting to the connection pad 52 of a respective lateral arm. The patient interface connection area 97 may comprise a hooked surface connection for engaging with a looped surface.

Two spaced apart, upper and lower connection areas 98a, 98b for coupling to the headgear are provided on an under surface of the device at a second end of the device. The upper and lower connection areas 98a, 98b may comprise a hooked connector for engaging with a looped surface.

The flexible connector 93 may comprise outer fabric layers 94, 95, with a stiffening layer 96, sandwiched between. The stiffening layer is typically a flexible polymer layer and may, for example, comprise a nylon sheet. The stiffening layer is smaller than the outer fabric layers 94, 95 such that the stiffening layer does not extend fully to the peripheral edge of the connector, thereby softening the edge of the connector. Figure 89 illustrates the position and size of the stiffening layer 96 with respect to the patient-facing layer 94. In an embodiment, the edge 96a of the stiffening layer is spaced about 2.5mm from the edge of the patient-facing layer.

The front outer fabric layer 95 may comprise an engagement surface to enable the attachment of other components to the surface of the connector. In an exemplary embodiment, the front outer layer 95 may comprise unbroken looped (UBL) fabric.

The patient facing fabric layer 94 is a comfort layer, for example comprising a material that is soft on skin, unlikely to pinch skin. In one embodiment, the patient facing fabric layer 94 may comprise the same or similar fabric to the base layer 72 of the headgear 71 described above. The comfort fabric layer maybe larger than the front layer 95 and the stiffening layer, such that the edge 94a of the inner fabric layer extends around the perimeter of the connector, creating a soft edge to protect the skin from the stiffening layer 96.

In one embodiment, the stiffening layer is offset about between about 1mm and about 2.5 mm inwards from the perimeter of the comfort layer. For example, the stiffening layer may be offset about 2.5 mm inwards from the perimeter of the comfort layer and about inwards from the front layer. However, it will be apparent that other configurations are possible.

The patient facing fabric layer 94 and/or the front outer fabric layer 95 preferably each comprise a material that is fusible using the selected fusing method. The layers should have sufficient density such that the stiffening layer 96 does not melt through the outer layers during the fusing process, and such that the edges do not significantly fray or curl.

In some embodiments, the patient facing fabric layer 94 and/or the front outer fabric layer 95 comprise non-stretch fabrics, or fabrics with low levels of stretch. In other embodiments, the layers may be fused so as to minimise any stretch in the connector as a whole.

A patient-contacting underside of the flexible connector 93 may include a tacky surface to provide a region of increased friction between the headgear and the wearer to reduce the incidence of movement of the connector on the patient's face. The tacky surface may comprise an adhesive film or coating, or may comprise a friction layer adhered or bonded to an inner surface of the patient facing fabric layer 94. The tacky surface may be continuous along the length of the connector, or may comprise discrete regions. The discrete regions may be provided at areas where increased friction is desired. The tacky surface may comprise a linear strip, or may be applied to form a pattern, for example including curved lines and/or dots.

In one embodiment the tacky surface is provided by a polyurethane elastic, neoprene, non-stick silicone, and/or thermoplastic polyurethane. The tacky surface may comprise one suitable for contact with skin and may be provided over substantially all of the patient facing side of the connector, the majority of the patient facing side of the connector, or may only cover portions of the patient facing side of the connector 93.

The layers of the connection member may be fused together using any of the methods described above in relation to the headband of the headgear. The connection member may comprise areas of fused material and areas of unfused material, creating a pattern such as the one illustrated in Figure 90. The pattern created by the areas of fused material and areas of unfused material is visible on at least the outer (non-patient facing) side. The patterning may be utilised to indicate a correct orientation of the connector, to identify a size or other property of the connector side, or may be a pattern that corresponds with a pattern or connector zone of the headgear.

The pattern may also be selected to provide connection zones at parts of the connector for the attachment of further components or connectors, such as the chin strap 1500 described below. For example, such connection zones may include a larger region of unfused UBL, for stronger attachment with hooked connectors.

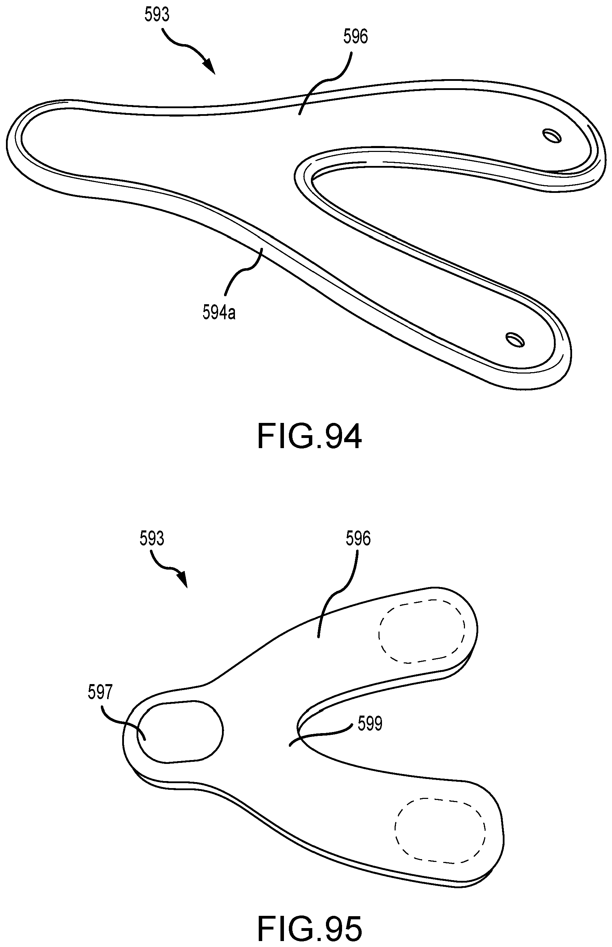

Figures 94 to 104 and 121 to123 illustrate details of exemplary further embodiment flexible connectors comprising a comfort surface at least on a patient facing side of the connector and optionally a stiffening component or layer. The comfort surface may comprise a fabric or a non-fabric material. This patient-facing comfort surface may be textured to reduce the potential surface contact with the patient and thereby minimise potential for the connector to feel 'tacky' or 'sticky'.

The connectors increase in width from the first end to the second end. A patient interface connection area is provided proximal a first end of the connector, and at least one connection area is provided proximal a second end of the connector for coupling to the headgear.

Figures 94 to 99 and 121 to 123 illustrate three further embodiment flexible connectors 593, 693, 1793 comprising a stiffening layer 596, 696, 1796 and a comfort layer 594, 694, 1794. As for the previously described embodiment, the connector 593, 693, 1793 comprises a patient interface connection area 597, 697, 1797 proximal a first end of the connector. Two spaced apart, upper and lower connection areas 598a, 598b, 698a, 698b, 1798a, 1798b are provided for coupling to the headgear. The comfort layer may be a non-fabric layer.

The patient interface connection area 597, 697, 1797 is provided on the outer, non-patient facing surface of the connector, for connecting to the pad 52 of a respective lateral arm, or to another component. The headgear connection areas 598a, 598b, 698a, 698b, 1798a, 1798b are provided on a patient-facing, under surface of the connector. The patient interface connection area 597,697, 1797 and the upper and lower connection areas 598a, 598b, 698a, 698b, 1798a, 1798b may each comprise connection pad or region. The connection pad or region may comprise a mechanical fastener, such as a hooked connection pad or region for engaging with a looped surface, or a looped pad for engaging with a hooked surface.

The stiffening layer 596, 696, 1796 comprises a material that is harder and substantially more rigid than the material of the comfort layer 594, 694, 1794. For example, the stiffening layer may comprise a relatively inelastic thermoplastic material such as polypropylene, polyethylene, nylon, PET, or polyurethane, depending on the material of the comfort layer. This stiffening layer 596, 696, 1796 enables the connector 593, 693, 1793 to hold its shape while preferably still permitting some flexing of the connector, and enables the connector to transmit loads and withstand torsional loading during use without buckling or twisting excessively.

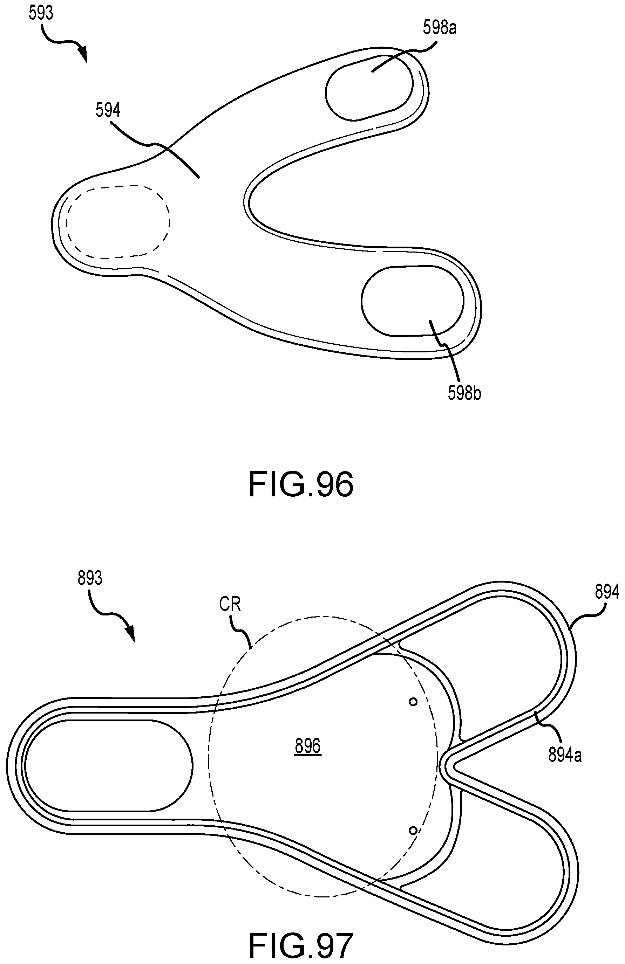

The stiffening layer 596, 696, 1796 may extend across substantially all or most of the connector 593, 693, 1793, or may just be provided in one or more regions that require stiffening. In some embodiments, the stiffening layer may be a unitary member. In other embodiments, the stiffening layer may consist of multiple components, for example that can move relative to each other. Figures 97 and 121 to 123 illustrate two embodiments of the connector in which the stiffening layer 596, 1796 is a unitary member but which does not lie along the full length of the connector 593, 1793. In these embodiments, the stiffening layer 596, 1796 extends from the connector first end and through a central region of the connector 593, 1793 but not to the second end of the connector. This enables the second end of the connector 593, 1793 to flex for better conformity and thereby a stronger connection to the headgear.

Other shapes, positioning or configurations of the stiffening layer or other stiffening components are envisaged, to prevent buckling and twisting of the connector in use. The stiffening layer is preferably at least provided in a central region CR of the connector (indicated in Figure 97), where tendency for buckling or twisting is generally highest. The stiffening layer may follow the general shape of the connector or may have a different shape that is selected to support loading that the connector experiences in use.

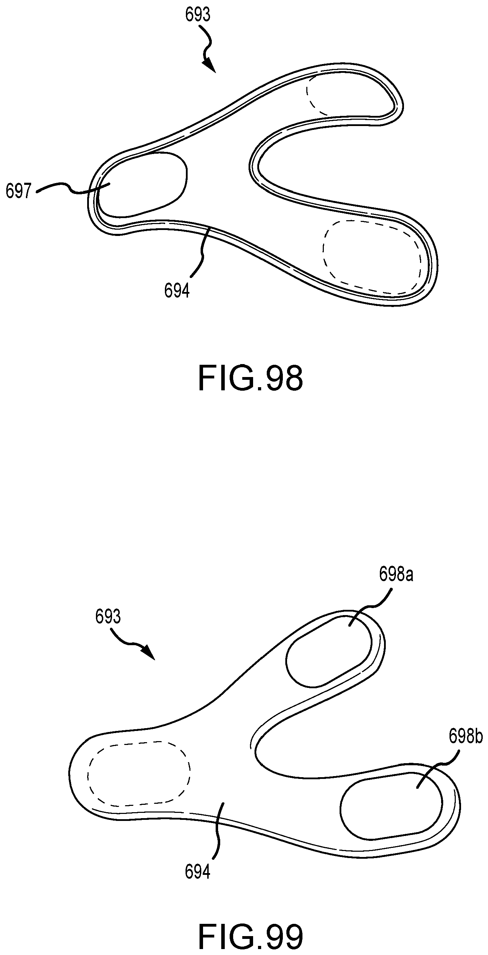

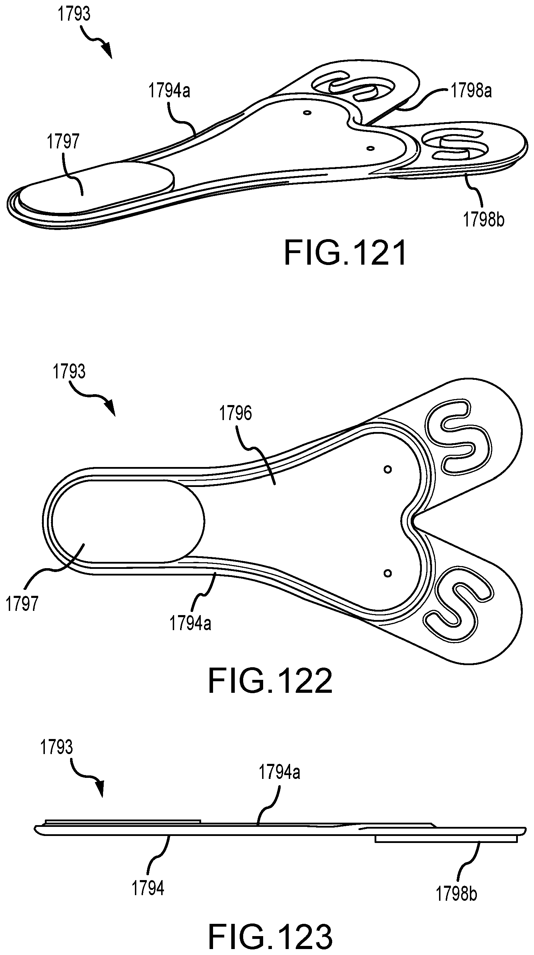

The stiffening layer may be generally planar, as shown for the connector 593 of Figures 94 to 97 and the connector 1793 of Figures 121 to 124(iii), or it may be curved as shown in the embodiment 693 of Figures 98 and 99. The curved profile of the connector 693 of Figures 98 and 99 is shaped to generally follow the contours of a wearer's face. That is, a patient facing surface of the connector 693 has a concave curvature, and the outward facing surface of the connector 693 is convex.

This curved profile may advantageously reduce the likelihood the "flatness" of the arm pulls the connector from the headgear. The curved profile reduces the force required to bend/conform the arm around the face and may reduce the required connection force between the connector and the headgear and/or the interface assembly. The curved profile may therefore assist to retain connection between the connector and the headgear.

This curved profile may advantageously move the mid region of the connector outward from the face near a patient's eyes, reducing the risk of contact between the connector and the eye region. It may also reduce the likelihood of pressure points developing between the mid region of the connector and the patient's face.

The stiffening layer may have substantially uniform thickness, or the thickness may vary across the connector or in different regions of the connector.

The comfort layer 594, 694, 1794 comprises a relatively softer material provided on at least the patient facing side of the connector. The comfort layer 594, 694 may comprise a compliant, optionally non-fabric material. The patient facing surface of the connector is shaped to be generally smooth and free from projections. The patient facing surface may comprise a textured surface to reduce the potential surface contact with the patient and thereby minimise potential for the connector to feel 'tacky' or 'sticky'. The comfort layer typically extends across substantially all of the connector 593, 693, 1793 on the patient-facing side and covers a larger area than the stiffening layer 596, 696, 1796 extending beyond the periphery of the stiffening layer 596, 696, 1796. This ensures that only the softer comfort layer 594, 694, 1794 will come into contact with the patient, protecting the patient from contact with the relatively harder edges of the stiffening layer.

As one example, in the embodiment of Figure 97, the peripheral edge 96a of the stiffening layer 596, 696 is spaced about 2.5mm from the peripheral edge of the comfort layer at their closest point. However, this spacing may be smaller or larger depending on the properties and/or geometry of the connector, the properties and/or geometry of the comfort layer, including the presence of any edge features as will be described in more detail below.

The comfort layer may comprise an elastomeric material. Non-limiting examples include silicone, foamed polymer or a thermoplastic elastomer. Alternatively, the comfort layer may at least in part comprise a fabric. The comfort layer 594, 1794 may be over-moulded or co-moulded with the stiffening layer 596, 696, 1796 to ensure a strong connection between the layers.

The geometry of the stiffening layer may be shaped or comprise features such as cut-outs, scored features, recesses or apertures to manipulate the stiffness of the stiffening layer and/or strengthen the connection with the comfort layer. In some embodiments, the stiffening layer may include cut outs adjacent the first or second ends, at the connection points. Such cut-outs may increase flexibility at the connection point with the interface and/or headgear and thereby improve the strength of the connection with the interface and/or headgear. In other embodiments, the stiffening layer may include cut-outs in at least a middle portion of the stiffening layer to manipulate the flexibility / stiffness of the stiffening layer in different directions.

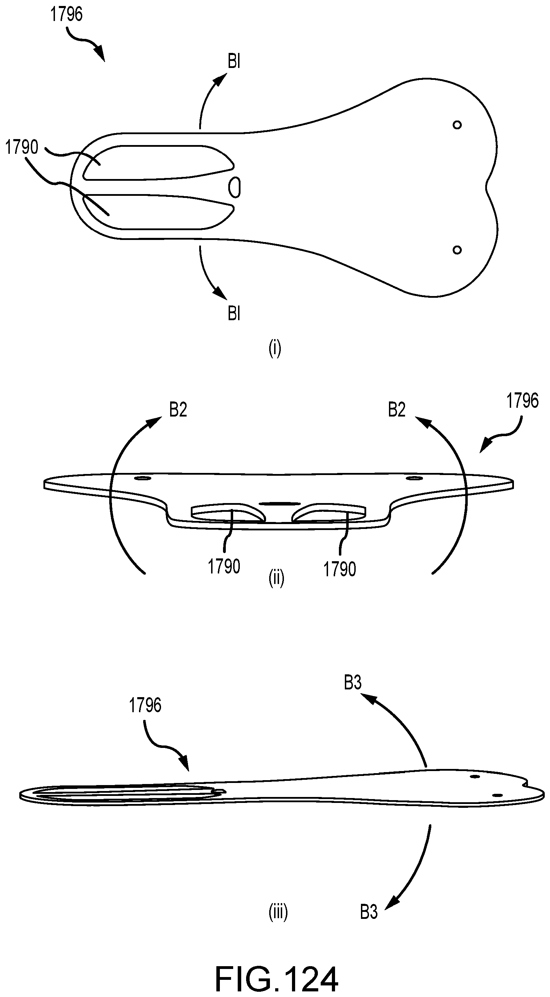

Referring to Figures 124(i) to (iii), the stiffening layer 1796 is stiff in the in-plane direction B1 (Figure 124(i)) and out of plane direction B2 about its longitudinal axis (Figure 124 (ii)), but flexible in an out of plane direction B3 about a transverse axis (Figure 124(iii)). This flexibility allows the side connector to curve to fit around a patients face and facilitates a secure connection at both ends of the connector. The stiffness in the directions B1 and B2 enables load transfer along the connector and prevents twisting of the connector.

Cut-outs 1790 may be provided in the stiffening layer 1796 adjacent a first end of the connector 1793 to enhance flexibility. The cut-outs 1790 may assist with engagement of the first connection pad 179, for example if the components are overmoulded.

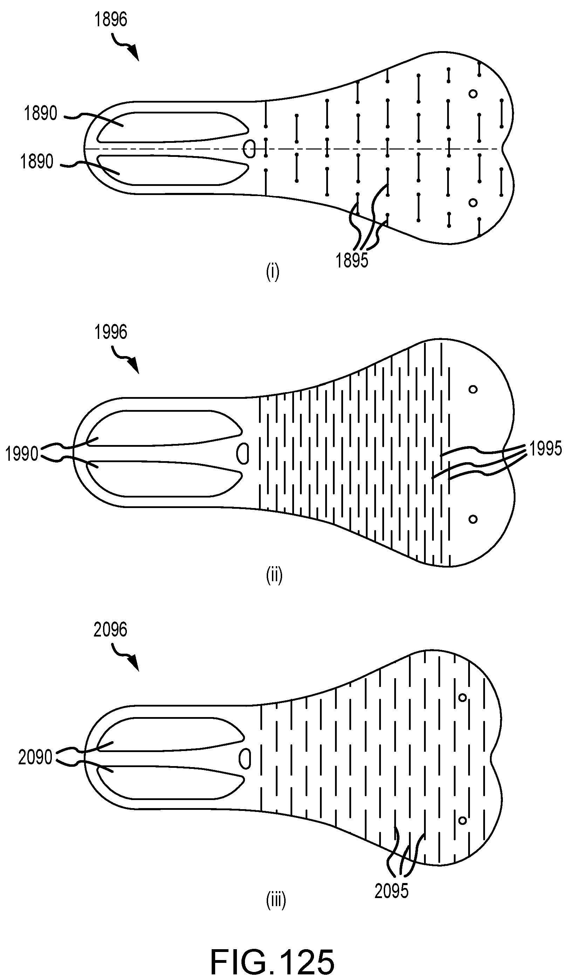

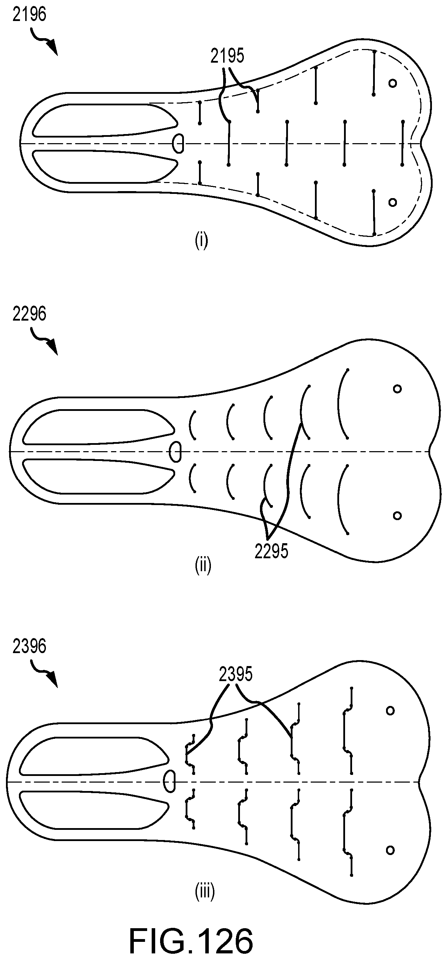

Figures 125 (i) to 127 provide examples of exemplary embodiment stiffening layers 1896, 1996, 2096, 2196, 2296, 2396, 2496 having a pattern of thinned regions or cut-outs in the form of slits, 1895, 1995, 2095, 2195, 2295, 2395, 2495 of various shapes, sizes, and pattern densities, to increase flexibility in the direction B3 to a desired level. In some embodiments such as those in Figures 125(ii), 126(ii), and 126(iii) the slits or thinned regions 1995, 2295, 2395 are provided on a central portion of the connector. In other embodiments, the area having slits may extend to the second end of the connector.

Cut-outs 1890, 1990, 2090 may be provided in the stiffening layer 1896, 1996, 2096 adjacent a first end of the connector to enhance flexibility. Cut-outs 1890, 1990, 2090 may assist with engagement of the first connection pad.

In some embodiments such as those in Figures 125(i) to 125(iii), the slits or thinned regions 1895, 1995, 2095 may extend to the periphery of the stiffening layer. In alternative embodiments, such as those in Figures 126(i) to 127, the slits 2195, 2295, 2395, 2495 may terminate at points spaced in from the periphery of the stiffening layer, leaving the peripheral edge of the stiffening layer unbroken. An unbroken periphery may advantageously increase stiffness in the directions B1, B2 where it is desired.

The slits or thinned regions may be substantially straight, or they may be curved or otherwise shaped, for example, they may be linear, arcuate, semi-circular, angular lines, or formed from a combination of straight and curved portions. A given stiffening layer may comprise slits or thinned regions of a variety of different shapes. Non-linear slits 2295, 2395, 2495 such as those in Figures 126(ii), 126 (iii), and 127 may each define a flap that moves relative to the rest of the body of the stiffening layer during flexing. This may result in a greater increase in flexibility compared to straight slits.

The slits or thinned regions may be arranged in rows and/or columns. The rows or columns or slits or thinned regions may be aligned, for example as illustrated in Figures 125(ii) and 126(iii) or they may be staggered, for example as illustrated in Figures 125(i) to 126(i). The spacing, positioning, and size of the slits or thinned regions may be such that there are minimal or no overlap between parallel rows, or so there is significant overlap.

The slits or thinned regions may all be the same size, or they may be of varying width and/or length. For example, larger slits or thinned regions may be provided in wider regions of the stiffening layer and/or where more flexing is required. The orientation of the slits or thinned regions may be consistent across the stiffening layer or may vary. For example, in embodiments where these are arranged in rows or columns, the orientation may vary between rows or columns. Figure 126(iii) illustrates one example embodiment in which each slit 2395 has a three-sided central portion that defines a generally rectangular flap or tab, with two transverse legs of the slit extending from either side of the central portion. The transition between the sides of the central portion and to the transverse legs may be angular or curved. The slits of the embodiment of Figure 126(iii) are arranged in two longitudinal rows, symmetrically about a central longitudinal axis of the stiffening layer. Alternatively the stiffening layer may include more or fewer rows or columns of slits. Adjacent columns may be aligned or staggered. In the embodiment shown, the dimension of the slits in the transverse dimension of the increases along the stiffening layer, as the width of the stiffening layer increases. In alternative embodiments the slits may all have substantially the same size, or may vary in another dimension.

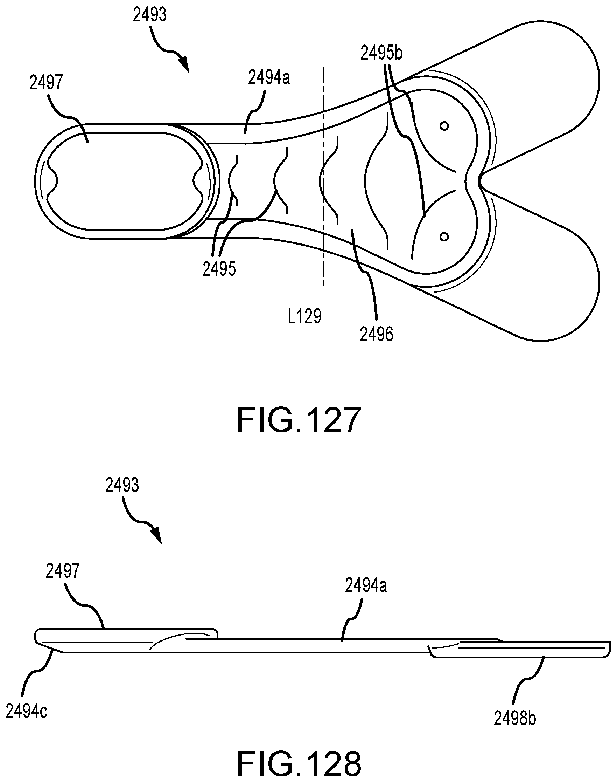

The slits or thinned regions may all be the same shape and/or orientation, or the shape and/or orientation may change. For example, in the embodiment shown in Figure 127, two additional curved slits 2495b are provided nearest the connection point 2498b for the headgear. These curved slits are symmetrical and generally convex towards a midline of the connector and convex to the respective headgear connection point 2498b.

Other shapes, slits, slots, scored features, recesses or apertures are envisaged to tailor the flexibility of the stiffening layer. The shape, density, and position of such features can be selected based on the desired level of flexibility, and accounting for the size, thickness, and material of the stiffening layer, and the features of the comfort layer to which the stiffening layer is bonded.

The connection pads 597, 598a, 598b, 1797, 17981, 1798b, 2498b on the connector may be over-moulded with the comfort layer and/or the stiffening layer. Optionally, the connector pads may include one or more apertures, for example, at either longitudinal end of the pad, which enables the overmoulded material to pass through during manufacture to creating a stability portion or spine along at least part of the length of the connection pad.

The connection pads may be positioned in a recess in the comfort layer such that only a portion of the thickness of the connection pads protrudes from the connector. In some embodiments, the connection pads may have one or more slits or slots to improve flexing of the connector pad. In one embodiment, the connection pads include slits in a pattern that may include, for example, alternating columns of slits. Each column may include arched and/or semi-circular slits with shapes that face the opposite direction to, and are at least partially offset from, the columns on the other side. Portions of the columns of slits may overlap in the longitudinal direction of the hook pad. Other arrangements of slits are possible, such as arrangements described in PCT/NZ2016/050041.

In some embodiments, the connector may be configured for use with a 'comfort sleeve' made of fabric or an alternative comfort material intended to be in contact with the patient's face. The comfort sleeve can be placed over at least part or substantially all of the connector. The comfort sleeve may comprise a plush or wicking material. The comfort sleeve may be provided as an option to clinicians who prefer to have an alternative material contacting the patient to the material of the connector.

As well as extending beyond the periphery of the stiffening layer, 596, 696, 1796 comfort layer 594, 694, 1794 preferably also wraps around the peripheral edge of the stiffening layer 596, 696, 1796. The comfort layer may project forward of a front surface of the stiffening layer and may overlap at least an edge region of the stiffening layer 596, 696, 1796 on the outward face of the connector. This enhances the retention of the stiffening layer 596, 696, 1796 with respect to the comfort layer 594, 694, 1794 and also protects the patient from contact with the edge of the harder stiffening layer 596, 696, 1796.

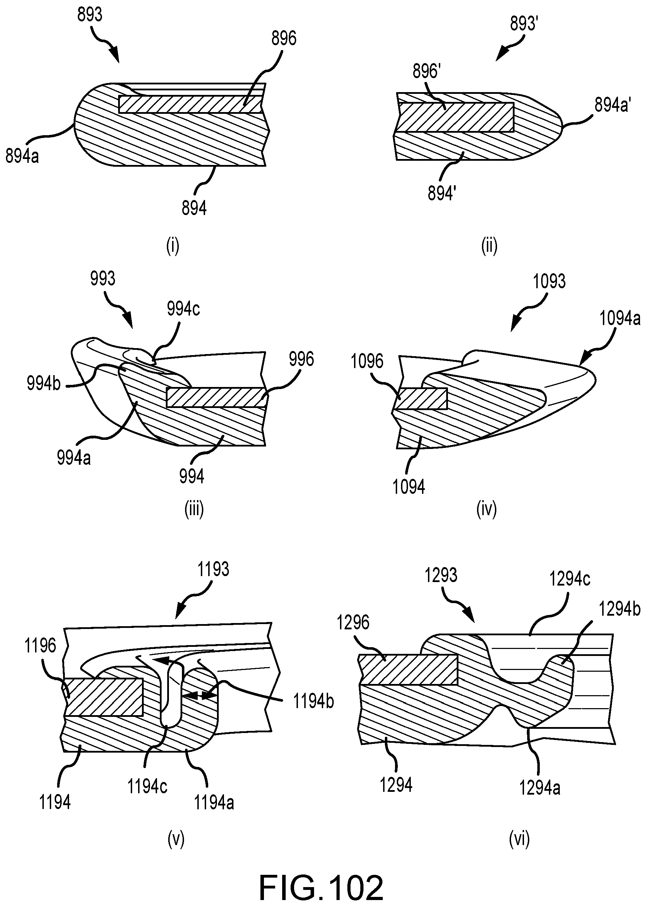

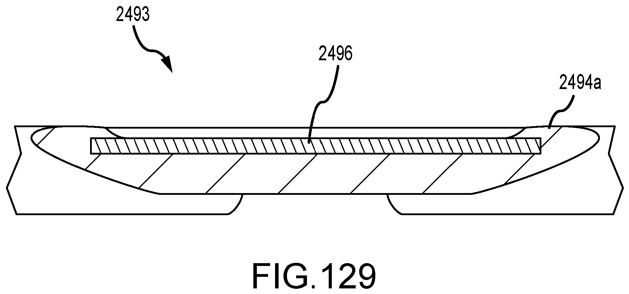

Referring to Figures 102(i) to 102(vi) and 129, the peripheral edge 594a, 894a, 994a, 1094a, 1194a, 1294a, 1749a, 2494a of the comfort layer may comprise an edge feature, shaped to further enhance comfort and reduce discomfort, marking or damage from contact or rubbing. The edge profile may be shaped to increase the compliance and/or flexibility and/or compressibility of the connector at its edge. The edge feature may extend around the entire periphery of the connector, as illustrated in the connectors 593, 693 of Figures 94 to 99. Or may extend only around a portion of the connector 1793 as illustrated in the embodiment of Figures 121 to 123 and 127 to 129.

In some embodiments, and as illustrated in Figures 121 to 123, it may be desirable to omit the edge feature from the portion of the connector adjacent the second end to improve conformance of that part of the connector to the headgear. Omitting the edge feature in this region may also reduce potential discomfort to a patient lying prone on the connector in embodiments where the lip feature includes a protrusion.

Optionally, and as illustrated in the connector embodiment 2493 of Figures 127 to 129, it may be desirable to omit the edge feature from the portion of the connector adjacent the first end such that the edge feature is provided around the periphery of the stiffening region 2496. In this embodiment, the edge feature 2494a does not extend around the connector pad 2497 at the first end. Adjacent the connector pad, the edge feature 2494a may smoothly transition from the tapered profile of Figure 129 to the rounded edge extending around the connector pad 2497.

Referring again to Figures 102(i) to to 102(iv) and 129, in the simplest form, the comfort layer 594 of the connector 593 may be rounded (Figure 102(i)) at its perimeter, and free from sharp or abrupt edges. The comfort layer also preferably encases the perimeter edge of the stiffening layer 596, 896, in a manner to form a raised lip on the front surface of the connector. This raised lip provides additional protection from the edge of the stiffening layer when the connector is deformed.

Alternatively, the comfort layer 894, 994, 1094 of the connector 893, 993, 1093 may be tapered (Figures 102(ii) to 102(iv) and 129) such that the thinned edge of the taper deflects on contact with and/or due to movement of the patient's face. The amount of deflection and the ease of the deflection depends on the profile of the edge feature 894a, 994a, 1094a. For example, a wider taper (for example, as shown in Figure 102(iv)) may enable a higher degree of deflection. However, the edge feature should not be so long the edge of the connector interferes with facial features, such as by causing the connector to be close to the wearer's eye. In the embodiment shown in Figure 102(iv), the width W of the taper is about 1.5mm, however in alternative embodiments, the taper may be narrower or wider.

A steeper taper angle away from the wearer's face, as illustrated in Figure 102(iii) may also reduce the likelihood the edge of the connector interfering with facial features. The steeper taper angle may also reduce the likelihood that the edge is inadvertently folded under the connector such that it catches between the patient and the underside of the connector. A steeper taper angle also allows for a longer deflecting edge for a given connector footprint. A longer edge allows the thickness to taper more gradually.

Additionally or alternatively, the edge 994a, 1194a, 1294a, 1394a may comprise a flexing or hinging fin 994b, 1194b, 1294b, 1394b that is configured to flex, hinge, or roll upon contact with a wearer's face, thereby enhancing conformability of the connector to the wearer's facial contours. The edge region 994a, 1194a, 1294a, 1394a may comprise a depression or recess 994c, 1194c, 1294c, 1394c which at least partly defines the fin and enhances hinging of the fin and/or provides a void into which the fin can deflect or folds inwards upon contact or pressure from the wearer.

The thickness of the fin is selected to be sufficient to ensure the fin is self-supporting and to prevent inadvertent deflection of the fin towards wearer, particularly upon deflection of the connector. However, the fin should not be so thick as to create a pressure point or hard edge.

In some embodiments, the fin may be formed by an edge having a U-shaped or V-shaped cross-sectional profile. In embodiments having a U-shaped profile, the fin extends approximately at a right angle (90 degrees) to the wearer's face and is generally able to fold inwards readily. Embodiments in which the fin extends at a shallower angle from the wearer's face generally require more force to fold or deflect the fin, which may be advantageous by preventing premature folding of the fin.

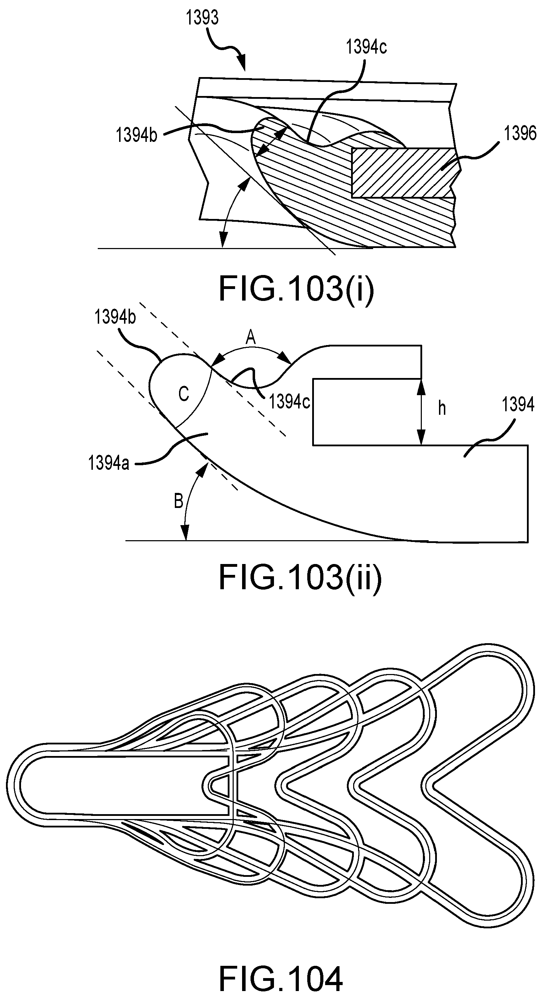

Figures 103(i) and (ii) show an exemplary embodiment fin 1393 in which the fin extends at an angle B that is non-orthogonal to the wearer. Referring to those figures, it is envisaged that in other suitable embodiments the peripheral edge or fin of the patient facing surface could extend at an angle B between 0 and 90 degrees. The angle A of any recess may be any angle less than 180 degrees, and the peripheral edge or fin of the connector may have a taper angle C of 0 to 30 degrees. In the embodiment shown, the fin has a taper angle C of about 7 degrees, and a thickness of about 0.6 mm but the taper angle and/or thickness could be smaller or larger. In one embodiment, the fin has a thickness of about 0.25 mm.

The height h marked in Figure 103(ii) indicates the thickness of the stiffening layer. This thickness is selected to be as thin as possible to minimise the overall thickness of the connector without compromising the function of the connector such that the connector can transmit loads and withstand torsional loading during use without buckling or twisting excessively. The thickness will be dependent on the material properties, size, and shape of the connector.

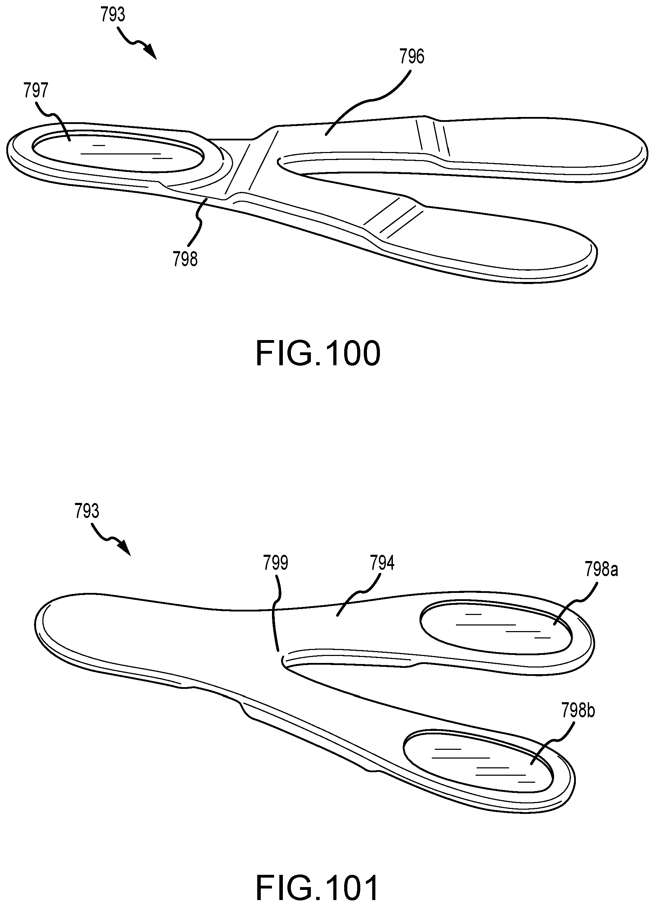

Figures 100 and 101 illustrate a further embodiment flexible connector 793, in which the connector comprises a single material with one or more stiffened regions 796 created by one or more regions of increased material thickness. The stiffened region(s) serve the same purpose as the stiffening layers in the previously described embodiments, enabling the connector to transmit loads and withstand torsional loading during use without buckling or twisting.

As for the previous embodiments, the patient facing surface 794 of the connector 793 is preferably substantially smooth, with the region(s) of increased thickness provided by outward protrusions on a front side of the connector. The thickness of the stiffened region(s) may be substantially constant over the region, or it may vary. For example, the stiffened region(s) may be thicker where more stiffness is required, and/or where the geometry requires a thicker section to achieve the required stiffness, and less thick (but still thicker than non-stiffened regions) where less stiffness is required, and/or where the geometry dictates that the thickness may be reduced.

In one embodiment the connector has a first, greatest, thickness at a central region of the connector, a second, intermediate, thickness at one or both ends of the connector, and a third, thinnest, thickness at the hinging region(s). The second, intermediate thickness at the connector ends may be selected to ensure the connector can flex sufficiently at the connection points.

The connector 793 may comprise one or more hinge regions at which the connector is substantially more flexible than the surrounding parts of the connector. The hinge regions(s) 798 allow the connector 793 to flex in a predictable way to accommodate face contours, with a majority of the flexing of the connector occurring at the hinge regions rather than elsewhere in the body of the connector. The hinge regions(s) 798 allow the connector 793 to flex in and out of the plane of the connector, about an axis that is generally in line with a longitudinal axis of the wearer, in use.

The hinge region may comprise an area of decreased material thickness, a necked area of reduced width, cut-outs or slits, and/or a different material such as a more flexible material. In the embodiment shown, a hinge region 798 is provided between the first end/interface connection point 797 and a bifurcation point 799 of the connector 793, that is, between the first end/interface connection point and the centrally positioned stiffened region 796.

The force required to flex the connector 793 about the hinge region 798 should be less than the force required to detach the first connection point 797 from the patient interface. This prevents disconnection of the connector 793 from the interface as the connector is flexed and installed.

The thickness of the connector 793 gradually increases from the hinge region 798 to the thickened stiffening region 796, for example, by way of a fillet, taper or round at the respective edge of the stiffening region 796. The thickness of the connector 793 gradually decreases from the thickened stiffening region 796 to the adjacent parts of the connector, for example, by way of a fillet or round at the respective edges of the stiffening region 796.

The thickness of the stiffening region may be 2-6 times greater than the thickness of the hinge region 798, for example 2.5-4 times greater. In the example embodiment shown, the stiffening region 796 has a thickness of about 2.25mm and the hinge region a thickness of around 0.8mm. However, other wall thicknesses and/or ratios are envisaged. The thickness of the various regions of the connector body is preferably selected to be as low as possible while still ensuring the connector has the required thickness to resist buckling and torsional loading during use, and so that no region is at a high risk of tearing during use.

The connector 793 may comprise any of the edge features described above in relation to the previous embodiments, to reduce contact forces with a patient's face.

In the embodiment shown, the connector comprises a shallow depression or recess 797, 798a, 798b at each connection point to receive the respective hook or loop pads and to facilitate bonding of the connector body and the pads. The recess may be defined by a raised rim that surrounds the received pad. In other embodiments, the connector may not include such depressions or recesses. The thickness of the connector 793 in the area proximal to and including the connection points 797, 798a, 798b is selected to be sufficient to enable the connector to flex at those areas to at least the extent to ensure a strong connection. For example, to allow the hook and/or loop pads to flex and engage the respective interface or headgear connection along the full length of the pads.

The connector may comprise any suitable flexible, resilient material, for example and elastomer such as a thermoplastic elastomer.

The connector generally increases in width from the first end to the second end. The widening may be non-uniform or non-linear.

A first portion of the connector adjacent the first end of the connector may have a first, substantially uniform width. A second portion of the connector adjacent the second end of the connector may gradually widen towards the second end. This widening may be general widening of the connector body or may comprise a bifurcation of the connector, for example in Y-shaped embodiments.

The first portion of the connector, having a substantially uniform width, may have a length that is from about 1/3 to about 2/3 of the length of the second portion. In one embodiment the first portion may be about half of the length of the second portion, but other shapes and configurations are envisioned.

In the embodiments of Figures 87 to 90, 94-101, and 121 to 123 the connector 93, 593, 693, 793, 1793 is Y-shaped, resembling a wishbone with a single arm branching into a pair of diverging arms at a bifurcation point 599, 699, 799. This increase in width from the first end to the second end improves the stability of the connection with the rounded surface of the headgear. In embodiments having a Y-shape, the pair of arms and therefore the headgear connectors are able to move and flex relative to each other to connect to a larger rounded surface more securely. However, other shapes of connector are envisaged. In some embodiments, the connector may have an asymmetric shape.

In connectors having a Y-shape, the pair of arms may form an angle therebetween of between about 10 degrees and about 60 degrees. In some example embodiments, the angle between the connector arms is about 25 degrees, but other angles are envisioned.

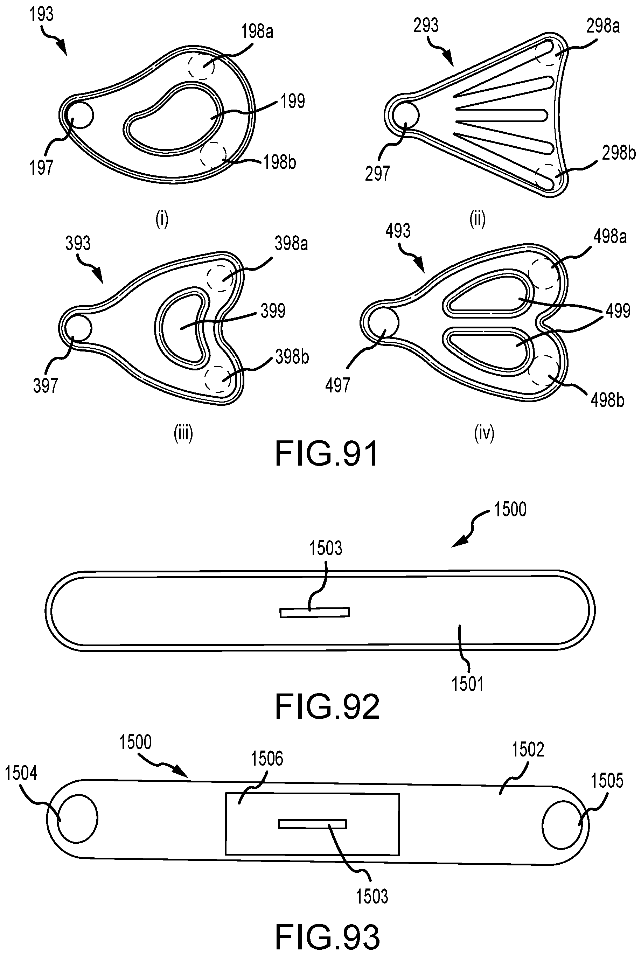

Figures 91(i) to 91(iv) give examples of four embodiment connectors 193, 293, 393, 493, and Figure 104 illustrates several different sized connectors including a single arm connector that increases in width from the first end to the second end, as well connectors of varying sizes. Provision of the connectors in multiple sizes enables the use of a standardised interface frame and conduits for a range of patients of varying sizes through the selection of an appropriately sized connector for each patient.

The connectors may comprise at least two spaced apart headgear connectors 198a, 198b, 298a, 298b, 398a, 398b, 498a, 498b at a wide end of the device, and a single patient interface connection point at a narrow first end of the device. Some connectors, such as the smallest connector shown in Figure 104 may include only a single connection point for connecting to the headgear. The wide second end with spaced apart attachment points at its second end provides for a greater distribution of loads to the headgear and resists twisting of the connector, while the narrow first end minimises material positioned adjacent the wearer's face. The alternative shaped connectors may comprise any suitable shape to accommodate the interface and headgear connection points. The connectors may comprise cut-outs 199, 399, 499 to reduce the weight of the connector. The connectors may be symmetrical or asymmetrical about a horizontal mid-line of the connector.

This single arm connector of Figure 104 may be more appropriate for smaller connectors where the space required at the second end of the connector to accommodate the two headgear connector pads doesn't permit for two diverging arms.

For Y-shaped embodiments having a stiffening layer or stiffened regions, the stiffening layer or region enables the bifurcation point of the Y shape to be positioned closer to the second end of the connector than to the first end as illustrated in Figure 104. This advantageously reduces the width of the connector in the region that will be closest to a wearer's eye. The spacing of the diverging arms is also minimised and selected create space between the connector and the wearer's eyes. The stiffening layer or stiffened region enables this positioning of the bifurcation point and the narrowed spacing of the arms, without compromising the torsional stability of the connector.

All of the corners of the connector are preferably rounded or tapered to reduce pressure points for a wearer. For example, in the embodiment 2493 illustrated in Figures 127 to 129, the edges around the connection pads are rounded. On a patient-facing side of the connector at the first end of the connector, an angled surface 2494c is provided to reduce potential pressure points with a wearer.

### Chin strap

Referring to Figures 92, 93, 118(i) and 118(ii), the headgear assembly may further include a chin strap 1500, 1600 for coupling to the headgear or to connectors, for holding a patient's mouth shut. A chin strap 1500, 1600 may be utilised alongside nasal interfaces such as illustrated for the assembly of Figure 3B, particularly for CPAP applications.

The chin strap 1500, 1600 may comprise multiple layers 1501, 1502; 1601, 1602. The patient facing layer 1502, 1602 and/or the front layer 1501, 1601 may each comprise a material such as a fabric, that is fusible using a selected fusing method. For example, to be fused by way of Radio-frequency (RF) welding or high-frequency (HF) welding, or ultrasonic, vibration or friction welding, hot edge welding, hot air welding, or induction welding. Alternatively the layers of the chin strap may be otherwise bonded together.

In an embodiment, the front layer 1501, 1601 may comprise a fabric that is the same or a similar fabric to the base layer 72 of the headgear 71 described above.

The patient facing layer 1502, 1602 may comprise an engagement surface to enable the chin strap to attach other components to the surface of the chin strap or to itself. In an exemplary embodiment, the patient facing layer 1502, 1602 may comprise unbroken looped (UBL) fabric. This patient facing layer may be larger than the outer, front layer 1501, 1601 such that a perimeter of the inner fabric layer extends around the perimeter of the outer front layer and beyond any harder fused regions, creating a soft edge to protect the skin from the front layer 1501, 1601.

In some embodiments the patient facing fabric layer 1502, 1602 and/or the front fabric layer 1501, 1601 comprise non-stretch fabrics, or fabrics with low levels of stretch. In other embodiments, the layers may be fused so as to remove or minimise any stretch in the chin strap as a whole.

A patient-contacting underside of the chin strap may include a tacky surface 1506, 1606 to provide a region of increased friction between the headgear and the wearer to reduce the incidence of movement on the patient's face. The tacky surface 1506, 1606 is preferably provided in at least a generally central, chin region of the chin strap 1600.

The tacky surface 1506, 1606 may comprise an adhesive film or coating, or may comprise a friction layer adhered or bonded to an inner surface of the patient facing fabric layer 94. The tacky surface may be continuous along all or a portion of the length of the chin strap 1500, 1600, or may comprise discrete regions. The discrete regions may be provided at areas where increased friction is desired. The tacky surface may comprise a linear strip, or may be applied to form a pattern, for example including curved lines and/or dots.

In one embodiment the tacky surface is provided by a polyurethane elastic, neoprene, non-stick silicone, and/or thermoplastic polyurethane. The tacky surface may comprise one suitable for contact with skin and may be provided over substantially all of the patient facing side 1502, 1602 of the chin strap 1500, 1600, the majority of the patient facing side of the chin strap, or may only cover portions of the patient facing side 1502, 1602 of the chin strap 1500, 1602, as shown.

Referring to Figure 93, in a first embodiment, the underside of the chin strap 1500 comprises two engagement surfaces 1504, 1505 provided adjacent opposite ends of the strap 1500. These engagement surfaces 1504, 1505 may comprise hook surfaces for engagement with looped surfaces on other components. For example, engagement surfaces 1504, 1505 may facilitate connection to headgear, for example to the over-ear regions 74 or temple regions of the headgear described above. Alternatively, the engagement surfaces 1504, 1505 may facilitate connection to other connectors such as the side connectors 93 described above.

An opening 1503 may be provided in the chin strap 1500, preferably at or near a centre of the strap. The opening 1503 may have the form of a slot or slit to allow for increased movement of the chinstrap in the vicinity of the opening 1503, for improved stability. This opening 1503 may also be utilised in some applications for the receipt of an orogastric tube. Alternatively, one or more additional openings may be provided for receipt of an orogastric tube or other respiratory system components.

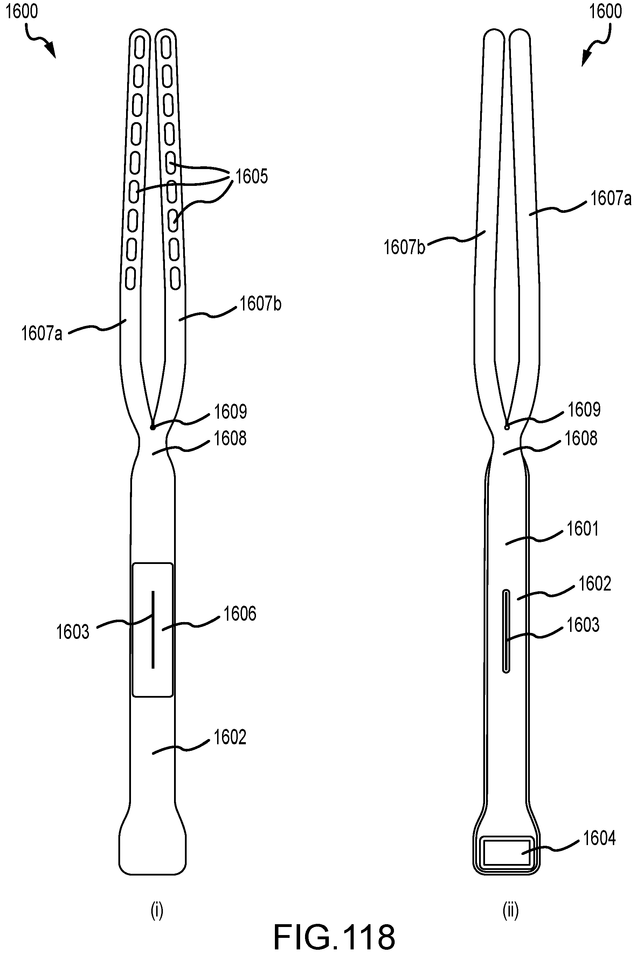

Figures 118(i) to 120 illustrate a second embodiment in which the chin strap 1600 wraps around the patient's head and secures to itself. The chin strap 1600 comprises a first portion with an engagement surface 1604 on the outer surface of the strap and a second portion with two arms 1607a, 1607b.

The engagement surface 1604 of the first portion may comprise a hook-type surface or other suitable surface for engagement with engagement surfaces on the headgear. This engagement surface 1604 may be provided at or adjacent a first end of the chin strap.

The first portion of the strap provides a chin contacting portion 1606. At a point intermediate the chin contacting portion 1606 of the strap and a second end of the strap, the chin strap 1600 bifurcates into a first securing arm 1607a and a second securing arm 1607b. The patient facing side of each securing arm 1607a, 1607b comprises one or more engagement surfaces 1605 of a type to engage with the engagement surface 1604 at the first end of the chin strap 1600.

In this embodiment, the two layers 1601, 1602 of the chin strap 1600 are fused together and the engagement surfaces 1605 on the two arms 1607a, 1607b are loop-type connectors formed by unfused regions of material. However, alternative connectors are envisaged. The chin strap may alternatively comprise a single layer of material or may comprise more than two layers of material fused, bonded, or otherwise joined together. In some embodiments fusing or marking may be utilised to identify the engagement surfaces and/or the intended orientation or use of the chin strap.

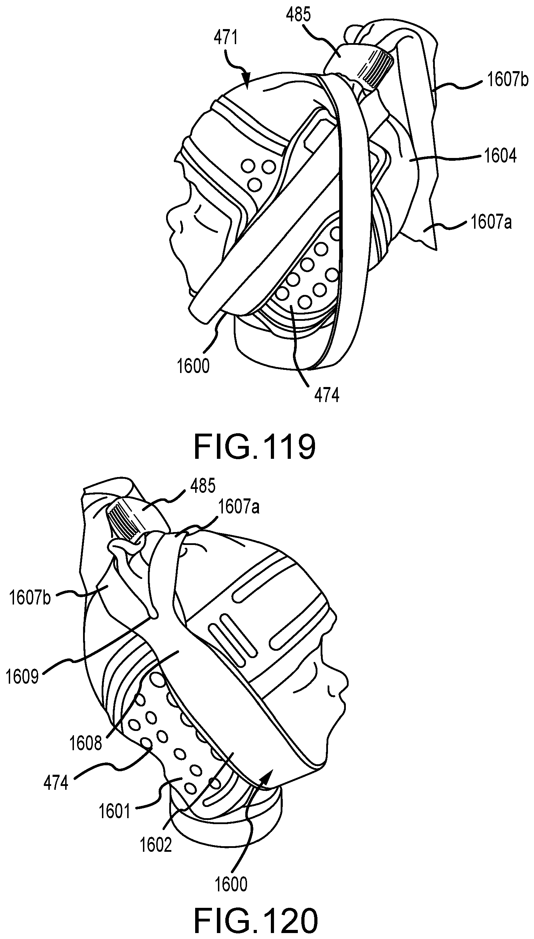

Figures 119 and 120 illustrate the chin strap 1600 secured in place on a patient. To install the chin strap 1600, the first end of the chin strap 1600 is placed against a side of the patient's head, on top of the headgear, in a position such that the chin slit 1603 and the chin contacting portion 1606 are positioned under the chin of the patient. In the embodiment shown the headgear 471 has an adjustment device 485 at a top of the head, and the first end of the chin strap 1600 is positioned between the adjustment device and the patient's ear.

In the present example, the chin strap is then wrapped up, around the opposite side of the patient's head such that the bifurcation point 1609 is positioned between the adjustment device and the patient's ear on the opposite side to the first end of the strap 1600. A first one of the arms 1607a wraps over the patients head forward of the adjustment device 485 and secures to the engagement surface 1604 at the first end of the chin strap 1600, and the second arm 1607b wraps over the patient's head behind the adjustment device 485 and secures to the engagement surface 1604 at the first end of the chin strap and/or to a top surface of the first arm 1607a at a securement point. The first and second arms 1607a, 1607b may cross over at or adjacent to the securement point.

It will be appreciated that in alternative embodiments, the chin strap may be wrapped so that both arms 1607a, 1607b of the chin strap may be positioned forward of or behind the adjustment device. Alternatively, the chin strap may be used with headgear without an adjustment device.

It is envisaged that the chin strap 1600 is not limited for use with the presently described headgear. The chin strap 1600 may be used in combination with alternative headgear including headgear without a top adjustment member, or in applications without headgear. The two arms 1607a, 1607b of the chin strap 1600 may provide a more secure or stable connection to the patient's head.

The two arms 1607a, 1607b and/or the bifurcation region of the chin strap 1600 may be shaped to ensure the arms of the chin strap 1600 and the bifurcation region of the strap lie substantially flat against the headgear 471 and are able to extend around the adjustment device 485 without bowing or twisting.

In the embodiment shown, the chin strap 1600 comprises a necked region 1608 adjacent the bifurcation point and/or a notch 1609 between the two arms at the bifurcation point. These features allow the first and second arms 1607a, 1607b to be moved away from each other while minimising resulting deformation of the chin strap 1600 adjacent the bifurcation point.

The arms 1607a, 1607b of the strap may be shaped such that the spacing between the arms is greater towards the bifurcation point, where the arms will extend around the adjustment member, and less at the second end of the chin strap 1600. This minimises the in-plane curving of the arms 1607a, 1607b in use and thereby reduces puckering, buckling or twisting of the arms.

The chin strap 1600 may have an increased width adjacent the first end to accommodate a wider engagement surface 1604 to ensure there is sufficient area to comfortably engage with both the first and second arms 1607a, 1607b.

Preferred embodiments of the invention have been described by way of example only and modifications may be made thereto without departing from the scope of the invention, which is defined by the claims.

## Claims

1. A headgear (71) for securing a patient interface to a patient, the headgear (71) comprising:
a base layer (72) forming a body of the headgear; and
a headband region (73);
wherein the headband region (73) comprises an outer engagement layer that at least partly overlaps a lower portion of the base layer (72), the outer engagement layer being fused to an underlying portion of the base layer (72);
wherein the headband region (73) comprises areas of fused material and areas of unfused material, the unfused areas at least in part defining connection zones for releasably securing connectors to the headgear (71);
**characterised in that**:
the unfused areas form a raised engagement surface, and the fused areas form depressions.

2. The headgear (71) as claimed in claim 1, wherein the outer engagement layer is fused to the underlying portion of the base layer (72) across a majority of the outer engagement layer.

3. The headgear (71) as claimed in any one of claims 1 or 2, wherein the base layer (72) comprises a single panel of material.

4. The headgear (71) as claimed in any one of claims 1 to 3, wherein the outer engagement layer comprises a single panel of material.

5. The headgear (71) as claimed in any one of claims 1 to 4, wherein the headband region (73) comprises enlarged over-ear regions (74) shaped to at least partly cover the patient's ears.

6. The headgear (71) as claimed in claim 5, wherein the over-ear regions (74) are configured to provide a larger engagement surface compared to other regions of the headband, to facilitate the attachment of securing members to the headband to hold the patient interface (11) in place.

7. The headgear (71) as claimed in claims 5 or 6, wherein the headband region (73) further comprises an extension portion extending from one or both over-ear regions (74).

8. The headgear (71) as claimed in any one of claims 1 to 7, wherein the headband region (73) and a lower portion of the body are configured to wrap around the head of the wearer such that opposing end portions of the headband overlap and secure to each other, thereby providing an adjustable fit.

9. The headgear (71) as claimed in any one of claims 1 to 8, comprising an end fixture (81) securing a top edge of the headgear body together at a securement point.

10. The headgear (71) as claimed in any one of claims 1 to 9, wherein the headgear (71) is a bonnet.

11. The headgear (71) as claimed in any one of claims 1 to 10, wherein the areas of fused material exhibit different stretch to the areas of unfused material.

12. The headgear (71) as claimed in claim 11, wherein the shape and/or the orientation and/or the position of the fused areas is selected to reduce the stretch of a respective region of the headband in one or more directions.

13. The headgear (71) as claimed in any one of claims 5 to 7, or any of claims 8-12 insofar as dependent on claim 5 directly or indirectly, wherein the headband region (73) comprises a region of increased stretch between the over-ear regions (74).

14. A patient interface assembly (1, 101, 1001), comprising:
the headgear (71) as claimed in any one of claims 1 to 13; and
a patient interface (101) configured to be coupled to the headgear (71) by releasably securing the connectors (93) to the connection zones of the headband region (73) of the headgear, the
patient interface (101) being a respiratory mask or nasal cannula (11).

## Patentansprüche

1. Kopfbedeckung (71) zur Befestigung einer Patientenschnittstelle an einem Patienten, wobei die Kopfbedeckung (71) umfasst:
eine Basisschicht (72), die einen Körper der Kopfbedeckung bildet; und
einen Kopfbandbereich (73);
wobei der Kopfbandbereich (73) eine äußere Eingriffsschicht umfasst, die einen unteren Abschnitt der Basisschicht (72) zumindest teilweise überlappt, wobei die äußere Eingriffsschicht mit einem darunterliegenden Abschnitt der Basisschicht (72) verschmolzen ist;
wobei der Kopfbandbereich (73) Bereiche aus verschmolzenem Material und Bereiche aus unverschmolzenem Material umfasst, wobei die unverschmolzenen Bereiche zumindest teilweise Verbindungszonen zum lösbaren Befestigen von Verbindern an der Kopfbedeckung (71) definieren;
**dadurch gekennzeichnet, dass**
die unverschmolzenen Bereiche eine erhabene Eingriffsfläche bilden und die verschmolzenen Bereiche Vertiefungen bilden.

2. Kopfbedeckung (71) nach Anspruch 1, wobei die äußere Eingriffsschicht über einen Großteil der äußeren Eingriffsschicht hinweg mit dem darunterliegenden Abschnitt der Basisschicht (72) verschmolzen ist.

3. Kopfbedeckung (71) nach einem der Ansprüche 1 oder 2, wobei die Basisschicht (72) eine einzelne Materialbahn umfasst.

4. Kopfbedeckung (71) nach einem der Ansprüche 1 bis 3, wobei die äußere Eingriffsschicht eine einzelne Materialbahn umfasst.

5. Kopfbedeckung (71) nach einem der Ansprüche 1 bis 4, wobei der Kopfbandbereich (73) vergrößerte Über-Ohr-Bereiche (74) umfasst, die so geformt sind, dass sie die Ohren des Patienten zumindest teilweise bedecken.

6. Kopfbedeckung (71) nach Anspruch 5, wobei die Über-Ohr-Bereiche (74) dazu konfiguriert sind, im Vergleich zu anderen Bereichen des Kopfbands eine größere Eingriffsfläche bereitzustellen, um die Anbringung von Befestigungselementen an dem Kopfband zu erleichtern, um die Patientenschnittstelle (11) an Ort und Stelle zu halten.

7. Kopfbedeckung (71) nach Anspruch 5 oder 6, wobei der Kopfbandbereich (73) ferner einen Verlängerungsabschnitt umfasst, der sich von einem oder beiden Über-Ohr-Bereichen (74) erstreckt.

8. Kopfbedeckung (71) nach einem der Ansprüche 1 bis 7, wobei der Kopfbandbereich (73) und ein unterer Abschnitt des Körpers dazu konfiguriert sind, sich um den Kopf des Trägers zu wickeln, sodass gegenüberliegende Endabschnitte des Kopfbands einander überlappen und aneinander befestigt werden, wodurch eine einstellbare Passform bereitgestellt wird.

9. Kopfbedeckung (71) nach einem der Ansprüche 1 bis 8, umfassend eine Endbefestigung (81), die eine obere Kante des Kopfbedeckungskörpers an einem Befestigungspunkt zusammenhält.

10. Kopfbedeckung (71) nach einem der Ansprüche 1 bis 9, wobei die Kopfbedeckung (71) eine Haube ist.

11. Kopfbedeckung (71) nach einem der Ansprüche 1 bis 10, wobei die Bereiche aus verschmolzenem Material eine andere Dehnbarkeit aufweisen als die Bereiche aus unverschmolzenem Material.

12. Kopfbedeckung (71) nach Anspruch 11, wobei die Form und/oder die Ausrichtung und/oder die Position der verschmolzenen Bereiche ausgewählt ist, um die Dehnbarkeit eines jeweiligen Bereichs des Kopfbands in einer oder mehreren Richtungen zu reduzieren.

13. Kopfbedeckung (71) nach einem der Ansprüche 5 bis 7 oder nach einem der Ansprüche 8 bis 12, insoweit diese direkt oder indirekt von Anspruch 5 abhängig sind, wobei der Kopfbandbereich (73) einen Bereich erhöhter Dehnbarkeit zwischen den Über-Ohr-Bereichen (74) umfasst.

14. Patientenschnittstellenanordnung (1, 101, 1001), umfassend:
die Kopfbedeckung (71) nach einem der Ansprüche 1 bis 13; und
eine Patientenschnittstelle (101), die dazu konfiguriert ist, mit der Kopfbedeckung (71) gekoppelt zu werden, indem die Verbinder (93) lösbar an den Verbindungszonen des Kopfbandbereichs (73) der Kopfbedeckung befestigt werden, wobei die Patientenschnittstelle (101) eine Atemmaske oder Nasenkanüle (11) ist.

## Revendications

1. Harnais (71) pour fixer une interface patient à un patient, le harnais (71) comprenant :
une couche de base (72) formant un corps du harnais ; et
une région de serre-tête (73) ;
dans lequel la région de serre-tête (73) comprend une couche de mise en prise externe qui chevauche au moins partiellement une partie inférieure de la couche de base (72), la couche de mise en prise externe étant fusionnée à une partie sous-jacente de la couche de base (72) ;
dans lequel la région de serre-tête (73) comprend des zones de matériau fondu et des zones de matériau non fondu, les zones non fusionnées définissant au moins en partie des zones de connexion pour fixer de manière détachable des connecteurs au harnais (71) ;
**caractérisé en ce que**
les zones non fusionnées forment une surface de mise en prise surélevée et les zones fusionnées forment des dépressions.

2. Harnais (71) selon la revendication 1, dans lequel la couche de mise en prise externe est fusionnée à la partie sous-jacente de la couche de base (72) sur une majorité de la couche de mise en prise externe.

3. Harnais (71) selon l'une quelconque des revendications 1 ou 2, dans lequel la couche de base (72) comprend un seul panneau de matériau.

4. Harnais (71) selon l'une quelconque des revendications 1 à 3, dans lequel la couche de mise en prise externe comprend un seul panneau de matériau.

5. Harnais (71) selon l'une quelconque des revendications 1 à 4, dans lequel la région de serre-tête (73) comprend des régions supra-auriculaires (74) agrandies formées pour recouvrir au moins partiellement les oreilles du patient.

6. Harnais (71) selon la revendication 5, dans lequel les régions supra-auriculaires (74) sont configurées pour fournir une surface de mise en prise plus grande par rapport à d'autres régions du serre-tête, pour faciliter l'attache d'éléments de fixation au serre-tête pour maintenir l'interface patient (11) en place.

7. Harnais (71) selon les revendications 5 ou 6, dans lequel la région de serre-tête (73) comprend en outre une partie d'extension s'étendant à partir d'une ou des deux régions supra-auriculaires (74).

8. Harnais (71) selon l'une quelconque des revendications 1 à 7, dans lequel la région de serre-tête (73) et une partie inférieure du corps sont configurées pour s'enrouler autour de la tête du porteur de sorte que des parties d'extrémité opposées du serre-tête se chevauchent et se fixent l'une à l'autre, fournissant ainsi un ajustement réglable.

9. Harnais (71) selon l'une quelconque des revendications 1 à 8, comprenant une fixation d'extrémité (81) fixant un bord supérieur du corps de harnais ensemble au niveau d'un point de fixation.

10. Harnais (71) selon l'une quelconque des revendications 1 à 9, dans lequel le harnais (71) est une coiffe.

11. Harnais (71) selon l'une quelconque des revendications 1 à 10, dans lequel les zones de matériau fondu présentent un étirement différent des zones de matériau non fondu.

12. Harnais (71) selon la revendication 11, dans lequel la forme et/ou l'orientation et/ou la position des zones fusionnées est choisie pour réduire l'étirement d'une région respective du serre-tête dans une ou plusieurs directions.

13. Harnais (71) selon l'une quelconque des revendications 5 à 7, ou l'une quelconque des revendications 8-12 dans la mesure où elles dépendent directement ou indirectement de la revendication 5,
dans lequel la région de serre-tête (73) comprend une région d'étirement accru entre les régions supra-auriculaires (74).

14. Ensemble d'interface patient (1, 101, 1001), comprenant :
le harnais (71) selon l'une quelconque des revendications 1 à 13 ; et
une interface patient (101) configurée pour être couplée au harnais (71) en fixant de manière détachable les connecteurs (93) aux zones de connexion de la région de serre-tête (73) du harnais, l'interface patient (101) étant un masque respiratoire ou une canule nasale (11).
